# EUROPEAN PATENT APPLICATION

(11) **EP 3 763 739 A1**
(43) Date of publication of application: **13.01.2021**
(21) Application number: 20163877.2
(22) Date of filing: 22.05.2015
(51) Int. Cl.: C07K 16/28

(54) **ANTIBODIES BINDING TO HUMAN AND CYNOMOLGUS CD3 EPSILON**

(30) Priority: 28.05.2014 EP 14170140; 11.08.2014 EP 14180572
(62) Divisional of application: 15725002.8
(71) Applicant: F. Hoffmann-La Roche AG, 4070 Basel (CH)
(72) Inventor: NEUMANN, Christiane, 8952 Schlieren (CH); LIFKE, Valeria, 82377 Penzberg (DE); MOESSNER, Ekkehard, 8952 Schlieren (CH); OFFNER, Sonja, 82377 Penzberg (DE); PLATZER, Josef, 82377 Penzberg (DE); TIEFENTHALER, Georg, 82377 Penzberg (DE); RITTER, Mirko, 82377 Penzberg (DE)
(74) Representative: Burger, Alexander

(57) **Abstract**

One aspect as reported herein is using a method comprising the step of immunizing an experimental animal, three times with primary cynomolgus PBLs, whereby the PBLs are optionally enriched for T cells without using primary human PBLs as immunogen and without using a denaturing agent for producing a human cynomolgus cross-reactive antibody specifically binding to human CD3 epsilon of SEQ ID NO: 02 and specifically binding to a polypeptide of SEQ ID NO: 01, wherein the human cynomolgus cross-reactive antibody specifically binds to human and cynomolgus T cells, activates human T cells and does not bind to the same epitope as the antibody OKT3, the antibody UCHT1 and/or antibody the SP34.

## Description

The current invention is in the field of cross-reactive antibodies. Herein is reported a method and its use for generating human cynomolgus cross-reactive antibodies.

### Background of the Invention

T cells are key effectors of the adaptive immune response, with a number of important roles in the elimination of pathogens and in autoimmune diseases. There are several subsets of T cells, each with a distinct function.

The TCRs (T-cell receptors), which are found on the surface of T cells, are heterodimers composed of either an alpha and beta polypeptide chain a composition, constituting approximately 95% of the TCR population, or a gamma and delta polypeptide chain (Pitcher and van Oers, 2003; Malissen, 2008). Each polypeptide contains a constant (C) and variable (V) region. The constant region is anchored in the cell membrane, while the variable region extends extracellularly and is responsible for binding antigen. The short cytoplasmic tail of the TCR lacks the ability to signal. Intracellular signaling is initiated by the CD3 protein complex, which comprises intracellular immunoreceptor tyrosine activation motifs (ITAMs).

The CD3 (cluster of differentiation 3) T-cell co-receptor is a protein complex and is composed of four distinct chains. In mammals, the complex contains a CD3γ (gamma) chain, a CD3δ (delta) chain, and two CD3ε (epsilon) chains. These chains associate with the TCR and the ζ-chain (zeta-chain) to generate an activation signal in T lymphocytes. The TCR, ζ-chain, and CD3 molecules together comprise the TCR complex. The CD3γ, CD3δ, and CD3ε chains are highly related cell-surface proteins of the immunoglobulin superfamily containing a single extracellular immunoglobulin domain.

TCRs cannot bind free epitopes/antigens, instead TCRs bind enzymatically cleaved fragments of larger polypeptides associated with major histocompatibility complexes (MHC), which is synonymous with the human leukocyte antigen (HLA) system in humans (Rudd 1990; Gao et al., 2002). This interaction occurs in a space that has become known as the immunological synapse. MHC class I molecules are expressed on all nucleated cells of the body and present antigen to cytotoxic T cells and CD8 on these cells stabilizes the MHC/TCR interaction. The activation of cytotoxic T cells subsequently results in the destruction of (virally) infected cells. MHC class II is found on macrophages, B cells and dendritic cells. These immune cells present antigen to helper T cells with CD4 stabilizing the MHC/TCR interaction. The interaction between MHC class II and the TCR ultimately results in an antibody mediated immune response. Other co-stimulatory molecules, such as CD45, CD28 and CD2 aid in T cell activation in the immunological synapse and initiate the formation of the TCR signalosome, a macromolecular protein complex responsible for intracellular signaling.

Several antibodies that bind to human CD3 epsilon are known in the art, e.g. the antibody OKT3 (see e.g. Kung, P. et al., Science 206 (1979) 347-349; Salmeron, A. et al., J Immunol 147 (1991) 3047-3052), the antibody UCHT1 (see e.g. Callard, R.E. et al., Clin Exp Immunol 43 (1981) 497-505) or the antibody SP34 (see e.g. Pessano, S. et al., EMBO J 4 (1985) 337-344). From these, seemingly only the antibody SP34 is human cynomolgus cross-reactive (Conrad M.L., et. al., Cytometry A 71 (2007) 925-933).

WO 2007/042261 reports compositions comprising cross-species-specific antibodies and uses thereof. In Soo Young Yang, et al., LN USA 137 (1986) 1097-1100) a common pathway for T lymphocyte activation involving both the CD3-Ti complex and CD2 sheep erythrocyte receptor determinants are reported. WO 2012/158818 reports multi-specific Fab fusion proteins and methods of use. In DD 272473 a method for the production of monoclonal antibodies against the epsilon chain of the CD3 antigen of human T lymphocytes is reported.

### Summary of the Invention

The invention provides a method and its use for producing a human cynomolgus cross-reactive antibody.

It has been found that by immunizing a non-human experimental animal solely with a cynomolgus antigen, i.e. without immunizing the experimental animal before or thereafter with the human homolog, a human cynomolgus cross-reactive antibody can be obtained.

One aspect as reported herein is the use of a method comprising the step of immunizing a non-human experimental animal with a native cynomolgus antigen as sole antigen for producing/generating/obtaining a human cynomolgus cross-reactive antibody.

In one embodiment the native cynomolgus antigen lacks one or more (contiguous) amino acid stretches that are present in the corresponding human antigen, whereby one of the lacking (contiguous) amino acid stretches in the corresponding human antigen is the main immunogenic site/epitope of the human antigen. In one embodiment the (native cynomolgus) antigen is CD3 epsilon and the human cynomolgus cross-reactive antibody specifically binds to (native) human CD3 epsilon of SEQ ID NO: 02 and specifically binds to a polypeptide of SEQ ID NO: 01. In one embodiment the non-human experimental animal is immunized one or more times with primary cynomolgus PBLs, whereby the PBLs are (optionally) enriched for T cells. In one embodiment the immunizing comprises as first step (injection) an intradermal application, as second step (injection) an intramuscular application and as third step (injection) a subcutaneous application. In one embodiment the method is without the use of/using a denaturing agent. In one embodiment the human cynomolgus cross-reactive antibody specifically binds to human and cynomolgus CD3 epsilon, to the polypeptide of SEQ ID NO: 01 and activates human T cells.

Another aspect as reported herein is the use of a method comprising the step of immunizing a non-human experimental animal three times with primary cynomolgus PBLs, (optionally) enriched for T cells, without the use of/using primary human PBLs as immunogen and without the use of/using a denaturing agent for producing/generating/obtaining a human cynomolgus cross-reactive antibody specifically binding to human CD3 epsilon of SEQ ID NO: 02 and specifically binding to a polypeptide of SEQ ID NO: 01, wherein the antibody specifically binds to human and cynomolgus T cells, activates human T cells, and does not bind to the same epitope as the antibody OKT3 (see e.g. Kung, P. et al., Science 206 (1979) 347-349; Salmeron, A. et al., J Immunol 147 (1991) 3047-3052), the antibody UCHT1 (see e.g. Callard, R.E. et al., Clin Exp Immunol 43 (1981) 497-505) or the antibody SP34 (see e.g. Pessano, S. et al., EMBO J 4 (1985) 337-344). In one embodiment the antibody as reported herein does not bind to the same epitope as the antibody reported in WO 2007/042261. In one embodiment the antibody as reported herein does not bind to the same epitope as the antibody reported in WO 2007/042261 and specifically binds to human and cynomolgus T cells and(/or) activates human T cells.

Another aspect as reported herein is a method for producing a human cynomolgus cross-reactive antibody comprising the step of immunizing a non-human experimental animal with a native cynomolgus antigen as sole antigen.

In one embodiment the native cynomolgus antigen lacks one or more (contiguous) amino acid stretches that are present in the corresponding human antigen, whereby one of the lacking (contiguous) amino acid stretches in the corresponding human antigen is the main immunogenic epitope of the human antigen. In one embodiment the non-human experimental animal is immunized one or more times with primary cynomolgus PBLs, whereby the PBLs are (optionally) enriched for T cells. In one embodiment the immunizing comprises as first step an intradermal application, as second step an intramuscular application and as third step a subcutaneous application.

Another aspect as reported herein is a method for producing a human cynomolgus cross-reactive antibody that specifically binds to human CD3 epsilon of SEQ ID NO: 02 and that specifically binds to a polypeptide of SEQ ID NO: 01 comprising the step of immunizing a non-human experimental animal three times with primary cynomolgus PBLs, whereby the PBLs are (optionally) enriched for T cells, without using primary human PBLs as immunogen and without using a denaturing agent, wherein the human cynomolgus cross-reactive antibody specifically binds to human and cynomolgus T cells, activates human T cells, and does not bind to the same epitope as the antibody OKT3, the antibody UCHT1 and/or the antibody SP34.

Another aspect as reported herein is a human cynomolgus cross-reactive antibody that specifically binds to human CD3 epsilon of SEQ ID NO: 02 and that specifically binds to a polypeptide of SEQ ID NO: 01, wherein the human cynomolgus cross-reactive antibody specifically binds to human and cynomolgus T cells and activates human T cells. In one embodiment the antibody as reported herein does not bind to the same epitope as the antibody reported in WO 2007/042261. In one embodiment the antibody as reported herein does not bind to the same epitope as the antibody reported in WO 2007/042261 and specifically binds to human and cynomolgus T cells and(/or) activates human T cells.

Another aspect as reported herein is a human cynomolgus cross-reactive antibody that specifically binds to human CD3 epsilon of SEQ ID NO: 02 and that specifically binds to a polypeptide of SEQ ID NO: 01, wherein the human cynomolgus cross-reactive antibody specifically binds to human and cynomolgus T cells, activates human T cells and does not bind to the same epitope as the antibody OKT3, the antibody UCHT1 and/or the antibody SP34 and/or the antibody reported in WO 2007/042261.

Another aspect as reported herein is a human cynomolgus cross-reactive antibody that specifically binds to human CD3 epsilon of SEQ ID NO: 02 and that specifically binds to a polypeptide of SEQ ID NO: 01 obtainable/obtained by immunizing a non-human experimental animal three times with primary cynomolgus PBLs, whereby the PBLs are (optionally) enriched for T cells, without the use of/using primary human PBLs as immunogen and without using a denaturing agent, wherein the human cynomolgus cross-reactive antibody specifically binds to human and cynomolgus T cells, activates human T cells, and does not bind to the same epitope as the antibody OKT3, the antibody UCHT1 and/or the antibody SP34. In one embodiment the antibody as reported herein does not bind to the same epitope as the antibody reported in WO 2007/042261. In one embodiment the antibody as reported herein does not bind to the same epitope as the antibody reported in WO 2007/042261 and specifically binds to human and cynomolgus T cells and(/or) activates human T cells.

### Detailed Description of the Invention

T cells are key effectors of the adaptive immune response, with a number of important roles in the elimination of pathogens and in autoimmune diseases. There are several subsets of T cells, each with a distinct function.

T helper cells assist other white blood cells in immunologic processes, including maturation of B cells into plasma cells and memory B cells, and activation of cytotoxic T cells and macrophages. These cells are also known as CD4+ T cells because they express the CD4 glycoprotein on their surface. T helper cells become activated when they are presented with peptide antigens by MHC class II molecules, which are expressed on the surface of APCs. Once activated, they divide rapidly and secrete small proteins called cytokines that regulate or assist in the active immune response.

Cytotoxic T cells destroy virally infected cells and tumor cells, and are also implicated in transplant rejection. These cells are also known as CD8+ T cells since they express the CD8 glycoprotein at their surface. These cells recognize their targets by binding to antigen associated with MHC class I, which is present on the surface of all nucleated cells.

Memory T cells are a subset of antigen-specific T cells that persist long-term after an infection has resolved. They quickly expand to large numbers of effector T cells upon re-exposure to their cognate antigen, thus providing the immune system with "memory" against past infections. Memory cells may be either CD4+ or CD8+.

Regulatory T cells, formerly known as suppressor T cells, are crucial for the maintenance of immunological tolerance. Their major role is to shut down T cell-mediated immunity toward the end of an immune reaction and to suppress auto-reactive T cells that escaped the process of negative selection in the thymus.

Natural killer T cells bridge the adaptive immune system with the innate immune system. Unlike conventional T cells that recognize peptide antigens presented by major histocompatibility complex (MHC) molecules, NKT cells recognize glycolipid antigen presented by a molecule called CD1d. Once activated, these cells can perform functions ascribed to both helper T cells and cytotoxic T cells (i.e., cytokine production and release of cytolytic/cell killing molecules).

Following interaction of the TCR with a peptide-MHC complex, the TCR co-receptors CD4 and CD8 are important for targeting the delivery of the Src kinase LCK into close proximity to its substrates (Veillette et al., 1988, Cell): the TCR-associated CD3 and zeta-chain immunoreceptor tyrosine-based activation motifs (ITAMs) (Artyomov et al., 2010, Proc. Natl Acad. Sci. USA). Live unstimulated T cells were shown to have the signature tyrosine, and leucine or isoleucine, residues of their CD3ε ITAMs buried in the lipid bilayer of the plasma membrane. On activation, these transmembrane domains are released from the lipid bilayer and become available as substrates for LCK (Xu et al., 2008, Cell). It is unclear what triggers the dissociation of the CD3 epsilon cytoplasmic domain, but transitional changes in the local lipid environment on TCR engagement (Xu et al., 2008, Cell), or mechano-sensing of a torque exerted on the CD3 epsilon chain by the TCRs binding to a peptide-MHC complex, have been postulated as possible causes (Kim et al., 2012, Front. Immunol.). The kinetic segregation model suggests that TCR signaling is triggered as a result of the TCR being partitioned into areas of the lipid membrane that are rich in LCK and that lack the transmembrane phosphatase CD45.

Triggering of the TCR, the abundance of LCK together with the abundance and location of its regulators dictate the extent to which the targets of LCK will be phosphorylated (Lovatt et al., 2006, Mol. Cell. Biol.). These targets include the tyrosine residues in the ITAMs of TCR-associated CD3 gamma chain, CD3 delta chain, CD3 epsilon chains and the zeta-chains, and the SYK family kinase ZAP70 (zeta-chain associated protein kinase of 70 kDa). As a consequence several major signaling branches can be activated (Acuto et al., 2008, Nature Rev. Immunol.): up-regulation of integrin affinity, which promotes cell adhesion; the coordinated mobilization to the nucleus of transcription factors that are crucial for the expression of genes necessary for T cell growth and differentiation; and actin reorganization, which is essential for T cell activation, proliferation, adhesion and differentiation of T cells into effector T cells (for more details see review: Brownlie & Zamoyska , 2013, Nat Rev Immunol.).

Various studies have revealed that the CD3 molecules are important for the proper cell surface expression of the alpha beta TCR and normal T cell development (Berkhout et al., 1988, J. Biol. Chem.; Wang et al., 1998, J. Exp. Med.; Kappes, 1995, Curr. Opin. Immunol.). Although the cysteine-rich stalk appears to play an important role in driving CD3 dimerization (Su, loc. cit., Borroto, 1998, J. Biol. Chem.), interaction by means of the extracellular domains (ECDs) of CD3 epsilon (CD3e) and CD3 gamma is sufficient for assembly of these proteins with TCR beta (Manolios, 1994, Eur. J. Immunol.; Manolios & Li, 1995, Immunol. Cell Biol.). The stoichiometry of the TCR most likely comprises one alpha beta TCR, one CD3 epsilon gamma heterodimer, one CD3 epsilon delta heterodimer and one CD3 zeta zeta homodimer. Given the central role of the human CD3 epsilon gamma heterodimer in the immune response, the crystal structure of this complex bound to the therapeutic antibody OKT3 had been elucidated (Kjer-Nielsen, 2004, PNAS).

A number of therapeutic strategies modulate T cell immunity by targeting TCR signaling, particularly by anti-human CD3 monoclonal antibodies that are clinically used. Animal studies have shown that anti-CD3 antibodies induce tolerance to allografts (Nicolls et al., 1993) and OKT3, an anti-CD3 antibody directed against CD3 epsilon, has been clinically approved for use in humans for the induction of immunosuppression in solid organ transplantation for the prevention and treatment of rejection (Norman 1995). Interestingly, susceptibility to type I diabetes has been associated with the CD3 epsilon genetic locus (Wong et al., 1991) and anti-CD3 antibodies have been shown to ameliorate the symptoms of type I diabetes and other auto-immune disorders (Sprangers et al., 2011). CD3 specific antibodies (Tunnacliffe, 1989, Int. Immunol.) are able to induce various T cell responses such as lymphokine production (Von Wussow, 1981, J. Immunol.; Palacious, 1982, J. Immunol.), proliferation (Van Wauve, 1980, J. Immunol.) and suppressor-T cell induction (Kunicka, 1986, in "Lymphocyte Typing II"). Depending on the experimental conditions, CD3 specific monoclonal antibody can either inhibit or induce cytotoxicity (Leewenberg, 1985, J. Immunol.; Phillips, 1986, J . Immunol.; Platsoucas, 1981, Proc. Natl. Acad. Sci.; Itoh, 1987, Cell. Immunol.; Mentzer, 1985, J. Immunol.; Landegren, 1982, J. Exp. Med.; Choi, 2001, Eur. J. Immunol.; Xu, 2000, Cell Immunol.; Kimball, 1995, Transpl. Immunol.).

Several studies report that the most widely used CD3 epsilon monoclonal antibodies OKT3, WT31, UCHT1, 7D6 and Leu-4 did not bind to cells singly transfected with the CD3-epsilon chain. However, these antibodies stained cells doubly transfected with a combination of CD3 epsilon plus either CD3 gamma or CD3 delta (Tunnacliffe, 1989, Int. Immunol.; Law, 2002, Int. Immunol.; Salmeron, 1991, J. Immunol.; Coulie, 1991, Eur. J. Immunol.). In a second smaller group, the conformational epitope is being formed within the CD3 epsilon subunit itself. A member of this group is for instance mAb APA 1 /1 which has been raised against denatured CD3 epsilon (Risueno, 2005, Blood). Taken together, most of the CD3 epsilon antibodies described in the art recognize conformational epitopes located on two or more subunits of CD3 and, thus, only recognize CD3 epsilon in the native context of the TCR.

The species specificity is a significant impediment to the development of antibodies as therapeutic agents for the treatment of human diseases. In order to obtain market approval any new candidate medication must pass through preclinical and clinical phases: Whereas the latter is performed in human patients, the former is performed in animals. The aim of pre-clinical testing is to prove that the drug candidate has the desired activity and most importantly is safe. Only when the safety in animals and possible effectiveness of the drug candidate has been established in preclinical testing this drug candidate will be approved for clinical testing in humans by the respective regulatory authority. Preferably lower primates like cynomolgus are used for safety testing of drug candidates interfering with the immune system since chimpanzees are considered as endangered species and due to their human-like nature, the use of such animals for drug safety testing has been banned in Europe and is highly restricted elsewhere.

Many CD3 antibodies have been found to be species-specific. One of the most widely used and best characterized monoclonal antibodies specific for the CD3 complex is OKT-3. This antibody reacts with chimpanzee CD3 but not with the CD3 homolog of other primates, such as macaques (e.g. cynomolgus monkey), or with dog CD3 (Sandusky et al., 1986, J. Med. Primatol.). The anti-CD3 monoclonal antibody UCHT-1 is also reactive with CD3 from chimpanzee but not with CD3 from macaques. On the other hand, there are also examples of monoclonal antibodies, which recognize macaque antigens, but not their human counterparts like the antibody FN18.

As mentioned above there are a few antibodies available that bind and activate human T cells via CD3 epsilon (CD3e), a subunit of the CD3 complex: OKT3 (Kung et al., 1979, Science; Salmeron et al., 1991, J Immunol), UCHT1 (Callard et al., 1981, Clin Exp Immunol), its derivative V9 (Zhu & Carter, 1995, J Immunol) and SP34 (Pessano et al., 1985, EMBO J). OKT3, UCHT1 (= derivative V9) are not cross-reactive to cynomolgus T cells. The only antibody that binds and activates cynomolgus T cells seems to be the SP34 antibody (Conrad et al., 2007, Cytometry A).

The generation of suitable antibodies is massively hampered by the fact that it is difficult to obtain recombinant monomeric CD3e with its native conformation since for example the recombinantly expressed CD3e forms a homo dimer that has an artificial conformation (Su et al., 2009, Int J Mol Med). In addition, a retention signal for the endoplasmatic reticulum within CD3e causes an intracellular accumulation with the consequence that CD3e does not reach the cell surface, if recombinantly expressed in cells (Brodeur et al., 2009, Int Immunol). Even worse, highly charged amino acid in the intracellular tail also hinders appropriate recombinant expression in and on cells (Call & Wucherpfennig 2005, Annu Rev Immunol).

Further, the low sequence similarity of human and cynomolgus CD3 epsilon ECD of 72% identical amino acids makes it quite challenging to generate functional human cynomolgus cross-reactive antibodies.

The current invention is based, at least in part, on the finding that human cynomolgus cross-reactive antibodies can be obtained by immunizing a non-human experimental animal solely with a cynomolgus antigen, i.e. without immunizing the experimental animal before or thereafter with the human homolog. The skilled person understands that immunizing a cynomolgus monkey as experimental animal would not be suitable to obtain human cynomolgus cross-reactive antibodies, i.e. the non-human experimental animal is also a non-cynomolgus experimental animal.

Accordingly, one aspect as reported herein is a method comprising the step of immunizing an experimental animal with a native cynomolgus antigen as sole antigen for producing a human cynomolgus cross-reactive antibody. In one embodiment the native cynomolgus antigen has less than 80% sequence identity to the corresponding human antigen. In one embodiment the native cynomolgus antigen has 80% to 60% sequence identity to the corresponding human antigen. In one embodiment the native cynomolgus antigen has 80% to 70% sequence identity to the corresponding human antigen.

In has been found that human cynomolgus cross-reactive antibodies can be obtained when amino acid stretches that are highly immunogenic in the human antigen are avoided for immunization. In one embodiment the native cynomolgus antigen lacks one or more (contiguous) amino acid stretches that are present in the corresponding human antigen, whereby one of the lacking (contiguous) amino acid stretches in the corresponding human antigen is the main immunogenic epitope of the human antigen.

In one embodiment the native cynomolgus antigen is a T cell antigen. In one embodiment the native cynomolgus antigen is CD3 epsilon. In one embodiment the native cynomolgus antigen is CD3 epsilon and the human cynomolgus cross-reactive antibody specifically binds to (native) human CD3 epsilon of SEQ ID NO: 02 and specifically binds to a polypeptide of SEQ ID NO: 01 (YPRGSKPEDANFYL YLRARV).

In one embodiment the experimental animal is immunized one or more times with primary cynomolgus PBLs, whereby the PBLs are optionally enriched for T cells. In one embodiment the experimental animal is immunized three times with primary cynomolgus PBLs, whereby the PBLs are optionally enriched for T cells.

It has been found that human cynomolgus cross-reactive antibodies, which activate T cells, can be generated by immunizing the experimental animal with an antigen that is in its native form, i.e. not denatured. Therefore, in one embodiment the method is without using a denaturing agent. In one embodiment the method is without using complete Freud's adjuvant.

It has been found that with the method as described herein, human cynomolgus cross-reactive antibodies can be found that specifically bind to human as well as cynomolgus CD3 epsilon and in addition are capable of activating T cells. T-cell activation can be shown using a calcium flux assay. Therefore, in one embodiment the human cynomolgus cross-reactive antibody as reported herein specifically binds to human and cynomolgus CD3 epsilon, to the polypeptide of SEQ ID NO: 01 and activates human T cells.

One aspect as reported herein is a method comprising the step of immunizing an experimental animal, three times with primary cynomolgus PBLs, whereby the PBLs are optionally enriched for T cells, without using primary human PBLs as immunogen and without using a denaturing agent for producing a human cynomolgus cross-reactive antibody, wherein the antibody specifically binds to human and cynomolgus T cells, to the polypeptide of SEQ ID NO: 01 and activates human T cells.

Another aspect as reported herein is a method comprising the step of immunizing an experimental animal, three times with primary cynomolgus PBLs, whereby the PBLs are optionally enriched for T cells, without using primary human PBLs as immunogen and without using a denaturing agent for producing a human cynomolgus cross-reactive antibody that specifically binds to human CD3 epsilon of SEQ ID NO: 02 and that specifically binds to a polypeptide of SEQ ID NO: 01, wherein the antibody specifically binds to human and cynomolgus T cells, activates human T cells and does not bind to the same epitope as the antibody OKT3, the antibody UCHT1 and/or the antibody SP34. Moreover, the antibody also does not bind to the same epitope as the antibody reported in WO 2007/042261.

Another aspect as reported herein is a method for recombinantly producing a human cynomolgus cross-reactive antibody comprising the following steps:
a) producing a human cynomolgus cross-reactive antibody with a method as reported herein,
b) providing a cell comprising the nucleic acid encoding the antibody produced in step a),
c) cultivating the cell of step b),
d) recovering the antibody from the cell or the cultivation supernatant,
and thereby recombinantly producing the human cynomolgus cross-reactive antibody.

Another aspect as reported herein is a method for recombinantly producing a human cynomolgus cross-reactive antibody comprising the following steps:
a) producing an antibody with a method as reported herein,
b) isolating the nucleic acid encoding the antibody produced in step a),
c) optionally humanizing the antibody,
d) cloning the nucleic acid encoding the antibody isolated in step b) or obtained in step c) in an expression vector,
e) transfecting a cell with the expression vector obtained in step d),
f) cultivating the cell of step e),
g) recovering the antibody from the cell or the cultivation supernatant,
and thereby recombinantly producing the human cynomolgus cross-reactive antibody.

Another aspect as reported herein is a human cynomolgus cross-reactive antibody that specifically binds to human CD3 epsilon of SEQ ID NO: 02 and that specifically binds to a polypeptide of SEQ ID NO: 01, wherein the human cynomolgus cross-reactive antibody specifically binds to human and cynomolgus T cells, to the polypeptide of SEQ ID NO: 01 and activates human T cells.

A further aspect as reported herein is a human cynomolgus cross-reactive antibody that specifically binds to human CD3 epsilon of SEQ ID NO: 02 and that specifically binds to a polypeptide of SEQ ID NO: 01, wherein the human cynomolgus cross-reactive antibody specifically binds to human and cynomolgus T cells, activates human T cells and does not bind to the same epitope as the antibody OKT3, the antibody UCHT1 and/or the antibody SP34.

In one embodiment the human cynomolgus cross-reactive antibody comprises (a) HVR-H3 comprising the amino acid sequence of SEQ ID NO: 09, (b) HVR-L3 comprising the amino acid sequence of SEQ ID NO: 13, and (c) HVR-H2 comprising one amino acid sequence selected from the group consisting of SEQ ID NO: 06 to SEQ ID NO: 08. In one embodiment the human cynomolgus cross-reactive antibody comprises (a) HVR-H1 comprising one amino acid sequence selected from the group consisting of SEQ ID NO: 04 to SEQ ID NO: 05, (b) HVR-H2 comprising one amino acid sequence selected from the group consisting of SEQ ID NO: 06 to SEQ ID NO: 08, and (c) HVR-H3 comprising one amino acid sequence of SEQ ID NO: 09. In one embodiment the human cynomolgus cross-reactive antibody comprises (a) HVR-L1 comprising one amino acid sequence selected from the group consisting of SEQ ID NO: 10 to SEQ ID NO: 11; (b) HVR-L2 comprising the amino acid sequence of SEQ ID NO: 12; and (c) HVR-L3 comprising the amino acid sequence of SEQ ID NO: 13. In one embodiment the human cynomolgus cross-reactive antibody comprises (a) HVR-H1 comprising the amino acid sequence of SEQ ID NO: 05, (b) HVR-H2 comprising the amino acid sequence of SEQ ID NO: 08; (c) HVR-H3 comprising the amino acid sequence of SEQ ID NO: 09; (d) HVR-L1 comprising the amino acid sequence of SEQ ID NO: 11; (e) HVR-L2 comprising the amino acid sequence of SEQ ID NO: 12; and (f) HVR-L3 comprising the amino acid sequence of SEQ ID NO: 13. In one embodiment the human cynomolgus cross-reactive antibody comprises (a) a VH sequence having at least 95% sequence identity to the amino acid sequence of SEQ ID NO: 14; (b) a VL sequence having at least 95% sequence identity to the amino acid sequence of SEQ ID NO: 15; or (c) a VH sequence as in (a) and a VL sequence as in (b). In one embodiment the human cynomolgus cross-reactive antibody comprises a VH sequence of SEQ ID NO: 14. In one embodiment the human cynomolgus cross-reactive antibody comprises a VL sequence of SEQ ID NO: 15. In one embodiment the human cynomolgus cross-reactive antibody comprises a VH sequence of SEQ ID NO: 14 and a VL sequence of SEQ ID NO: 15.

One aspect as reported herein is an immunoconjugate comprising the human cynomolgus cross-reactive antibody as reported herein and a cytotoxic agent.

One aspect as reported herein is a pharmaceutical formulation comprising the human cynomolgus cross-reactive antibody as reported herein and a pharmaceutically acceptable carrier.

One aspect as reported herein is a human cynomolgus cross-reactive antibody obtainable by a method comprising the step of immunizing an experimental animal with a native cynomolgus antigen as sole antigen.

One aspect as reported herein is a human cynomolgus cross-reactive antibody obtainable by immunizing an experimental animal, three times with primary cynomolgus PBLs, whereby the PBLs are optionally enriched for T cells, without using primary human PBLs as immunogen and without using a denaturing agent, wherein the human cynomolgus cross-reactive antibody specifically binds to human and cynomolgus T cells, to the polypeptide of SEQ ID NO: 01 and activates human T cells.

One aspect as reported herein is a human cynomolgus cross-reactive antibody that specifically binds to human CD3 epsilon of SEQ ID NO: 02 and that specifically binds to a polypeptide of SEQ ID NO: 01 obtainable by immunizing an experimental animal, three times with primary cynomolgus PBLs, whereby the PBLs are optionally enriched for T cells, without using primary human PBLs as immunogen and without using a denaturing agent, wherein the human cynomolgus cross-reactive antibody specifically binds to human and cynomolgus T cells, activates human T cells and does not bind to the same epitope as the antibody OKT3, the antibody UCHT1 and/or the antibody SP34.

A person skilled in the art is aware of the fact that if a cynomolgus derived antigen is used, the experimental animal has to be a non-cynomolgus experimental animal in order to obtain an immune response.

### Definitions

An "acceptor human framework" for the purposes herein is a framework comprising the amino acid sequence of a light chain variable domain (VL) framework or a heavy chain variable domain (VH) framework derived from a human immunoglobulin framework or a human consensus framework, as defined below. An acceptor human framework "derived from" a human immunoglobulin framework or a human consensus framework may comprise the same amino acid sequence thereof, or it may contain amino acid sequence changes. In some embodiments, the number of amino acid changes are 10 or less, 9 or less, 8 or less, 7 or less, 6 or less, 5 or less, 4 or less, 3 or less, or 2 or less. In some embodiments, the VL acceptor human framework is identical in sequence to the VL human immunoglobulin framework sequence or human consensus framework sequence.
The terms "anti-CD3 epsilon antibody" and "an antibody that binds to CD3 epsilon" refer to an antibody that is capable of binding CD3 epsilon with sufficient affinity such that the antibody is useful as a diagnostic and/or therapeutic agent in targeting CD3 epsilon. In one embodiment, the extent of binding of an anti-CD3 epsilon antibody to an unrelated, non-CD3 epsilon protein is less than about 10% of the binding of the antibody to human and/or cynomolgus CD3 epsilon as measured, e.g., by surface plasmon resonance (SPR).
The term "specifically binds" or "specifically binding" refers to binding of an antibody to an antigen with a K_{D} value of less than 10⁻⁵ M (M=mol/l) (e.g. 10⁻⁶ M) determined in a BIAcore assay (SPR).
The term "human CD3 epsilon" as used herein denotes the extracellular domain of the full length amino acid sequence of human CD3 epsilon, i.e. not including the signal sequence, the transmembrane domain or the cytoplasmic domain and has the amino acid sequence DGNEEMGGITQTPYKVSISGTTVILTCPQYPGSEILWQHNDKNIGGDEDDK NIGSDEDHLSLKEFSELEQSGYYVCYPRGSKPEDANFYLYLRARVCENCME MD (SEQ ID NO: 02).
The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.
An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; single-chain antibody molecules (e.g. scFv); and multispecific antibodies formed from antibody fragments.
The term "human cynomolgus cross-reactive antibody" refers to a molecule that binds specifically to a human antigen as well as to the corresponding cynomolgus antigen.
An "antibody that binds to the same epitope" as the antibody that binds to CD3 epsilon as reported herein refers to an antibody that binds to/interacts with the same amino acid residues of CD3 epsilon as determined e.g. by X-ray crystallography or in a peptide scan
The term "chimeric" antibody refers to an antibody in which a portion of the heavy and/or light chain is derived from a particular source or species, while the remainder of the heavy and/or light chain is derived from a different source or species.
The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG₁, IgG₂, IgG₃, IgG₄, IgA₁, and IgA₂. The heavy chain constant domains that correspond to the different classes of immunoglobulins are called α, δ, ε, γ, and µ, respectively.
The term "cytotoxic agent" as used herein refers to a substance that inhibits or prevents a cellular function and/or causes cell death or destruction. Cytotoxic agents include, but are not limited to, radioactive isotopes (e.g., At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu); chemotherapeutic agents or drugs (e.g., methotrexate, adriamicin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents disclosed below.
"Effector functions" refer to those biological activities attributable to the Fc region of an antibody, which vary with the antibody isotype. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (e.g. B cell receptor); and B cell activation.
An "effective amount" of an agent, e.g., a pharmaceutical formulation, refers to an amount effective, at dosages and for periods of time necessary, to achieve the desired therapeutic or prophylactic result.
The term "Fc region" herein is used to define a C-terminal region of an immunoglobulin heavy chain that contains at least a portion of the constant region. The term includes native sequence Fc regions and variant Fc regions. In one embodiment, a human IgG heavy chain Fc region extends from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat, E.A. et al., Sequences of Proteins of Immunological Interest, 5th ed., Public Health Service, National Institutes of Health, Bethesda, MD (1991), NIH Publication 91-3242.
"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.
The terms "full length antibody," "intact antibody," and "whole antibody" are used herein interchangeably to refer to an antibody having a structure substantially similar to a native antibody structure or having heavy chains that contain an Fc region as defined herein.
The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.
A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.
A "human consensus framework" is a framework which represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat, E.A. et al., Sequences of Proteins of Immunological Interest, 5th ed., Bethesda MD (1991), NIH Publication 91-3242, Vols. 1-3. In one embodiment, for the VL, the subgroup is subgroup kappa I as in Kabat et al., *supra.* In one embodiment, for the VH, the subgroup is subgroup III as in Kabat et al., *supra.*
A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.
The term "hypervariable region" or "HVR" as used herein refers to each of the regions of an antibody variable domain which are hypervariable in sequence ("complementarity determining regions" or "CDRs") and/or form structurally defined loops ("hypervariable loops") and/or contain the antigen-contacting residues ("antigen contacts"). Generally, antibodies comprise six HVRs: three in the VH (HI, H2, H3), and three in the VL (L1, L2, L3).
   Exemplary HVRs herein include:
   (a) hypervariable loops occurring at amino acid residues 26-32 (L1), 50-52 (L2), 91-96 (L3), 26-32 (HI), 53-55 (H2), and 96-101 (H3) (Chothia and Lesk, J. Mol. Biol. 196:901-917 (1987));
   (b) CDRs occurring at amino acid residues 24-34 (L1), 50-56 (L2), 89-97 (L3), 31-35b (HI), 50-65 (H2), and 95-102 (H3) (Kabat et al., Sequences of Proteins of Immunological Interest, 5th Ed. Public Health Service, National Institutes of Health, Bethesda, MD (1991));
   (c) antigen contacts occurring at amino acid residues 27c-36 (L1), 46-55 (L2), 89-96 (L3), 30-35b (HI), 47-58 (H2), and 93-101 (H3) (MacCallum et al. J. Mol. Biol. 262: 732-745 (1996)); and
   (d) combinations of (a), (b), and/or (c), including HVR amino acid residues 46-56 (L2), 47-56 (L2), 48-56 (L2), 49-56 (L2), 26-35 (HI), 26-35b (HI), 49-65 (H2), 93-102 (H3), and 94-102 (H3).
   Unless otherwise indicated, HVR residues and other residues in the variable domain (e.g., FR residues) are numbered herein according to Kabat et al., *supra.*
An "immunoconjugate" is an antibody conjugated to one or more heterologous molecule(s), including but not limited to a cytotoxic agent.
The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic experimental animals containing all or part of the human immunoglobulin loci, such methods and other exemplary methods for making monoclonal antibodies being described herein.
The term "peripheral blood lymphocytes" or "PBLs" as used herein denotes mature lymphocytes that circulate in the blood, rather than localizing to organs (such as the spleen or lymph nodes). PBLs comprise T cells, NK cells and B cells.
"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary.
   In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
   100 times the fraction X/Y
   where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.
The term "pharmaceutical formulation" refers to a preparation which is in such form as to permit the biological activity of an active ingredient contained therein to be effective, and which contains no additional components which are unacceptably toxic to a subject to which the formulation would be administered.
A "pharmaceutically acceptable carrier" refers to an ingredient in a pharmaceutical formulation, other than an active ingredient, which is nontoxic to a subject., A pharmaceutically acceptable carrier includes, but is not limited to, a buffer, excipient, stabilizer, or preservative.
The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, e.g., Kindt, T.J. et al. Kuby Immunology, 6th ed., W.H. Freeman and Co., N.Y. (2007), page 91) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, e.g., Portolano, S. et al., J. Immunol. 150 (1993) 880-887; Clackson, T. et al., Nature 352 (1991) 624-628).
The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors"

### A. Exemplary Anti-CD3 epsilon Antibodies

In one aspect, the invention provides isolated antibodies that bind to human and cynomolgus CD3 epsilon. In certain embodiments, an anti-CD3 epsilon antibody
- binds to the polypeptide of SEQ ID NO: 01, and/or
- binds to the ECD of human (SEQ ID NO: 02) and cynomolgus (SEQ ID NO: 28) CD3 epsilon, and/or
- activates human and/or cynomolgus T cells, and/or
- is an agonist of CD3 epsilon, and/or
- binds with an affinity of ≤ 10 µM to its antigen.

In one embodiment the human cynomolgus cross-reactive antibody comprises (a) HVR-H3 comprising the amino acid sequence of SEQ ID NO: 09, (b) HVR-L3 comprising the amino acid sequence of SEQ ID NO: 13, and (c) HVR-H2 comprising one amino acid sequence selected from the group consisting of SEQ ID NO: 06 to SEQ ID NO: 08. In one embodiment the human cynomolgus cross-reactive antibody comprises (a) HVR-H1 comprising one amino acid sequence selected from the group consisting of SEQ ID NO: 04 to SEQ ID NO: 05, (b) HVR-H2 comprising one amino acid sequence selected from the group consisting of SEQ ID NO: 06 to SEQ ID NO: 08, and (c) HVR-H3 comprising one amino acid sequence of SEQ ID NO: 09. In one embodiment the human cynomolgus cross-reactive antibody comprises (a) HVR-L1 comprising one amino acid sequence selected from the group consisting of SEQ ID NO: 10 to SEQ ID NO: 11; (b) HVR-L2 comprising the amino acid sequence of SEQ ID NO: 12; and (c) HVR-L3 comprising the amino acid sequence of SEQ ID NO: 13. In one embodiment the human cynomolgus cross-reactive antibody comprises (a) HVR-H1 comprising the amino acid sequence of SEQ ID NO: 05, (b) HVR-H2 comprising the amino acid sequence of SEQ ID NO: 08; (c) HVR-H3 comprising the amino acid sequence of SEQ ID NO: 09; (d) HVR-L1 comprising the amino acid sequence of SEQ ID NO: 11; (e) HVR-L2 comprising the amino acid sequence of SEQ ID NO: 12; and (f) HVR-L3 comprising the amino acid sequence of SEQ ID NO: 13. In one embodiment the human cynomolgus cross-reactive antibody comprises (a) a VH sequence having at least 95% sequence identity to the amino acid sequence of SEQ ID NO: 14; (b) a VL sequence having at least 95% sequence identity to the amino acid sequence of SEQ ID NO: 15; or (c) a VH sequence as in (a) and a VL sequence as in (b). In one embodiment the human cynomolgus cross-reactive antibody comprises a VH sequence of SEQ ID NO: 14. In one embodiment the human cynomolgus cross-reactive antibody comprises a VL sequence of SEQ ID NO: 15. In one embodiment the human cynomolgus cross-reactive antibody comprises a VH sequence of SEQ ID NO: 14 and a VL sequence of SEQ ID NO: 15.

In a further embodiment, an anti-CD3 epsilon antibody according to any of the above embodiments is a monoclonal antibody, including a chimeric, humanized or human antibody.

In one embodiment, an anti-CD3 epsilon antibody is an antibody fragment, e.g., a Fv, Fab, Fab', scFv, diabody, or F(ab')₂ fragment.

In another embodiment, the antibody is a full length antibody, e.g., an intact IgG1 antibody or other antibody class or isotype as defined herein.

In a further embodiment, an anti-CD3 epsilon antibody according to any of the above embodiments may incorporate any of the features, singly or in combination, as described in Sections 1-7 below:

### 1. Antibody Affinity

In certain embodiments, an antibody provided herein has a dissociation constant (Kd) of ≤ 100 µM or ≤ 10 µM, (e.g. 10⁻⁵ M or less).

According to another embodiment, the Kd value is measured using surface plasmon resonance assays using a BIACORE®-T100 (GE Healthcare) at 25°C with immobilized antigen on a CM4 chip. For example, around 2000 resonance units (RU) of the capturing system (10 µg/ml goat anti rabbit IgG Fc Fragment specific; Order Code: 111-005-046; Jackson Immuno Research) are coupled on a CM4 chip (GE Healthcare, BR-1005-34) at pH 5.0 by using an amine coupling kit supplied by the GE Healthcare. Running buffer for Immobilization was HBS-N pH 7.4 (10 mM HEPES, 150 mM NaCl, pH 7.4, GE Healthcare, BR-1006-70). For the followed kinetic assay running and dilution buffer is HBS-P pH 7.4 (10 mM HEPES, 150 mM NaCl, 0.05% Surfactant P20, pH 7.4, GE Healthcare, BR-1006-71). The flow cell is set to 25 °C - and the sample block set to 12 °C - and primed with running buffer twice. The clone 645 antibody is captured by injecting a 1 µg/ml solution for 60 sec at a flow of 10 µl/min. Association is measured by injection of human CD3e(stalk)Fc-Knob-CD3d(stalk)FcHole or cynomolgus CD3e(stalk)Fc-Knob-CD3d(stalk)FcHole in various concentrations in solution for 180 sec at a flow of 30 µl/min starting with 1350 nM, followed by one 1:1.5 dilution and further in 1:3 dilutions. The dissociation phase is monitored for up to 300 sec and triggered by switching from the sample solution to running buffer. The surface is regenerated by washing with two consecutive injections of a Glycine pH 1.7 solution for 60 sec at a flow rate of 10 µl/min. Bulk refractive index differences are corrected by subtracting the response obtained from a goat anti rabbit IgG Fc surface. Blank injections are also subtracted (= double referencing). Association rates (kₒₙ) and dissociation rates (k_{off}) are calculated using a simple one-to-one Langmuir binding model (BIACORE® Evaluation Software version 3.2) by simultaneously fitting the association and dissociation sensorgrams. The equilibrium dissociation constant (Kd) is calculated as the ratio k_{off}/k_{on.} See, e.g., Chen, Y. et al., J. Mol. Biol. 293 (1999) 865-881.

### 2. Antibody Fragments

In certain embodiments, an antibody provided herein is an antibody fragment. Antibody fragments include, but are not limited to, Fab, Fab', Fab'-SH, F(ab')₂, Fv, and scFv fragments, and other fragments described below. For a review of certain antibody fragments, see Hudson, P.J. et al., Nat. Med. 9 (2003) 129-134. For a review of scFv fragments, see, e.g., Plueckthun, A., In; The Pharmacology of Monoclonal Antibodies, Vol. 113, Rosenburg and Moore (eds.), Springer-Verlag, New York (1994), pp. 269-315; see also WO 93/16185; and U.S. Patent Nos. 5,571,894 and 5,587,458. For discussion of Fab and F(ab')₂ fragments comprising salvage receptor binding epitope residues and having increased in vivo half-life, see U.S. Patent No. 5,869,046.

Diabodies are antibody fragments with two antigen-binding sites that may be bivalent or bispecific. See, for example, EP 0 404 097; WO 1993/01161; Hudson, P.J. et al., Nat. Med. 9 (2003) 129-134; and Holliger, P. et al., Proc. Natl. Acad. Sci. USA 90 (1993) 6444-6448. Triabodies and tetrabodies are also described in Hudson, P.J. et al., Nat. Med. 9 (20039 129-134).

Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; *see,* e.g., U.S. Patent No. 6,248,516 B1).

Antibody fragments can be made by various techniques, including but not limited to proteolytic digestion of an intact antibody as well as production by recombinant host cells (e.g. *E. coli* or phage), as described herein.

### 3. Chimeric and Humanized Antibodies

In certain embodiments, an antibody provided herein is a chimeric antibody. Certain chimeric antibodies are described, e.g., in U.S. Patent No. 4,816,567; and Morrison, S.L. et al., Proc. Natl. Acad. Sci. USA 81 (1984) 6851-6855). In one example, a chimeric antibody comprises a non-human variable region (e.g., a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain embodiments, a chimeric antibody is a humanized antibody. Typically, a non-human antibody is humanized to reduce immunogenicity to humans, while retaining the specificity and affinity of the parental non-human antibody.

Generally, a humanized antibody comprises one or more variable domains in which HVRs, e.g., CDRs, (or portions thereof) are derived from a non-human antibody, and FRs (or portions thereof) are derived from human antibody sequences. A humanized antibody optionally will also comprise at least a portion of a human constant region. In some embodiments, some FR residues in a humanized antibody are substituted with corresponding residues from a non-human antibody (e.g., the antibody from which the HVR residues are derived), e.g., to restore or improve antibody specificity or affinity.

Humanized antibodies and methods of making them are reviewed, e.g., in Almagro, J.C. and Fransson, J., Front. Biosci. 13 (2008) 1619-1633, and are further described, e.g., in Riechmann, I. et al., Nature 332 (1988) 323-329; Queen, C. et al., Proc. Natl. Acad. Sci. USA 86 (1989) 10029-10033; US Patent Nos. 5, 821,337, 7,527,791, 6,982,321, and 7,087,409; Kashmiri, S.V. et al., Methods 36 (2005) 25-34 (describing SDR (a-CDR) grafting); Padlan, E.A., Mol. Immunol. 28 (1991) 489-498 (describing "resurfacing"); Dall'Acqua, W.F. et al., Methods 36 (2005) 43-60 (describing "FR shuffling"); and Osbourn, J. et al., Methods 36 (2005) 61-68 and Klimka, A. et al., Br. J. Cancer 83 (2000) 252-260 (describing the "guided selection" approach to FR shuffling).

Human framework regions that may be used for humanization include but are not limited to: framework regions selected using the "best-fit" method (see, e.g., Sims, M.J. et al., J. Immunol. 151 (1993) 2296-2308; framework regions derived from the consensus sequence of human antibodies of a particular subgroup of light or heavy chain variable regions (see, e.g., Carter, P. et al., Proc. Natl. Acad. Sci. USA 89 (1992) 4285-4289; and Presta, L.G. et al., J. Immunol. 151 (1993) 2623-2632); human mature (somatically mutated) framework regions or human germline framework regions (see, e.g., Almagro, J.C. and Fransson, J., Front. Biosci. 13 (2008) 1619-1633); and framework regions derived from screening FR libraries (see, e.g., Baca, M. et al., J. Biol. Chem. 272 (1997) 10678-10684 and Rosok, M.J. et al., J. Biol. Chem. 271 (19969 22611-22618).

### 4. Human Antibodies

In certain embodiments, an antibody provided herein is a human antibody. Human antibodies can be produced using various techniques known in the art. Human antibodies are described generally in van Dijk, M.A. and van de Winkel, J.G., Curr. Opin. Pharmacol. 5 (2001) 368-374 and Lonberg, N., Curr. Opin. Immunol. 20 (2008) 450-459.

Human antibodies may be prepared by administering an immunogen to a transgenic experimental animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, N., Nat. Biotech. 23 (2005) 1117-1125. See also, e.g., U.S. Patent Nos. 6,075,181 and 6,150,584 describing XENOMOUSE™ technology; U.S. Patent No. 5,770,429 describing HUMAB® technology; U.S. Patent No. 7,041,870 describing K-M MOUSE® technology, and U.S. Patent Application Publication No. US 2007/0061900, describing VELOCIMOUSE® technology). Human variable regions from intact antibodies generated by such animals may be further modified, e.g., by combining with a different human constant region.

Human antibodies can also be made by hybridoma-based methods. Human myeloma and mouse-human heteromyeloma cell lines for the production of human monoclonal antibodies have been described. (See, e.g., Kozbor, D., J. Immunol. 133 (1984) 3001-3005; Brodeur, B.R. et al., Monoclonal Antibody Production Techniques and Applications, Marcel Dekker, Inc., New York (1987), pp. 51-63; and Boerner, P. et al., J. Immunol. 147 (1991) 86-95) Human antibodies generated via human B-cell hybridoma technology are also described in Li, J. et al., Proc. Natl. Acad. Sci. USA 103 (2006) 3557-3562. Additional methods include those described, for example, in U.S. Patent No. 7,189,826 (describing production of monoclonal human IgM antibodies from hybridoma cell lines) and Ni, J., Xiandai Mianyixue 26 (2006) 265-268 (describing human-human hybridomas). Human hybridoma technology (Trioma technology) is also described in Vollmers, H.P. and Brandlein, S., Histology and Histopathology 20 (2005) 927-937 and Vollmers, H.P. and Brandlein, S., Methods and Findings in Experimental and Clinical Pharmacology 27 (2005) 185-191.

Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

### 5. Library-Derived Antibodies

Antibodies of the invention may be isolated by screening combinatorial libraries for antibodies with the desired activity or activities. For example, a variety of methods are known in the art for generating phage display libraries and screening such libraries for antibodies possessing the desired binding characteristics. Such methods are reviewed, e.g., in Hoogenboom, H.R. et al., Methods in Molecular Biology 178 (2001) 1-37 and further described, e.g., in the McCafferty, J. et al., Nature 348 (1990) 552-554; Clackson, T. et al., Nature 352 (1991) 624-628; Marks, J.D. et al., J. Mol. Biol. 222 (1992) 581-597; Marks, J.D. and Bradbury, A., Methods in Molecular Biology 248 (2003) 161-175; Sidhu, S.S. et al., J. Mol. Biol. 338 (2004) 299-310; Lee, C.V. et al., J. Mol. Biol. 340 (2004) 1073-1093; Fellouse, F.A., Proc. Natl. Acad. Sci. USA 101 (2004) 12467-12472; and Lee, C.V. et al., J. Immunol. Methods 284 (2004) 119-132.

In certain phage display methods, repertoires of VH and VL genes are separately cloned by polymerase chain reaction (PCR) and recombined randomly in phage libraries, which can then be screened for antigen-binding phage as described in Winter, G. et al., Ann. Rev. Immunol. 12 (1994) 433-455. Phage typically display antibody fragments, either as single-chain Fv (scFv) fragments or as Fab fragments. Libraries from immunized sources provide high-affinity antibodies to the immunogen without the requirement of constructing hybridomas. Alternatively, the naive repertoire can be cloned (e.g., from human) to provide a single source of antibodies to a wide range of non-self and also self-antigens without any immunization as described by Griffiths, A.D. et al., EMBO J. 12 (1993) 725-734. Finally, naive libraries can also be made synthetically by cloning non-rearranged V-gene segments from stem cells, and using PCR primers containing random sequence to encode the highly variable CDR3 regions and to accomplish rearrangement *in vitro,* as described by Hoogenboom, H.R. and Winter, G., J. Mol. Biol. 227 (1992) 381-388. Patent publications describing human antibody phage libraries include, for example: US Patent No. 5,750,373, and US Patent Publication Nos. 2005/0079574, 2005/0119455, 2005/0266000, 2007/0117126, 2007/0160598, 2007/0237764, 2007/0292936, and 2009/0002360.

Antibodies or antibody fragments isolated from human antibody libraries are considered human antibodies or human antibody fragments herein.

### 6. Multispecific Antibodies

In certain embodiments, an antibody provided herein is a multispecific antibody, e.g. a bispecific antibody. Multispecific antibodies are monoclonal antibodies that have binding specificities for at least two different sites. In certain embodiments, one of the binding specificities is for CD3 epsilon and the other is for any other antigen. In certain embodiments, bispecific antibodies may bind to two different epitopes of CD3 epsilon. Bispecific antibodies may also be used to localize cytotoxic agents to cells which express CD3 epsilon. Bispecific antibodies can be prepared as full length antibodies or antibody fragments.

Techniques for making multispecific antibodies include, but are not limited to, recombinant co-expression of two immunoglobulin heavy chain-light chain pairs having different specificities (see Milstein, C. and Cuello, A.C., Nature 305 (1983) 537-540, WO 93/08829, and Traunecker, A. et al., EMBO J. 10 (1991) 3655-3659), and "knob-in-hole" engineering (see, e.g., U.S. Patent No. 5,731,168). Multi-specific antibodies may also be made by engineering electrostatic steering effects for making antibody Fc-heterodimeric molecules (WO 2009/089004); crosslinking two or more antibodies or fragments (see, e.g., US Patent No. 4,676,980, and Brennan, M. et al., Science 229 (1985) 81-83); using leucine zippers to produce bi-specific antibodies (see, e.g., Kostelny, S.A. et al., J. Immunol. 148 (1992) 1547-1553; using "diabody" technology for making bispecific antibody fragments (see, e.g., Holliger, P. et al., Proc. Natl. Acad. Sci. USA 90 (1993) 6444-6448); and using single-chain Fv (sFv) dimers (see, e.g. Gruber, M et al., J. Immunol. 152 (1994) 5368-5374); and preparing trispecific antibodies as described, e.g., in Tutt, A. et al., J. Immunol. 147 (1991) 60-69).

Engineered antibodies with three or more functional antigen binding sites, including "Octopus antibodies," are also included herein (see, e.g. US 2006/0025576).

The antibody or fragment herein also includes a "Dual Acting Fab" or "DAF" comprising an antigen binding site that binds to CD3 epsilon as well as another, different antigen (see, US 2008/0069820, for example).

The antibody or fragment herein also includes multispecific antibodies described in WO 2009/080251, WO 2009/080252, WO 2009/080253, WO 2009/080254, WO 2010/112193, WO 2010/115589, WO 2010/136172, WO 2010/145792, and WO 2010/145793.

### 7. Antibody Variants

In certain embodiments, amino acid sequence variants of the antibodies provided herein are contemplated. For example, it may be desirable to improve the binding affinity and/or other biological properties of the antibody. Amino acid sequence variants of an antibody may be prepared by introducing appropriate modifications into the nucleotide sequence encoding the antibody, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, e.g., antigen-binding.

### a) Substitution, Insertion, and Deletion Variants

In certain embodiments, antibody variants having one or more amino acid substitutions are provided. Sites of interest for substitutional mutagenesis include the HVRs and FRs. Exemplary changes are provided in Table 1 under the heading of "exemplary substitutions", and as further described below in reference to amino acid side chain classes. Conservative substitutions are shown in Table 1 under the heading of "preferred substitutions". Amino acid substitutions may be introduced into an antibody of interest and the products screened for a desired activity, e.g., retained/improved antigen binding, decreased immunogenicity, or improved ADCC or CDC.

**TABLE 1**

| **Original Residue** | **Exemplary Substitutions** | **Preferred Substitutions** |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

One type of substitutional variant involves substituting one or more hypervariable region residues of a parent antibody (e.g. a humanized or human antibody). Generally, the resulting variant(s) selected for further study will have modifications (e.g., improvements) in certain biological properties (e.g., increased affinity, reduced immunogenicity) relative to the parent antibody and/or will have substantially retained certain biological properties of the parent antibody. An exemplary substitutional variant is an affinity matured antibody, which may be conveniently generated, e.g., using phage display-based affinity maturation techniques such as those described herein. Briefly, one or more HVR residues are mutated and the variant antibodies displayed on phage and screened for a particular biological activity (e.g. binding affinity).

Alterations (e.g., substitutions) may be made in HVRs, e.g., to improve antibody affinity. Such alterations may be made in HVR "hotspots," i.e., residues encoded by codons that undergo mutation at high frequency during the somatic maturation process (see, e.g., Chowdhury, P.S., Methods Mol. Biol. 207 (2008) 179-196), and/or SDRs (a-CDRs), with the resulting variant VH or VL being tested for binding affinity. Affinity maturation by constructing and reselecting from secondary libraries has been described, e.g., in Hoogenboom, H.R. et al. in Methods in Molecular Biology 178 (2002) 1-37. In some embodiments of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (e.g., 4-6 residues at a time) are randomized. HVR residues involved in antigen binding may be specifically identified, e.g., using alanine scanning mutagenesis or modeling. CDR-H3 and CDR-L3 in particular are often targeted.

In certain embodiments, substitutions, insertions, or deletions may occur within one or more HVRs so long as such alterations do not substantially reduce the ability of the antibody to bind antigen. For example, conservative alterations (e.g., conservative substitutions as provided herein) that do not substantially reduce binding affinity may be made in HVRs. Such alterations may be outside of HVR "hotspots" or SDRs. In certain embodiments of the variant VH and VL sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham, B.C. and Wells, J.A., Science 244 (1989) 1081-1085. In this method, a residue or group of target residues (e.g., charged residues such as arg, asp, his, lys, and glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the N- or C-terminus of the antibody to an enzyme (e.g. for ADEPT) or a polypeptide which increases the serum half-life of the antibody.

### b) Glycosylation variants

In certain embodiments, an antibody provided herein is altered to increase or decrease the extent to which the antibody is glycosylated. Addition or deletion of glycosylation sites to an antibody may be conveniently accomplished by altering the amino acid sequence such that one or more glycosylation sites is created or removed.

Where the antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, e.g., Wright, A. and Morrison, S.L., TIBTECH 15 (1997) 26-32. The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antibody of the invention may be made in order to create antibody variants with certain improved properties.

In one embodiment, antibody variants are provided having a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry, as described in WO 2008/077546, for example. Asn297 refers to the asparagine residue located at about position 297 in the Fc region (Eu numbering of Fc region residues); however, Asn297 may also be located about ± 3 amino acids upstream or downstream of position 297, i.e., between positions 294 and 300, due to minor sequence variations in antibodies. Such fucosylation variants may have improved ADCC function. See, e.g., US 2003/0157108; US 2004/0093621. Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO 2005/053742; WO 2002/031140; Okazaki, A. et al., J. Mol. Biol. 336 (2004) 1239-1249; Yamane-Ohnuki, N. et al., Biotech. Bioeng. 87 (2004) 614-622. Examples of cell lines capable of producing defucosylated antibodies include Lec13 CHO cells deficient in protein fucosylation (Ripka, J. et al., Arch. Biochem. Biophys. 249 (1986) 533-545; US 2003/0157108; and WO 2004/056312, especially at Example 11), and knockout cell lines, such as alpha-1,6-fucosyltransferase gene, *FUT8,* knockout CHO cells (see, e.g., Yamane-Ohnuki, N. et al., Biotech. Bioeng. 87 (2004) 614-622; Kanda, Y. et al., Biotechnol. Bioeng. 94 (2006) 680-688; and WO 2003/085107).

Antibodies variants are further provided with bisected oligosaccharides, e.g., in which a biantennary oligosaccharide attached to the Fc region of the antibody is bisected by GlcNAc. Such antibody variants may have reduced fucosylation and/or improved ADCC function. Examples of such antibody variants are described, e.g., in WO 2003/011878; US Patent No. 6,602,684; and US 2005/0123546. Antibody variants with at least one galactose residue in the oligosaccharide attached to the Fc region are also provided. Such antibody variants may have improved CDC function. Such antibody variants are described, e.g., in WO 1997/30087; WO 1998/58964; and WO 1999/22764.

### c) Fc region variants

In certain embodiments, one or more amino acid modifications may be introduced into the Fc region of an antibody provided herein, thereby generating an Fc region variant. The Fc region variant may comprise a human Fc region sequence (e.g., a human IgG1, IgG2, IgG3 or IgG4 Fc region) comprising an amino acid modification (*e.g*. a substitution) at one or more amino acid positions.

In certain embodiments, the invention contemplates an antibody variant that possesses some but not all effector functions, which make it a desirable candidate for applications in which the half-life of the antibody *in vivo* is important yet certain effector functions (such as complement and ADCC) are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the antibody lacks FcγR binding (hence likely lacking ADCC activity), but retains FcRn binding ability. The primary cells for mediating ADCC, NK cells, express Fc(RIII only, whereas monocytes express FcγRI, FcγRII and FcγRIII. FcR expression on hematopoietic cells is summarized in Table 3 on page 464 of Ravetch, J.V. and Kinet, J.P., Annu. Rev. Immunol. 9 (1991) 457-492. Non-limiting examples of *in vitro* assays to assess ADCC activity of a molecule of interest is described in U.S. Patent No. 5,500,362 (see, e.g. Hellstrom, I. et al., Proc. Natl. Acad. Sci. USA 83 (1986) 7059-7063; and Hellstrom, I. et al., Proc. Natl. Acad. Sci. USA 82 (1985) 1499-1502); U.S. Patent No. 5,821,337 (see Bruggemann, M. et al., J. Exp. Med. 166 (1987) 1351-1361). Alternatively, non-radioactive assays methods may be employed (see, for example, ACTI™ non-radioactive cytotoxicity assay for flow cytometry (CellTechnology, Inc. Mountain View, CA; and CytoTox 96® non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed *in vivo, e.g.,* in an animal model such as that disclosed in Clynes, R. et al., Proc. Natl. Acad. Sci. USA 95 (1998) 652-656. C1q binding assays may also be carried out to confirm that the antibody is unable to bind C1q and hence lacks CDC activity. See, e.g., C1q and C3c binding ELISA in WO 2006/029879 and WO 2005/100402. To assess complement activation, a CDC assay may be performed (see, for example, Gazzano-Santoro, H. et al., J. Immunol. Methods 202 (1996) 163-171; Cragg, M.S. et al., Blood 101 (2003) 1045-1052; and Cragg, M.S. and M.J. Glennie, Blood 103 (2004) 2738-2743). FcRn binding and *in vivo* clearance/half-life determinations can also be performed using methods known in the art (see, e.g., Petkova, S.B. et al., Int. Immunol. 18 (2006: 1759-1769).

Antibodies with reduced effector function include those with substitution of one or more of Fc region residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Certain antibody variants with improved or diminished binding to FcRs are described. (See, e.g., U.S. Patent No. 6,737,056; WO 2004/056312, and Shields, R.L. et al., J. Biol. Chem. 276 (2001) 6591-6604)

In certain embodiments, an antibody variant comprises an Fc region with one or more amino acid substitutions which improve ADCC, e.g., substitutions at positions 298, 333, and/or 334 of the Fc region (EU numbering of residues).

In some embodiments, alterations are made in the Fc region that result in altered (*i.e.,* either improved or diminished) C1q binding and/or Complement Dependent Cytotoxicity (CDC), e.g., as described in US Patent No. 6,194,551, WO 99/51642, and Idusogie, E.E. et al., J. Immunol. 164 (2000) 4178-4184.

Antibodies with increased half-lives and improved binding to the neonatal Fc receptor (FcRn), which is responsible for the transfer of maternal IgGs to the fetus (Guyer, R.L. et al., J. Immunol. 117 (1976) 587-593, and Kim, J.K. et al., J. Immunol. 24 (1994) 2429-2434), are described in US 2005/0014934. Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, e.g., substitution of Fc region residue 434 (US Patent No. 7,371,826).

See also Duncan, A.R. and Winter, G., Nature 322 (1988) 738-740; US 5,648,260; US 5,624,821; and WO 94/29351 concerning other examples of Fc region variants.

### d) Cysteine engineered antibody variants

In certain embodiments, it may be desirable to create cysteine engineered antibodies, e.g., "thioMAbs," in which one or more residues of an antibody are substituted with cysteine residues. In particular embodiments, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described further herein. In certain embodiments, any one or more of the following residues may be substituted with cysteine: V205 (Kabat numbering) of the light chain; A118 (EU numbering) of the heavy chain; and S400 (EU numbering) of the heavy chain Fc region. Cysteine engineered antibodies may be generated as described, e.g., in U.S. Patent No. 7,521,541.

### e) Antibody Derivatives

In certain embodiments, an antibody provided herein may be further modified to contain additional non-proteinaceous moieties that are known in the art and readily available. The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, polypropylene oxide/ethylene oxide copolymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer is attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another embodiment, conjugates of an antibody and non-proteinaceous moiety that may be selectively heated by exposure to radiation are provided. In one embodiment, the non-proteinaceous moiety is a carbon nanotube (Kam, N.W. et al., Proc. Natl. Acad. Sci. USA 102 (2005) 11600-11605). The radiation may be of any wavelength, and includes, but is not limited to, wavelengths that do not harm ordinary cells, but which heat the non-proteinaceous moiety to a temperature at which cells proximal to the antibody-non-proteinaceous moiety are killed.

### Recombinant Methods and Compositions

Antibodies may be produced using recombinant methods and compositions, e.g., as described in U.S. Patent No. 4,816,567. In one embodiment, isolated nucleic acid encoding an anti-CD3 epsilon antibody described herein is provided. Such nucleic acid may encode an amino acid sequence comprising the VL and/or an amino acid sequence comprising the VH of the antibody (e.g., the light and/or heavy chains of the antibody). In a further embodiment, one or more vectors (e.g., expression vectors) comprising such nucleic acid are provided. In a further embodiment, a host cell comprising such nucleic acid is provided. In one such embodiment, a host cell comprises (e.g., has been transformed with): (1) a vector comprising a nucleic acid that encodes an amino acid sequence comprising the VL of the antibody and an amino acid sequence comprising the VH of the antibody, or (2) a first vector comprising a nucleic acid that encodes an amino acid sequence comprising the VL of the antibody and a second vector comprising a nucleic acid that encodes an amino acid sequence comprising the VH of the antibody. In one embodiment, the host cell is eukaryotic, e.g. a Chinese Hamster Ovary (CHO) cell or lymphoid cell (e.g., Y0, NS0, Sp20 cell). In one embodiment, a method of making an anti-CD3 epsilon antibody is provided, wherein the method comprises culturing a host cell comprising a nucleic acid encoding the antibody, as provided above, under conditions suitable for expression of the antibody, and optionally recovering the antibody from the host cell (or host cell culture medium).

For recombinant production of an anti-CD3 epsilon antibody, nucleic acid encoding an antibody, e.g., as described above, is isolated and inserted into one or more vectors for further cloning and/or expression in a host cell. Such nucleic acid may be readily isolated and sequenced using conventional procedures (e.g., by using oligonucleotide probes that are capable of binding specifically to genes encoding the heavy and light chains of the antibody).

Suitable host cells for cloning or expression of antibody-encoding vectors include prokaryotic or eukaryotic cells described herein. For example, antibodies may be produced in bacteria, in particular when glycosylation and Fc effector function are not needed. For expression of antibody fragments and polypeptides in bacteria, see, e.g., US 5,648,237, US 5,789,199, and US 5,840,523. (See also Charlton, K.A., In: Methods in Molecular Biology, Vol. 248, Lo, B.K.C. (ed.), Humana Press, Totowa, NJ (2003), pp. 245-254, describing expression of antibody fragments in *E. coli*.) After expression, the antibody may be isolated from the bacterial cell paste in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. See Gerngross, T.U., Nat. Biotech. 22 (2004) 1409-1414; and Li, H. et al., Nat. Biotech. 24 (2006) 210-215.

Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells.

Plant cell cultures can also be utilized as hosts. *See,* e.g., US Patent Nos. 5,959,177, 6,040,498, 6,420,548, 7,125,978, and 6,417,429 (describing PLANTIBODIES™ technology for producing antibodies in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, e.g., in Graham, F.L. et al., J. Gen Virol. 36 (1977) 59-74); baby hamster kidney cells (BHK); mouse sertoli cells (TM4 cells as described, e.g., in Mather, J.P., Biol. Reprod. 23 (1980) 243-252); monkey kidney cells (CV1); African green monkey kidney cells (VERO-76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, e.g., in Mather, J.P. et al., Annals N.Y. Acad. Sci. 383 (1982) 44-68; MRC 5 cells; and FS4 cells. Other useful mammalian host cell lines include Chinese hamster ovary (CHO) cells, including DHFR⁻ CHO cells (Urlaub, G. et al., Proc. Natl. Acad. Sci. USA 77 (1980) 4216-4220); and myeloma cell lines such as Y0, NS0 and Sp2/0. For a review of certain mammalian host cell lines suitable for antibody production, see, e.g., Yazaki, P. and Wu, A.M., Methods in Molecular Biology, Vol. 248, Lo, B.K.C. (ed.), Humana Press, Totowa, NJ (2004), pp. 255-268.

### Pharmaceutical Formulations

Pharmaceutical formulations of an anti-CD3 epsilon antibody as described herein are prepared by mixing such antibody having the desired degree of purity with one or more optional pharmaceutically acceptable carriers (Remington's Pharmaceutical Sciences, 16th edition, Osol, A. (ed.) (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyl dimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as poly(vinylpyrrolidone); amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g*. Zn-protein complexes); and/or non-ionic surfactants such as polyethylene glycol (PEG). Exemplary pharmaceutically acceptable carriers herein further include interstitial drug dispersion agents such as soluble neutral-active hyaluronidase glycoproteins (sHASEGP), for example, human soluble PH-20 hyaluronidase glycoproteins, such as rhuPH20 (HYLENEX®, Baxter International, Inc.). Certain exemplary sHASEGPs and methods of use, including rhuPH20, are described in US Patent Publication Nos. 2005/0260186 and 2006/0104968. In one aspect, a sHASEGP is combined with one or more additional glycosaminoglycanases such as chondroitinases.

Exemplary lyophilized antibody formulations are described in US Patent No. 6,267,958. Aqueous antibody formulations include those described in US Patent No. 6,171,586 and WO 2006/044908, the latter formulations including a histidine-acetate buffer.

The formulation herein may also contain more than one active ingredients as necessary for the particular indication being treated, preferably those with complementary activities that do not adversely affect each other. Such active ingredients are suitably present in combination in amounts that are effective for the purpose intended.

Active ingredients may be entrapped in microcapsules prepared, for example, by coacervation techniques or by interfacial polymerization, for example, hydroxymethylcellulose or gelatin-microcapsules and poly-(methyl methacrylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences, 16th edition, Osol, A. (ed.) (1980).

Sustained-release preparations may be prepared. Suitable examples of sustained-release preparations include semi-permeable matrices of solid hydrophobic polymers containing the antibody, which matrices are in the form of shaped articles, e.g. films, or microcapsules.

The formulations to be used for *in vivo* administration are generally sterile. Sterility may be readily accomplished, e.g., by filtration through sterile filtration membranes.

### C. Assays

Anti-CD3 epsilon antibodies provided herein may be identified, screened for, or characterized for their physical/chemical properties and/or biological activities by various assays known in the art.

### 1. Binding assays and other assays

In one aspect, an antibody of the invention is tested for its antigen binding activity, e.g., by known methods such as ELISA, Western blot, etc.

In another aspect, competition assays may be used to identify an antibody that competes with the antibody produced by clone 645 for binding to CD3 epsilon. In certain embodiments, such a competing antibody binds to the same epitope (e.g., a linear or a conformational epitope) that is bound by the antibody produced by clone 645. Detailed exemplary methods for mapping an epitope to which an antibody binds are provided in Morris, G.E. (ed.), Epitope Mapping Protocols, In: Methods in Molecular Biology, Vol. 66, Humana Press, Totowa, NJ (1996).

In an exemplary competition assay, immobilized CD3 epsilon is incubated in a solution comprising a first labeled antibody that binds to CD3 epsilon (e.g., the antibody produced by clone 645 and a second unlabeled antibody that is being tested for its ability to compete with the first antibody for binding to CD3 epsilon. The second antibody may be present in a hybridoma supernatant. As a control, immobilized CD3 epsilon is incubated in a solution comprising the first labeled antibody but not the second unlabeled antibody. After incubation under conditions permissive for binding of the first antibody to CD3 epsilon, excess unbound antibody is removed, and the amount of label associated with immobilized CD3 epsilon is measured. If the amount of label associated with immobilized CD3 epsilon is substantially reduced in the test sample relative to the control sample, then that indicates that the second antibody is competing with the first antibody for binding to CD3 epsilon. See Harlow, E. and Lane, D., Antibodies: A Laboratory Manual, Chapter 14, Cold Spring Harbor Laboratory, Cold Spring Harbor, NY (1988).

### 2. Activity assays

In one aspect, assays are provided for identifying anti-CD3 epsilon antibodies thereof having biological activity. Biological activity may include, e.g., activation of T cells. Antibodies having such biological activity in vivo and/or in vitro are also provided.

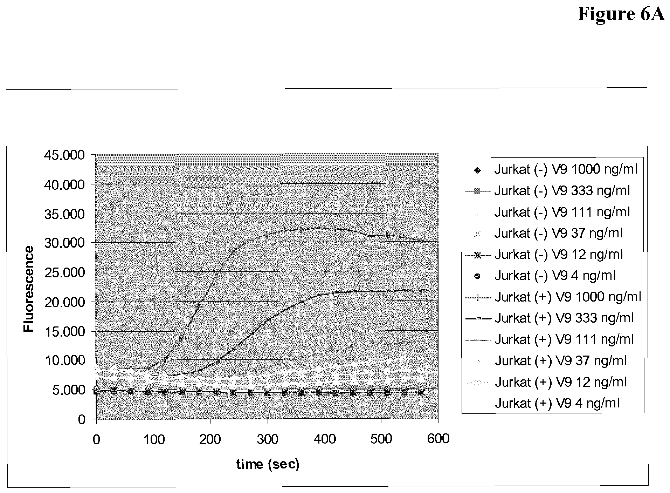

In certain embodiments, an antibody of the invention is tested for such biological activity. In order to test the functional activity of the anti-CD3 epsilon antibodies a calcium flux assay can be used using CD3-positive (Jurkat E6-1) and CD3-negative (Jurkat RT3-T3.5) human T-cell lines. Therefore, for example, CD3-positive Jurkat E6-1 cells or CD3-negative Jurkat RT3-T3.5 are plated in black-walled, clear bottom 96-well plates (BD Falcon) at 200,000 cells in 50 µl serum-free medium (RPMI 1640 / 2 mM Glutamine / 1mM Sodium pyruvate / 10 mM Hepes / 0.1mM NEAA) per well. Cells are loaded with the calcium sensitive dye (FLIPR® Calcium 5 Assay Kit, Molecular Devices). A stock solution of the dye is prepared according to the manufacturer's instructions. Directly before use Probenecid is added and 50 µl/well of the diluted dye are added to the cells (final concentration of Probenecid will be 2.5 mM/well). For efficient loading cells are incubated with the dye for 2h at room temperature in the dark. Subsequently, cells are stimulated by the addition of 20 µl rabbit anti-CD3 epsilon mAb (rabbit B-cell supernatants) or serial dilutions of chimeric V9 mAb, a chimeric anti-CD3 antibody consisting of rabbit immunoglobulin constant regions and the variable regions of the humanized anti-CD3 mAb V9. Unspecific polyclonal rabbit IgG serve as negative control. The kinetic of the anti-CD3 epsilon induced calcium flux is monitored by measuring the fluorescence (485 nm ex. / 530 nm em.) at 30 s time intervals for 7.5-10 min. The calcium flux induced by the chimeric V9 mAb is shown in Fig. 6A. The chimeric V9 mAb induces calcium mobilization only in CD3-positive Jurkat E6-1 cells and not in CD3-negative Jurkat RT3-T3.5 cells demonstrating the CD3 dependency of the effect. Likewise, there is no calcium flux observed when cells are treated with unspecific rabbit IgG.

### D. Immunoconjugates

The invention also provides immunoconjugates comprising an anti-CD3 epsilon antibody herein conjugated to one or more cytotoxic agents, such as chemotherapeutic agents or drugs, growth inhibitory agents, toxins (e.g., protein toxins, enzymatically active toxins of bacterial, fungal, plant, or animal origin, or fragments thereof), or radioactive isotopes.

In one embodiment, an immunoconjugate is an antibody-drug conjugate (ADC) in which an antibody is conjugated to one or more drugs, including but not limited to a maytansinoid (see US 5,208,020, US 5,416,064 and EP 0 425 235 B1); an auristatin such as monomethyl auristatin drug moieties DE and DF (MMAE and MMAF) (see US 5,635,483, US 5,780,588, and US 7,498,298); a dolastatin; a calicheamicin or derivative thereof (see US 5,712,374, US 5,714,586, US 5,739,116, US 5,767,285, US 5,770,701, US 5,770,710, US 5,773,001, and US 5,877,296; Hinman, L.M. et al., Cancer Res. 53 (1993) 3336-3342; and Lode, H.N. et al., Cancer Res. 58 (1998) 2925-2928); an anthracycline such as daunomycin or doxorubicin (see Kratz, F. et al., Curr. Med. Chem. 13 (2006) 477-523; Jeffrey, S.C. et al., Bioorg. Med. Chem. Lett. 16 (2006) 358-362; Torgov, M.Y. et al., Bioconjug. Chem. 16 (2005) 717-721; Nagy, A. et al., Proc. Natl. Acad. Sci. USA 97 (2000) 829-834; Dubowchik, G.M. et al., Bioorg. & Med. Chem. Letters 12 (2002) 1529-1532; King, H.D. et al., J. Med. Chem. 45 (20029 4336-4343; and U.S. Patent No. 6,630,579); methotrexate; vindesine; a taxane such as docetaxel, paclitaxel, larotaxel, tesetaxel, and ortataxel; a trichothecene; and CC1065.

In another embodiment, an immunoconjugate comprises an antibody as described herein conjugated to an enzymatically active toxin or fragment thereof, including but not limited to diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes.

In another embodiment, an immunoconjugate comprises an antibody as described herein conjugated to a radioactive atom to form a radioconjugate. A variety of radioactive isotopes are available for the production of radioconjugates. Examples include At²¹¹ , I¹³¹, I¹²⁵, Y⁹⁰, Re¹⁸⁶, Re¹⁸⁸, Sm¹⁵³, Bi²¹², P³², Pb²¹² and radioactive isotopes of Lu. When the radioconjugate is used for detection, it may comprise a radioactive atom for scintigraphic studies, for example TC^{99m} or I¹²³, or a spin label for nuclear magnetic resonance (NMR) imaging (also known as magnetic resonance imaging, MRI), such as iodine-123 again, iodine-131, indium-111, fluorine-19, carbon-13, nitrogen-15, oxygen-17, gadolinium, manganese or iron.

Conjugates of an antibody and cytotoxic agent may be made using a variety of bifunctional protein coupling agents such as N-succinimidyl-3-(2-pyridyldithio) propionate (SPDP), succinimidyl-4-(N-maleimidomethyl) cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta, E.S. et al., Science 238 (1987) 1098-1104. Carbon-14-labeled 1-isothiocyanatobenzyl-3-methyldiethylene triamine pentaacetic acid (MX-DTPA) is an exemplary chelating agent for conjugation of radionucleotide to the antibody. See WO 94/11026. The linker may be a "cleavable linker" facilitating release of a cytotoxic drug in the cell. For example, an acid-labile linker, peptidase-sensitive linker, photolabile linker, dimethyl linker or disulfide-containing linker (Chari, R.V. et al., Cancer Res. 52 (1992) 127-131; U.S. Patent No. 5,208,020) may be used.

The immunuoconjugates or ADCs herein expressly contemplate, but are not limited to such conjugates prepared with cross-linker reagents including, but not limited to, BMPS, EMCS, GMBS, HBVS, LC-SMCC, MBS, MPBH, SBAP, SIA, SIAB, SMCC, SMPB, SMPH, sulfo-EMCS, sulfo-GMBS, sulfo-KMUS, sulfo-MBS, sulfo-SIAB, sulfo-SMCC, and sulfo-SMPB, and SVSB (succinimidyl-(4-vinylsulfone)benzoate) which are commercially available (e.g., from Pierce Biotechnology, Inc., Rockford, IL., U.S.A).

### E. Methods and Compositions for Diagnostics and Detection

In certain embodiments, any of the anti-CD3 epsilon antibodies provided herein is useful for detecting the presence of CD3 epsilon in a biological sample. The term "detecting" as used herein encompasses quantitative or qualitative detection. In certain embodiments, a biological sample comprises a cell or tissue, such as blood.

In one embodiment, an anti-CD3 epsilon antibody for use in a method of diagnosis or detection is provided. In a further aspect, a method of detecting the presence of CD3 epsilon in a biological sample is provided. In certain embodiments, the method comprises contacting the biological sample with an anti-CD3 epsilon antibody as described herein under conditions permissive for binding of the anti-CD3 epsilon antibody to CD3 epsilon, and detecting whether a complex is formed between the anti-CD3 epsilon antibody and CD3 epsilon. Such method may be an *in vitro* or *in vivo* method. In one embodiment, an anti-CD3 epsilon antibody is used to select subjects eligible for therapy with an anti-CD3 epsilon antibody, e.g. where CD3 epsilon is a biomarker for selection of patients.

Exemplary disorders that may be diagnosed using an antibody of the invention include cancer.

In certain embodiments, labeled anti-CD3 epsilon antibodies are provided. Labels include, but are not limited to, labels or moieties that are detected directly (such as fluorescent, chromophoric, electron-dense, chemiluminescent, and radioactive labels), as well as moieties, such as enzymes or ligands, that are detected indirectly, e.g., through an enzymatic reaction or molecular interaction. Exemplary labels include, but are not limited to, the radioisotopes ³²P, ¹⁴C, ¹²⁵I, ³H, and ¹³¹I, fluorophores such as rare earth chelates or fluorescein and its derivatives, rhodamine and its derivatives, dansyl, umbelliferone, luceriferases, e.g., firefly luciferase and bacterial luciferase (U.S. Patent No. 4,737,456), luciferin, 2,3-dihydrophthalazinediones, horseradish peroxidase (HRP), alkaline phosphatase, β-galactosidase, glucoamylase, lysozyme, saccharide oxidases, e.g., glucose oxidase, galactose oxidase, and glucose-6-phosphate dehydrogenase, heterocyclic oxidases such as uricase and xanthine oxidase, coupled with an enzyme that employs hydrogen peroxide to oxidize a dye precursor such as HRP, lactoperoxidase, or microperoxidase, biotin/avidin, spin labels, bacteriophage labels, stable free radicals, and the like.

### F. Pharmaceutical Formulations

Pharmaceutical formulations of an anti-CD3 epsilon antibody as described herein are prepared by mixing such antibody having the desired degree of purity with one or more optional pharmaceutically acceptable carriers (Remington's Pharmaceutical Sciences, 16th edition, Osol, A. (ed.) (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyl dimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as poly(vinylpyrrolidone); amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g*. Zn-protein complexes); and/or non-ionic surfactants such as polyethylene glycol (PEG). Exemplary pharmaceutically acceptable carriers herein further include interstitial drug dispersion agents such as soluble neutral-active hyaluronidase glycoproteins (sHASEGP), for example, human soluble PH-20 hyaluronidase glycoproteins, such as rhuPH20 (HYLENEX®, Baxter International, Inc.). Certain exemplary sHASEGPs and methods of use, including rhuPH20, are described in US Patent Publication Nos. 2005/0260186 and 2006/0104968. In one aspect, a sHASEGP is combined with one or more additional glycosaminoglycanases such as chondroitinases.

Exemplary lyophilized antibody formulations are described in US Patent No. 6,267,958. Aqueous antibody formulations include those described in US Patent No. 6,171,586 and WO 2006/044908, the latter formulations including a histidine-acetate buffer.

The formulation herein may also contain more than one active ingredients as necessary for the particular indication being treated, preferably those with complementary activities that do not adversely affect each other. Such active ingredients are suitably present in combination in amounts that are effective for the purpose intended.

Active ingredients may be entrapped in microcapsules prepared, for example, by coacervation techniques or by interfacial polymerization, for example, hydroxymethylcellulose or gelatin-microcapsules and poly-(methyl methacrylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences, 16th edition, Osol, A. (ed.) (1980).

Sustained-release preparations may be prepared. Suitable examples of sustained-release preparations include semi-permeable matrices of solid hydrophobic polymers containing the antibody, which matrices are in the form of shaped articles, *e.g.* films, or microcapsules.

The formulations to be used for *in vivo* administration are generally sterile. Sterility may be readily accomplished, e.g., by filtration through sterile filtration membranes.

### G. Therapeutic Methods and Compositions

Any of the anti-CD3 epsilon antibodies provided herein may be used in therapeutic methods.

In one aspect, an anti-CD3 epsilon antibody for use as a medicament is provided. In further aspects, an anti-CD3 epsilon antibody for use in treating cancer is provided. In certain embodiments, an anti-CD3 epsilon antibody for use in a method of treatment is provided. In certain embodiments, the invention provides an anti-CD3 epsilon antibody for use in a method of treating an individual having cancer comprising administering to the individual an effective amount of the anti-CD3 epsilon antibody. In one such embodiment, the method further comprises administering to the individual an effective amount of at least one additional therapeutic agent, e.g., as described below. In further embodiments, the invention provides an anti-CD3 epsilon antibody for use in activating T cells. In certain embodiments, the invention provides an anti-CD3 epsilon antibody for use in a method of activating T cells in an individual comprising administering to the individual an effective of the anti-CD3 epsilon antibody to activate T cells. An "individual" according to any of the above embodiments is preferably a human

In a further aspect, the invention provides for the use of an anti-CD3 epsilon antibody in the manufacture or preparation of a medicament. In one embodiment, the medicament is for treatment of cancer. In a further embodiment, the medicament is for use in a method of treating cancer comprising administering to an individual having cancer an effective amount of the medicament. In one such embodiment, the method further comprises administering to the individual an effective amount of at least one additional therapeutic agent. In a further embodiment, the medicament is for activation of T cells. In a further embodiment, the medicament is for use in a method of activating T cells in an individual comprising administering to the individual an amount effective of the medicament to activate T cells. An "individual" according to any of the above embodiments may be a human.

In a further aspect, the invention provides pharmaceutical formulations comprising any of the anti-CD3 epsilon antibodies provided herein, e.g., for use in any of the above therapeutic methods. In one embodiment, a pharmaceutical formulation comprises any of the anti-CD3 epsilon antibodies provided herein and a pharmaceutically acceptable carrier. In another embodiment, a pharmaceutical formulation comprises any of the anti-CD3 epsilon antibodies provided herein and at least one additional therapeutic agent.

Antibodies of the invention can be used either alone or in combination with other agents in a therapy. For instance, an antibody of the invention may be co-administered with at least one additional therapeutic agent.

Such combination therapies noted above encompass combined administration (where two or more therapeutic agents are included in the same or separate formulations), and separate administration, in which case, administration of the antibody of the invention can occur prior to, simultaneously, and/or following, administration of the additional therapeutic agent and/or adjuvant. Antibodies of the invention can also be used in combination with radiation therapy.

An antibody of the invention (and any additional therapeutic agent) can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein.

Antibodies of the invention would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The antibody need not be, but is optionally formulated with one or more agents currently used to prevent or treat the disorder in question. The effective amount of such other agents depends on the amount of antibody present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For the prevention or treatment of a disease, the appropriate dosage of an antibody of the invention (when used alone or in combination with one or more other additional therapeutic agents) will depend on the type of disease to be treated, the type of antibody, the severity and course of the disease, whether the antibody is administered for preventive or therapeutic purposes, previous therapy, the patient's clinical history and response to the antibody, and the discretion of the attending physician. The antibody is suitably administered to the patient at one time or over a series of treatments. Depending on the type and severity of the disease, about 1 µg/kg to 15 mg/kg (e.g. 0.5mg/kg - 10 mg/kg) of antibody can be an initial candidate dosage for administration to the patient, whether, for example, by one or more separate administrations, or by continuous infusion. One typical daily dosage might range from about 1 µg/kg to 100 mg/kg or more, depending on the factors mentioned above. For repeated administrations over several days or longer, depending on the condition, the treatment would generally be sustained until a desired suppression of disease symptoms occurs. One exemplary dosage of the antibody would be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the patient. Such doses may be administered intermittently, e.g. every week or every three weeks (*e.g*. such that the patient receives from about two to about twenty, or *e.g.* about six doses of the antibody). An initial higher loading dose, followed by one or more lower doses may be administered. The progress of this therapy is easily monitored by conventional techniques and assays.

It is understood that any of the above formulations or therapeutic methods may be carried out using an immunoconjugate of the invention in place of or in addition to an anti-CD3 epsilon antibody.

### III. Articles of Manufacture

In another aspect of the invention, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). At least one active agent in the composition is an antibody of the invention. The label or package insert indicates that the composition is used for treating the condition of choice. Moreover, the article of manufacture may comprise (a) a first container with a composition contained therein, wherein the composition comprises an antibody of the invention; and (b) a second container with a composition contained therein, wherein the composition comprises a further cytotoxic or otherwise therapeutic agent. The article of manufacture in this embodiment of the invention may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

It is understood that any of the above articles of manufacture may include an immunoconjugate of the invention in place of or in addition to an anti-CD3 epsilon antibody.

### Specific embodiments of the invention

1. Use of a method comprising the step of immunizing an experimental animal with a native cynomolgus antigen as sole antigen for producing a human cynomolgus cross-reactive antibody.
2. Use according to embodiment 1 wherein the native cynomolgus antigen has less than 80% sequence identity to the corresponding human antigen.
3. Use according to any one of embodiments 1 to 2 wherein the native cynomolgus antigen has 80% to 50% sequence identity to the corresponding human antigen.
4. Use according to any one of embodiments 1 to 3 wherein the native cynomolgus antigen has 80% to 60% sequence identity to the corresponding human antigen.
5. Use according to any one of embodiments 1 to 4 wherein the native cynomolgus antigen has 80% to 70% sequence identity to the corresponding human antigen.
6. Use according to any one of embodiments 1 to 5 wherein the native cynomolgus antigen lacks one or more (contiguous) amino acid stretches that are present in the corresponding human antigen, whereby one of the lacking (contiguous) amino acid stretches in the corresponding human antigen is the main immunogenic epitope of the human antigen.
7. Use according to any one of embodiments 1 to 6 wherein the native cynomolgus antigen is a T cell antigen.
8. Use according to any one of embodiments 1 to 7 wherein the native cynomolgus antigen is CD3 epsilon.
9. Use according to any one of embodiments 1 to 8 wherein the native cynomolgus antigen is CD3 epsilon and the human cynomolgus cross-reactive antibody specifically binds to (native) human CD3 epsilon of SEQ ID NO: 02 and specifically binds to a polypeptide of SEQ ID NO: 01.
10. Use according to any one of embodiments 1 to 9 wherein the experimental animal is immunized one or more times with primary cynomolgus PBLs, whereby the PBLs are optionally enriched for T cells.
11. Use according to any one of embodiments 1 to 10 wherein the experimental animal is immunized two times with primary cynomolgus PBLs, whereby the PBLs are optionally enriched for T cells.
12. Use according to any one of embodiments 1 to 11 wherein the experimental animal is immunized three times with primary cynomolgus PBLs, whereby the PBLs are optionally enriched for T cells.
13. Use according to any one of embodiments 1 to 12 wherein the immunizing comprises an intradermal application, an intramuscular application and a subcutaneous application.
14. Use according to any one of embodiments 1 to 13 wherein the immunizing comprises as first step an intradermal application, as second step an intramuscular application and as third step a subcutaneous application.
15. Use according to any one of embodiments 1 to 14 wherein the experimental animal is immunized one or more times once weekly with primary cynomolgus PBLs, whereby the PBLs are optionally enriched for T cells.
16. Use according to any one of embodiments 10 to 15 wherein the experimental animal is immunized three times once weekly with primary cynomolgus PBLs, whereby the PBLs are optionally enriched for T cells.
17. Use according to any one of embodiments 1 to 16 wherein the experimental animal is a (human) transgenic experimental animal.
18. Use according to any one of embodiments 1 to 17 wherein the experimental animal is a mouse or a rat or a guinea pig or a rabbit.
19. Use according to any one of embodiments 1 to 18 wherein the experimental animal is a rabbit.
20. Use according to any one of embodiments 1 to 19 wherein the experimental animal is a rat.
21. Use according to any one of embodiments 1 to 20 wherein the method is without using a denaturing agent.
22. Use according to any one of embodiments 1 to 21 wherein the method is without using complete Freud's adjuvant.
23. Use according to any one of embodiments 1 to 22 wherein the human cynomolgus cross-reactive antibody specifically binds to human and cynomolgus T cells, to the polypeptide of SEQ ID NO: 01 and activates human T cells.
24. Use according to any one of embodiments 1 to 23 wherein the human cynomolgus cross-reactive antibody specifically binds to human and cynomolgus CD3 epsilon, to the polypeptide of SEQ ID NO: 01 and activates human T cells.
25. Use according to any one of embodiments 1 to 24 wherein the human cynomolgus cross-reactive antibody does not specifically bind to a polypeptide consisting of residues 30 to 60 of human CD3 epsilon of SEQ ID NO: 02.
26. Use according to any one of embodiments 1 to 25 wherein the human cynomolgus cross-reactive antibody does not specifically bind to a polypeptide consisting of residues 1 to 70 of human CD3 epsilon of SEQ ID NO: 02.
27. Use according to any one of embodiments 1 to 27 wherein the human cynomolgus cross-reactive antibody does not bind to the same epitope as the antibody OKT3, the antibody UCHT1 and/or the antibody SP34.
28. Use of a method comprising the step of immunizing an experimental animal, three times with primary cynomolgus PBLs, whereby the PBLs are optionally enriched for T cells, without using primary human PBLs as immunogen and without using a denaturing agent for producing a human cynomolgus cross-reactive antibody, wherein the antibody specifically binds to human and cynomolgus T cells, to the polypeptide of SEQ ID NO: 01 and activates human T cells.
29. Use of a method comprising the step of immunizing an experimental animal, three times with primary cynomolgus PBLs, whereby the PBLs are optionally enriched for T cells without using primary human PBLs as immunogen and without using a denaturing agent for producing a human cynomolgus cross-reactive antibody that specifically binds to human CD3 epsilon of SEQ ID NO: 02 and that specifically binds to a polypeptide of SEQ ID NO: 01, wherein the antibody specifically binds to human and cynomolgus T cells, activates human T cells and does not bind to the same epitope as the antibody OKT3, the antibody UCHT1 and/or the antibody SP34.
30. Method for producing a human cynomolgus cross-reactive antibody comprising the step of immunizing an experimental animal with a native cynomolgus antigen as sole antigen.
31. Method according to any one of embodiment 30 wherein the native cynomolgus antigen has less than 80% sequence identity to the corresponding human antigen.
32. Method according to any one of embodiments 30 to 31 wherein the native cynomolgus antigen has 80% to 50% sequence identity to the corresponding human antigen.
33. Method according to any one of embodiments 30 to 32 wherein the native cynomolgus antigen has 80% to 60% sequence identity to the corresponding human antigen.
34. Method according to any one of embodiments 30 to 33 wherein the native cynomolgus antigen has 80% to 70% sequence identity to the corresponding human antigen.
35. Method according to any one of embodiments 30 to 34 wherein the native cynomolgus antigen lacks one or more (contiguous) amino acid stretches that are present in the corresponding human antigen, whereby one of the lacking (contiguous) amino acid stretches in the corresponding human antigen is the main immunogenic epitope of the human antigen.
36. Method according to any one of embodiments 30 to 35 wherein the native cynomolgus antigen is a T cell antigen.
37. Method according to any one of embodiments 30 to 36 wherein the native cynomolgus antigen is CD3 epsilon.
38. Method according to any one of embodiments 30 to 37 wherein the native cynomolgus antigen is CD3 epsilon and the human cynomolgus cross-reactive antibody specifically binds to (native) human CD3 epsilon of SEQ ID NO: 02 and specifically binds to a polypeptide of SEQ ID NO: 01.
39. Method according to any one of embodiments 30 to 38 wherein the experimental animal is immunized one or more times with primary cynomolgus PBLs, whereby the PBLs are optionally enriched for T cells.
40. Method according to any one of embodiments 30 to 39 wherein the experimental animal is immunized two times with primary cynomolgus PBLs, whereby the PBLs are optionally enriched for T cells.
41. Method according to any one of embodiments 30 to 40 wherein the experimental animal is immunized three times with primary cynomolgus PBLs, whereby the PBLs are optionally enriched for T cells.
42. Method according to any one of embodiments 30 to 41 wherein the immunizing comprises an intradermal application, an intramuscular application and a subcutaneous application.
43. Method according to any one of embodiments 30 to 42 wherein the immunizing comprises as first step an intradermal application, as second step an intramuscular application and as third step a subcutaneous application.
44. Method according to any one of embodiments 30 to 43 wherein the experimental animal is immunized one or more times once weekly with primary cynomolgus PBLs, whereby the PBLs are optionally enriched for T cells.
45. Method according to any one of embodiments 39 to 44 wherein the experimental animal is immunized three times once weekly with primary cynomolgus PBLs, whereby the PBLs are optionally enriched for T cells.
46. Method according to any one of embodiments 30 to 45 wherein the experimental animal is a transgenic experimental animal.
47. Method according to any one of embodiments 30 to 46 wherein the experimental animal is a mouse or a rat or a guinea pig or a rabbit.
48. Method according to any one of embodiments 30 to 47 wherein the experimental animal is a rabbit.
49. Method according to any one of embodiments 30 to 48 wherein the experimental animal is a rat.
50. Method according to any one of embodiments 30 to 49 wherein the method is without using a denaturing agent.
51. Method according to any one of embodiments 30 to 50 wherein the method is without using complete Freud's adjuvant.
52. Method according to any one of embodiments 30 to 51 wherein the human cynomolgus cross-reactive antibody specifically binds to human and cynomolgus T cells, to the polypeptide of SEQ ID NO: 01 and activates human T cells.
53. Method according to any one of embodiments 30 to 52 wherein the human cynomolgus cross-reactive antibody specifically binds to human and cynomolgus CD3 epsilon, to the polypeptide of SEQ ID NO: 01 and activates human T cells.
54. Method according to embodiments 30 to 53 wherein the human cynomolgus cross-reactive antibody does not specifically bind to a polypeptide consisting of residues 30 to 60 of human CD3 epsilon of SEQ ID NO: 02.
55. Method according to any one of embodiments 30 to 54 wherein the human cynomolgus cross-reactive antibody does not specifically bind to a polypeptide consisting of residues 1 to 70 of human CD3 epsilon of SEQ ID NO: 02.
56. Method according to any one of embodiments 30 to 55 wherein the human cynomolgus cross-reactive antibody does not bind to the same epitope as the antibody OKT3, the antibody UCHT1 and/or the antibody SP34.
57. Method for producing a human cynomolgus cross-reactive antibody comprising the step of immunizing an experimental animal, three times with primary cynomolgus PBLs, whereby the PBLs are optionally enriched for T cells without using primary human PBLs, whereby the PBLs are optionally enriched for T cells as immunogen and without using a denaturing agent, wherein the human cynomolgus cross-reactive antibody specifically binds to human and cynomolgus T cells, to the polypeptide of SEQ ID NO: 01 and activates human T cells.
58. Method for producing a human cynomolgus cross-reactive antibody binding to human CD3 epsilon of SEQ ID NO: 02 and specifically binding to a polypeptide of SEQ ID NO: 01 comprising the step of immunizing an experimental animal, three times with primary cynomolgus PBLs, whereby the PBLs are optionally enriched for T cells without using primary human PBLs, whereby the PBLs are optionally enriched for T cells as immunogen and without using a denaturing agent, wherein the human cynomolgus cross-reactive antibody specifically binds to human and cynomolgus T cells, activates human T cells and does not bind to the same epitope as the antibody OKT3, the antibody UCHT1 and/or the antibody SP34.
59. Method for recombinantly producing a human cynomolgus cross-reactive antibody comprising the following steps:
   a) producing a human cynomolgus cross-reactive antibody with a method according to any one of embodiments 30 to 58,
   b) providing a cell comprising the nucleic acid encoding the antibody produced in step a)
   c) cultivating the cell of step b)
   d) recovering the antibody from the cell or the cultivation supernatant
   and thereby recombinantly producing the human cynomolgus cross-reactive antibody.
60. Method for recombinantly producing a human cynomolgus cross-reactive antibody comprising the following steps:
   a) producing an antibody with a method according to any one of embodiments 30 to 58,
   b) isolating the nucleic acid encoding the antibody produced in step a)
   c) optionally humanizing the antibody
   d) cloning the nucleic acid encoding the antibody isolated in step b) or obtained in step c) in an expression vector
   e) transfecting a cell with the expression vector obtained in step d)
   f) cultivating the cell of step e)
   g) recovering the antibody from the cell or the cultivation supernatant
   and thereby recombinantly producing the human cynomolgus cross-reactive antibody.
61. Human cynomolgus cross-reactive antibody specifically binding to human CD3 epsilon of SEQ ID NO: 02 and specifically binding to a polypeptide of SEQ ID NO: 01, wherein the human cynomolgus cross-reactive antibody specifically binds to human and cynomolgus T cells, to the polypeptide of SEQ ID NO: 01 and activates human T cells.
62. Human cynomolgus cross-reactive antibody specifically binding to human CD3 epsilon of SEQ ID NO: 02 and specifically binding to a polypeptide of SEQ ID NO: 01, wherein the human cynomolgus cross-reactive antibody specifically binds to human and cynomolgus T cells, activates human T cells and does not bind to the same epitope as the antibody OKT3, the antibody UCHT1 and/or the antibody SP34.
63. The human cynomolgus cross-reactive antibody of any one of embodiments 61 to 62, wherein the antibody comprises (a) HVR-H3 comprising the amino acid sequence of SEQ ID NO: 09, (b) HVR-L3 comprising the amino acid sequence of SEQ ID NO: 13, and (c) HVR-H2 comprising one amino acid sequence selected from the group consisting of SEQ ID NO: 06 to SEQ ID NO: 08.
64. The human cynomolgus cross-reactive antibody of any one of embodiments 61 to 63, wherein the antibody comprises (a) HVR-H1 comprising one amino acid sequence selected from the group consisting of SEQ ID NO: 04 to SEQ ID NO: 05, (b) HVR-H2 comprising one amino acid sequence selected from the group consisting of SEQ ID NO: 06 to SEQ ID NO: 08, and (c) HVR-H3 comprising one amino acid sequence of SEQ ID NO: 09.
65. The human cynomolgus cross-reactive antibody of any one of embodiments 61 to 64, comprising (a) HVR-L1 comprising one amino acid sequence selected from the group consisting of SEQ ID NO: 10 to SEQ ID NO: 11; (b) HVR-L2 comprising the amino acid sequence of SEQ ID NO: 12; and (c) HVR-L3 comprising the amino acid sequence of SEQ ID NO: 13.
66. The human cynomolgus cross-reactive antibody of any one of embodiments 61 to 65, wherein the antibody comprises (a) HVR-H1 comprising the amino acid sequence of SEQ ID NO: 05, (b) HVR-H2 comprising the amino acid sequence of SEQ ID NO: 08; (c) HVR-H3 comprising the amino acid sequence of SEQ ID NO: 09; (d) HVR-L1 comprising the amino acid sequence of SEQ ID NO: 11; (e) HVR-L2 comprising the amino acid sequence of SEQ ID NO: 12; and (f) HVR-L3 comprising the amino acid sequence of SEQ ID NO: 13.
67. The human cynomolgus cross-reactive antibody of any one of embodiments 61 to 66, comprising (a) a VH sequence having at least 95% sequence identity to the amino acid sequence of SEQ ID NO: 14; (b) a VL sequence having at least 95% sequence identity to the amino acid sequence of SEQ ID NO: 15; or (c) a VH sequence as in (a) and a VL sequence as in (b).
68. The human cynomolgus cross-reactive antibody of any one of embodiments 61 to 67, comprising a VH sequence of SEQ ID NO: 14.
69. The human cynomolgus cross-reactive antibody of any one of embodiments 61-68, comprising a VL sequence of SEQ ID NO: 15.
70. A human cynomolgus cross-reactive antibody comprising a VH sequence of SEQ ID NO: 14 and a VL sequence of SEQ ID NO: 15.
71. An immunoconjugate comprising the human cynomolgus cross-reactive antibody of any one of embodiments 61 to 70 and a cytotoxic agent.
72. A pharmaceutical formulation comprising the human cynomolgus cross-reactive antibody of any one of embodiments 61 to 71 and a pharmaceutically acceptable carrier.
73. The human cynomolgus cross-reactive antibody of any one of embodiments 61 to 72 for use as a medicament
74. The human cynomolgus cross-reactive antibody of any one of embodiments 61 to 73, wherein the antibody comprises a humanized variant of the rabbit VH sequence of SEQ ID NO: 14 and a humanized variant of the rabbit VL sequence of SEQ ID NO: 15.
75. The human cynomolgus cross-reactive antibody of any one of embodiments 61 to 74, wherein the antibody is specifically binding to human CD3 epsilon of SEQ ID NO: 02 and specifically binding to a polypeptide of SEQ ID NO: 01 and wherein the antibody specifically binds to human and cynomolgus T cells and activates human T cells.
76. The human cynomolgus cross-reactive antibody of any one of embodiments 61 to 75, wherein the antibody is specifically binding to human CD3 epsilon of SEQ ID NO: 02 and specifically binding to a polypeptide of SEQ ID NO: 01 and wherein the antibody specifically binds to human and cynomolgus T cells, activates human T cells and does not bind to the same epitope as the antibody OKT3, the antibody UCHT1 and/or the antibody SP34.
77. Human cynomolgus cross-reactive antibody obtainable by a method comprising the step of immunizing an experimental animal with a native cynomolgus antigen as sole antigen.
78. Human cynomolgus cross-reactive antibody obtainable by a method according to embodiment 77 wherein the native cynomolgus antigen has less than 80% sequence identity to the corresponding human antigen.
79. Human cynomolgus cross-reactive antibody obtainable by a method according to any one of embodiments 77 to 78 wherein the native cynomolgus antigen has 80% to 50% sequence identity to the corresponding human antigen.
80. Human cynomolgus cross-reactive antibody obtainable by a method according to any one of embodiments 77 to 79 wherein the native cynomolgus antigen has 80% to 60% sequence identity to the corresponding human antigen.
81. Human cynomolgus cross-reactive antibody obtainable by a method according to any one of embodiments 77 to 80 wherein the native cynomolgus antigen has 80% to 70% sequence identity to the corresponding human antigen.
82. Human cynomolgus cross-reactive antibody obtainable by a method according to any one of embodiments 77 to 81 wherein the native cynomolgus antigen lacks one or more (contiguous) amino acid stretches that are present in the corresponding human antigen, whereby one of the lacking (contiguous) amino acid stretches in the corresponding human antigen is the main immunogenic epitope of the human antigen.
83. Human cynomolgus cross-reactive antibody obtainable by a method according to any one of embodiments 77 to 82 wherein the native cynomolgus antigen is a T cell antigen.
84. Human cynomolgus cross-reactive antibody obtainable by a method according to any one of embodiments 77 to 83 wherein the native cynomolgus antigen is CD3 epsilon.
85. Human cynomolgus cross-reactive antibody obtainable by a method according to any one of embodiments 77 to 84 wherein the native cynomolgus antigen is CD3 epsilon and the antibody binds to (native) human CD3 epsilon of SEQ ID NO: 02 and specifically binds to a polypeptide of SEQ ID NO: 01.
86. Human cynomolgus cross-reactive antibody obtainable by a method according to any one of embodiments 77 to 85 wherein the experimental animal is immunized one or more times with primary cynomolgus PBLs, whereby the PBLs are optionally enriched for T cells.
87. Human cynomolgus cross-reactive antibody obtainable by a method according to any one of embodiments 77 to 86 wherein the experimental animal is immunized two times with primary cynomolgus PBLs, whereby the PBLs are optionally enriched for T cells.
88. Human cynomolgus cross-reactive antibody obtainable by a method according to any one of embodiments 77 to 87 wherein the experimental animal is immunized three times with primary cynomolgus PBLs, whereby the PBLs are optionally enriched for T cells.
89. Human cynomolgus cross-reactive antibody obtainable by a method according to any one of embodiments 77 to 88 wherein the immunizing comprises an intradermal application, an intramuscular application and a subcutaneous application.
90. Human cynomolgus cross-reactive antibody obtainable by a method according to any one of embodiments 77 to 89 wherein the immunizing comprises as first injection an intradermal application, as second injection an intramuscular application and as third injection a subcutaneous application.
91. Human cynomolgus cross-reactive antibody obtainable by a method according to any one of embodiments 77 to 90 wherein the experimental animal is immunized one or more times once weekly with primary cynomolgus PBLs, whereby the PBLs are optionally enriched for T cells.
92. Human cynomolgus cross-reactive antibody obtainable by a method according to any one of embodiments 77 to 91 wherein the experimental animal is immunized three times once weekly with primary cynomolgus PBLs, whereby the PBLs are optionally enriched for T cells.
93. Human cynomolgus cross-reactive antibody obtainable by a method according to any one of embodiments 77 to 92 wherein the experimental animal is a transgenic experimental animal.
94. Human cynomolgus cross-reactive antibody obtainable by a method according to any one of embodiments 77 to 93 wherein the experimental animal is a mouse or a rat or a guinea pig or a rabbit.
95. Human cynomolgus cross-reactive antibody obtainable by a method according to any one of embodiments 77 to 94 wherein the experimental animal is a rabbit.
96. Human cynomolgus cross-reactive antibody obtainable by a method according to any one of embodiments 77 to 95 wherein the experimental animal is a rat.
97. Human cynomolgus cross-reactive antibody obtainable by a method according to any one of embodiments 77 to 96 wherein the method is without using a denaturing agent.
98. Human cynomolgus cross-reactive antibody obtainable by a method according to any one of embodiments 77 to 97 wherein the method is without using complete Freud's adjuvant.
99. Human cynomolgus cross-reactive antibody obtainable by a method according to any one of embodiments 77 to 98 wherein the antibody specifically binds to human and cynomolgus T cells, to the polypeptide of SEQ ID NO: 01 and activates human T cells.
100. Human cynomolgus cross-reactive antibody obtainable by a method according to any one of embodiments 77 to 99 wherein the antibody specifically binds to human and cynomolgus CD3 epsilon, to the polypeptide of SEQ ID NO: 01 and activates human T cells.
101. Human cynomolgus cross-reactive antibody obtainable by a method according to any one of embodiments 77 to 100 wherein the antibody does not specifically bind to a polypeptide consisting of residues 30 to 60 of human CD3 epsilon of SEQ ID NO: 02.
102. Human cynomolgus cross-reactive antibody obtainable by a method according to any one of embodiments 77 to 101 wherein the antibody does not specifically bind to a polypeptide consisting of residues 1 to 70 of human CD3 epsilon of SEQ ID NO: 02.
103. Human cynomolgus cross-reactive antibody obtainable by a method according to any one of embodiments 77 to 102 wherein the antibody does not bind to the same epitope as the antibody OKT3, the antibody UCHT1 and/or the antibody SP34.
104. Human cynomolgus cross-reactive antibody obtainable by immunizing an experimental animal, three times with primary cynomolgus PBLs, whereby the PBLs are optionally enriched for T cells without using primary human PBLs as immunogen and without using a denaturing agent, wherein the human cynomolgus cross-reactive antibody specifically binds to human and cynomolgus T cells, to the polypeptide of SEQ ID NO: 01 and activates human T cells.
105. Human cynomolgus cross-reactive antibody specifically binding to human CD3 epsilon of SEQ ID NO: 02 and specifically binding to a polypeptide of SEQ ID NO: 01 obtainable by immunizing an experimental animal, three times with primary cynomolgus PBLs, whereby the PBLs are optionally enriched for T cells without using primary human PBLs as immunogen and without using a denaturing agent, wherein the human cynomolgus cross-reactive antibody specifically binds to human and cynomolgus T cells, ,activates human T cells and does not bind to the same epitope as the antibody OKT3, the antibody UCHT1 and/or the antibody SP34.

The following examples, figures and sequence are provided to aid the understanding of the present invention, the true scope of which is set forth in the appended claims. It is understood that modifications can be made in the procedures set forth without departing from the spirit of the invention.

### Description of the Sequences

| | |
|---|---|
| **SEQ ID NO: 01** | Amino acid sequence of residues 77-96 of human CD3 epsilon extracellular domain. |
| **SEQ ID NO: 02** | Amino acid sequence of extracellular domain of human CD3 epsilon. |
| **SEQ ID NO: 03** | Amino acid sequence of full length human CD3 epsilon. |
| **SEQ ID NO: 04** | Amino acid sequence of HVR-H1 variant 1 of antibody clone 645. |
| **SEQ ID NO: 05** | Amino acid sequence of HVR-H1 variant 2 of antibody clone 645. |
| **SEQ ID NO: 06** | Amino acid sequence of HVR-H2 variant 1 of antibody clone 645. |
| **SEQ ID NO: 07** | Amino acid sequence of HVR-H2 variant 2 of antibody clone 645. |
| **SEQ ID NO: 08** | Amino acid sequence of HVR-H2 variant 3 of antibody clone 645. |
| **SEQ ID NO: 09** | Amino acid sequence of HVR-H3 of antibody clone 645. |
| **SEQ ID NO: 10** | Amino acid sequence of HVR-L1 variant 1 of antibody clone 645. |
| **SEQ ID NO: 11** | Amino acid sequence of HVR-L1 variant 2 of antibody clone 645. |
| **SEQ ID NO: 12** | Amino acid sequence of HVR-L2 of antibody clone 645. |
| **SEQ ID NO: 13** | Amino acid sequence of HVR-L3 of antibody clone 645. |
| **SEQ ID NO: 14** | Amino acid sequence of heavy chain variable region (VH) of antibody clone 645. |
| **SEQ ID NO: 15** | Amino acid sequence of light chain variable region (VL) of antibody clone 645. |
| **SEQ ID NO: 16** | Amino acid sequence of human CD3g(G4S)5CD3e-AcTev-Fc(knob)-Avi fusion polypeptide. |
| **SEQ ID NO: 17** | Amino acid sequence of Fc(hole). |
| **SEQ ID NO: 18** | Amino acid sequence of cynomolgus CD3g(G4S)5CD3e-AcTev-Fc(knob)-Avi fusion polypeptide. |
| **SEQ ID NO: 19** | Amino acid sequence of human CD3e-stalk-Fc(knob)-Avi fusion polypeptide. |
| **SEQ ID NO: 20** | Amino acid sequence of human CD3d-stalk-Fc(hole)-Avi fusion polypeptide. |
| **SEQ ID NO: 21** | Amino acid sequence of human CD3gC85-stalk-Fc(hole)-Avi fusion polypeptide. |
| **SEQ ID NO: 22** | Amino acid sequence of cynomolgus CD3e stalk-Fc(knob)-Avi fusion polypeptide. |
| **SEQ ID NO: 23** | Amino acid sequence of cynomolgus CD3d stalk-Fc(hole)-Avi fusion polypeptide. |
| **SEQ ID NO: 24** | Amino acid sequence of human CD3e -1-26 - Fc(knob)Avi fusion polypeptide. |
| **SEQ ID NO: 25** | Amino acid sequence of cynomolgus CD3e 5-26 - Fc(knob)Avi fusion polypeptide. |
| **SEQ ID NO: 26** | Amino acid sequence of human CD3e 5-26 - Fc(knob)Avi fusion polypeptide. |
| **SEQ ID NO: 27** | Amino acid sequence of full length cynomolgus CD3 epsilon. |
| **SEQ ID NO: 28** | Amino acid sequence of cynomolgus CD3 epsilon extracellular domain. |
| **SEQ ID NO: 29** | Amino acid sequence of residues 69-88 of cynomolgus CD3 epsilon. |
| **SEQ ID NO: 30** | Nucleotide sequence of primer rbHCfinal.up. |
| **SEQ ID NO: 31** | Nucleotide sequence of primer rbHCfinal.do. |
| **SEQ ID NO: 32** | Nucleotide sequence of primer rbLCfinal.up. |
| **SEQ ID NO: 33** | Nucleotide sequence of primer rbLCfinal.do. |

### Description of the Figures

- **Figure 1:**: Schematic picture of human and cynomolgus CD3g(G4S)5CD3e-AcTev-Fc(knob)-Avi/ Fc(hole) fusion polypeptide.
- **Figure 2:**: SDS-Page Gels: 4-12 % Bis/Tris A) 1 - Mark 12 (Invitrogen), 2 - human CD3g(G4S)5CD3e-AcTev-Fc(knob)-Avi/ Fc(hole) reduced B) 1 - HiMark (Invitrogen), 2 - CD3g(G4S)5CD3e-AcTev-Fc(knob)-Avi/ Fc(hole) non reduced C) 1 - Mark 12 (Invitrogen), 2 - cynomolgus CD3g(G4S)5CD3e-AcTev-Fc(knob)-Avi/ Fc(hole) reduced; 3 - cynomolgus CD3g(G4S)5CD3e-AcTev-Fc(knob)-Avi/ Fc(hole) non reduced;
- **Figure 3:**: Schematic pictures of A) Human and cynomolgus CD3e stalkFc(knob)-Avi/CD3d-stalk-Fc(hole) fusion polypeptide, B) Human CD3e stalkFc(knob)-Avi/CD3gC85-stalk-Fc(hole) fusion polypeptide, C) Human CD3e -1-26 - Fc(knob)Avi/Fc(hole), human and cynomolgus CD3e 5-26 - Fc(knob)Avi/Fc(hole) fusion polypeptide,

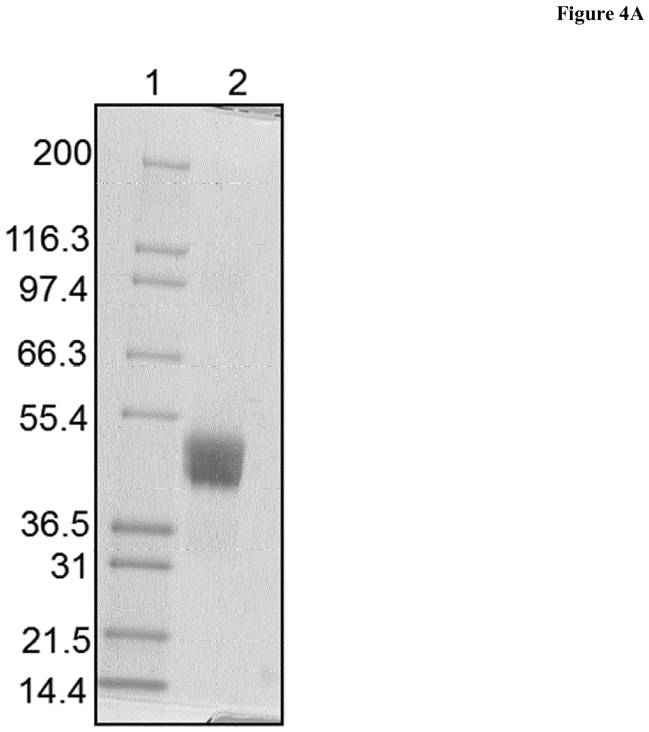

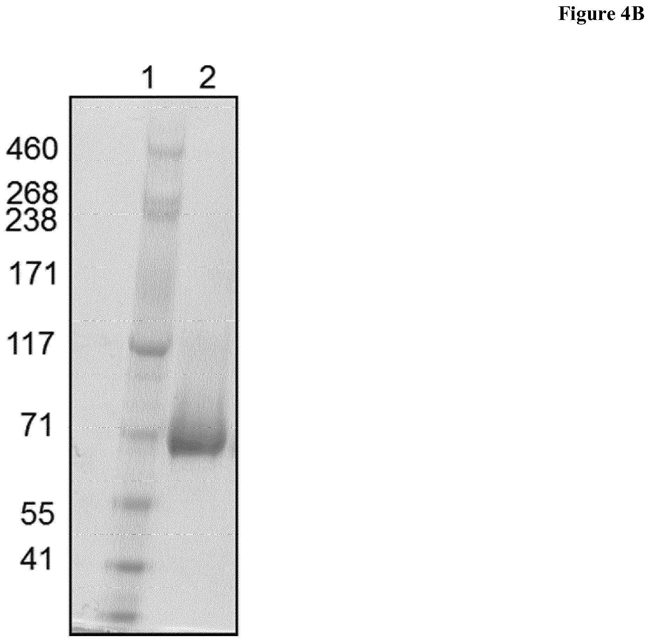

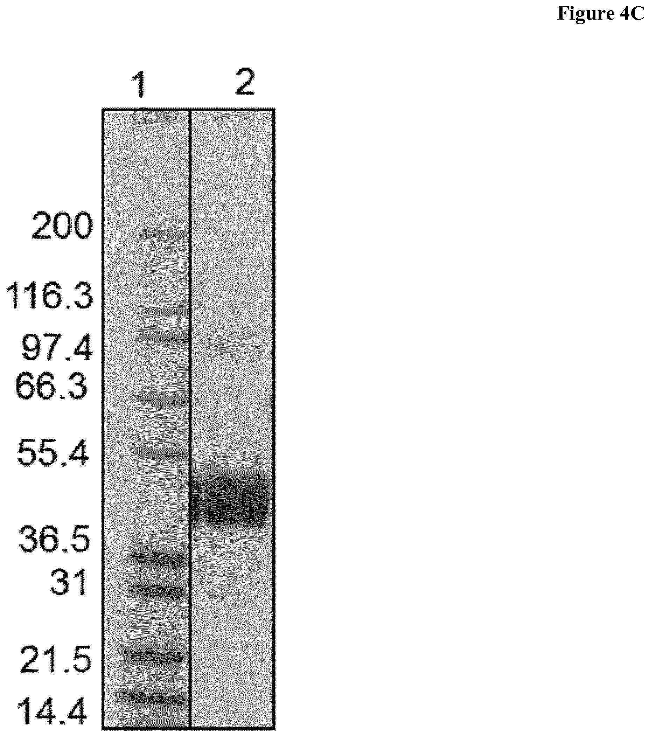

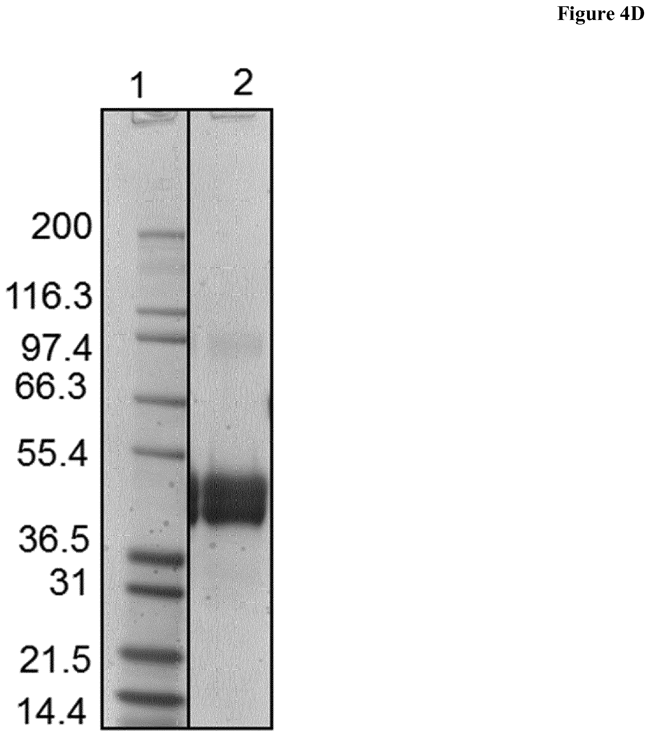

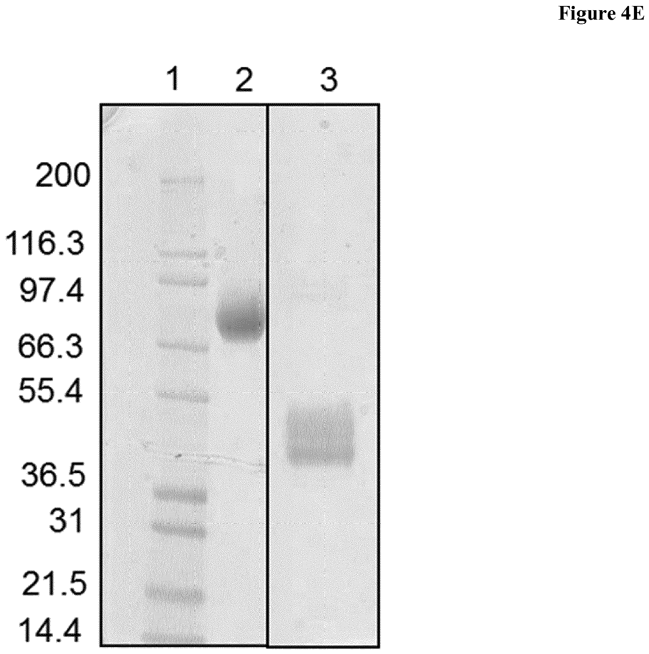

- **Figure 4:**: Representative SDS-Page Gels: 4-12 % Bis/Tris A) 1 - Mark 12 (invitrogen), 2 - human CD3e stalkFc(knob)-Avi/CD3d-stalk-Fc(hole) reduced; B) 1 - HiMark (Invitrogen), 2 - human CD3e stalkFc(knob)-Avi/CD3d-stalk-Fc(hole) non reduced;
- **Figure 5:**: Graphical representation of expansion of human T Lymphocytes
- **Figure 6:**: Calcium flux assay for assessing the ability of tested antibodies to activate human T cells (96-well format) A) Calcium flux assay without crosslinking of bound anti CD3 antibodies B) Calcium flux assay with crosslinking of bound anti CD3 antibodies for improved sensitivity
- **Figure 7:**: Calcium flux assay for assessing the ability of tested antibodies to activate human T cells (384-well format) A) Calcium flux assay without crosslinking of bound anti CD3 antibodies B) Calcium flux assay with crosslinking of bound anti CD3 antibodies for improved sensitivity
- **Figure 8:**: Comparison of binding of generated mAbs vs. anti-CD3 reference antibody (SP34-2) to human T cells
- **Figure 9:**: Comparison of binding of generated mAbs vs. anti-CD3 reference antibody (SP34-2) to cynomolgus T cells

### Material and Methods

### Example 1

### 1A) Preparation of recombinant human CD3g(G4S)5CD3e-AcTev-Fc(knob)-Avi/ Fc(hole) and cynomolgus CD3g(G4S)5CD3e-AcTev-Fc(knob)-Avi/ Fc(hole) for first immunization campaign

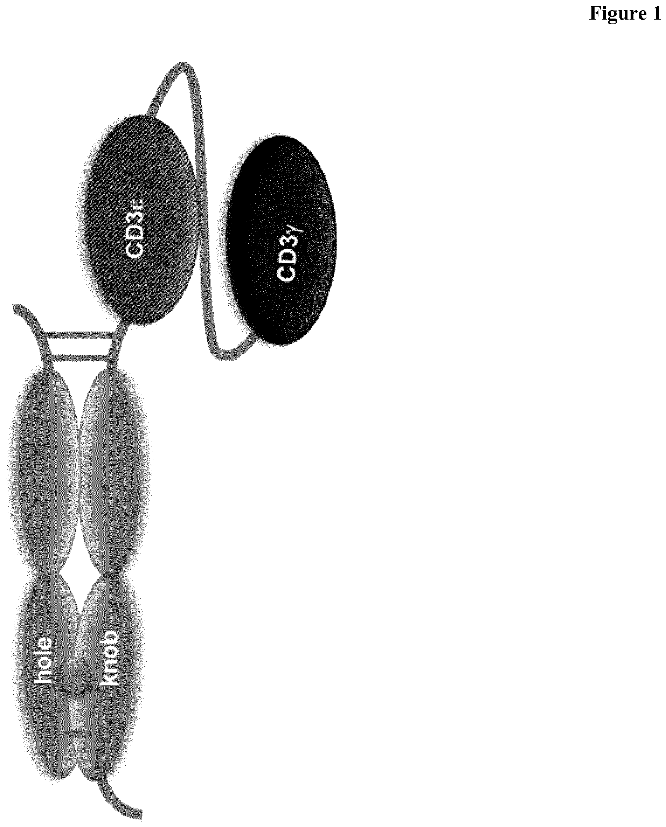

For the first immunization campaign human and cynomolgus recombinant proteins comprising CD3e in fusion with CD3g-chain were produced. Human CD3g(G4S)5CD3e-AcTev-Fc(knob)-Avi (SEQ ID NO: 16) and cynomolgus CD3g(G4S)5CD3e-AcTev-Fc(knob)-Avi (SEQ ID NO: 18) are recombinant proteins with the ectodomains of CD3e and CD3g connected by a Glycine-Serine linker ((G4S)5) fused to Fc(knob) with a C-terminal Avi-tag co-expressed with Fc(hole) (SEQ ID NO: 17) (Fig. 1).

The molecules are produced by co-transfecting HEK293-EBNA cells with the corresponding mammalian expression vectors using polyethylenimine (PEI). The cells are transfected with the corresponding expression vectors in a 1:1 ratio ("vector antigen-Fc(hole)" : "vector antigen-Fc(knob)").

HEK293-EBNA cells are cultivated in suspension serum free in CD CHO culture medium. For the production in 500 ml shake flask 400 million HEK293 EBNA cells are seeded 24 hours before transfection. For transfection cells are centrifuged for 5 min by 210 x g, supernatant is replaced by pre-warmed 20 ml CD CHO medium. Expression vectors are mixed in 20 ml CD CHO medium to a final amount of 200 µg DNA. After addition of 540 µl PEI solution is vortexed for 15 s and subsequently incubated for 10 min at room temperature. Afterwards cells are mixed with the DNA/PEI solution, transferred to a 500 ml shake flask and incubated for 3 hours by 37 °C in an incubator with a 5 % CO2 atmosphere. After incubation time 160 ml F17 medium is added and cell are cultivated for 24 hours. One day after transfection 1 mM valproic acid and 7 % Feed 1 is added. After 7 days cultivation supernatant is collected for purification by centrifugation for 15 min at 210 x g, the solution is sterile filtered (0.22 µm filter) and sodium azide in a final concentration of 0.01 % w/v is added, and kept at 4°C.

The secreted proteins are purified from cell culture supernatants by affinity chromatography using Protein A affinity chromatography, followed by a size exclusion chromatographic step.

For affinity chromatography supernatant is loaded on a HiTrap ProteinA HP column (CV=5 mL, GE Healthcare) equilibrated with 40 ml 20 mM sodium phosphate, 20 mM sodium citrate, 500 mM NaCl, 0.01% (v/v) Tween-20, pH 7.5. Unbound protein is removed by washing with at least 10 column volume equilibration buffer. Target protein is eluted in a linear pH-gradient over 20 column volume to 20 mM sodium citrate, 500 mM sodium chloride, 0.01% (v/v) Tween-20, pH 3.0. Column is washed subsequently with 10 column volume 20 mM sodium citrate, 500 mM NaCl, 0.01% (v/v) Tween-20, pH 3.0.

Protein solution is neutralized by adding 1/10 of 0.5M sodium phosphate. Target protein is concentrated prior loading on a HiLoad Superdex 200 column (GE Healthcare) equilibrated with 2 mM MOPS, 150 mM sodium chloride solution of pH 7.4 containing 0.01% (v/v) Tween-20.

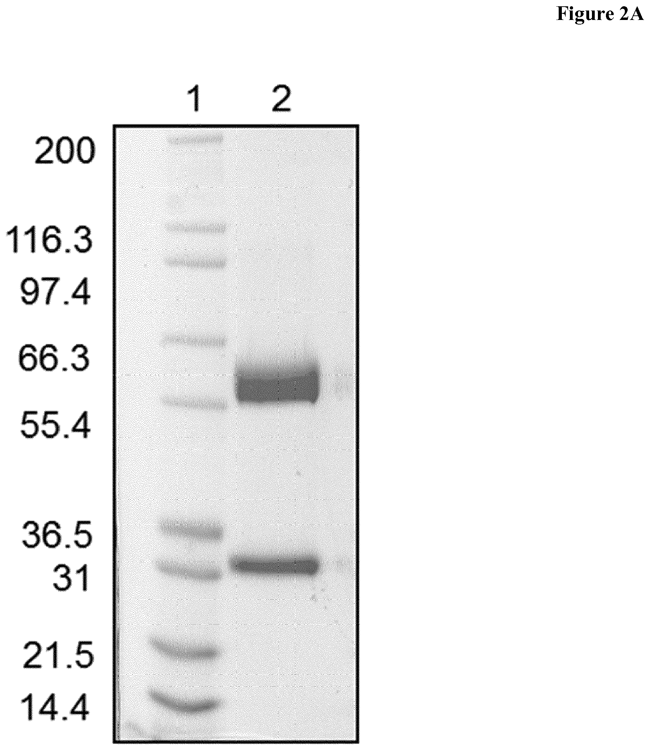

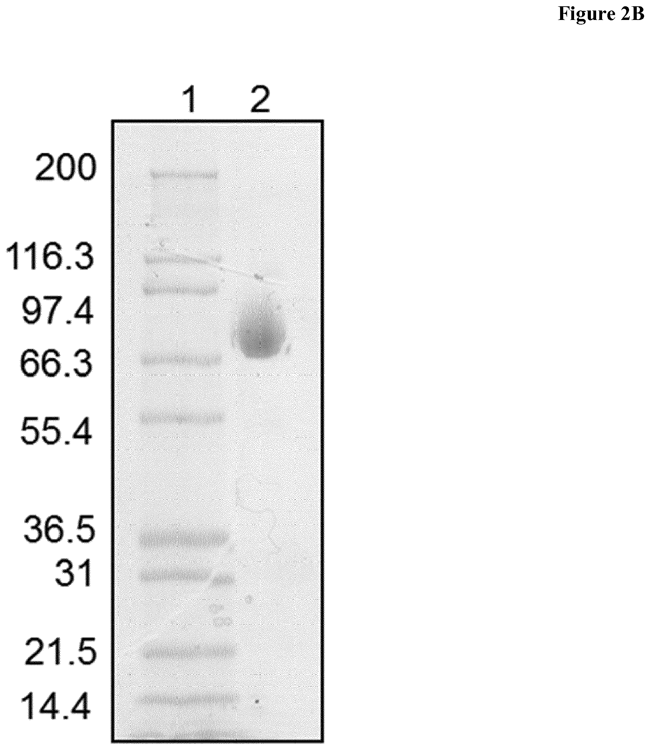

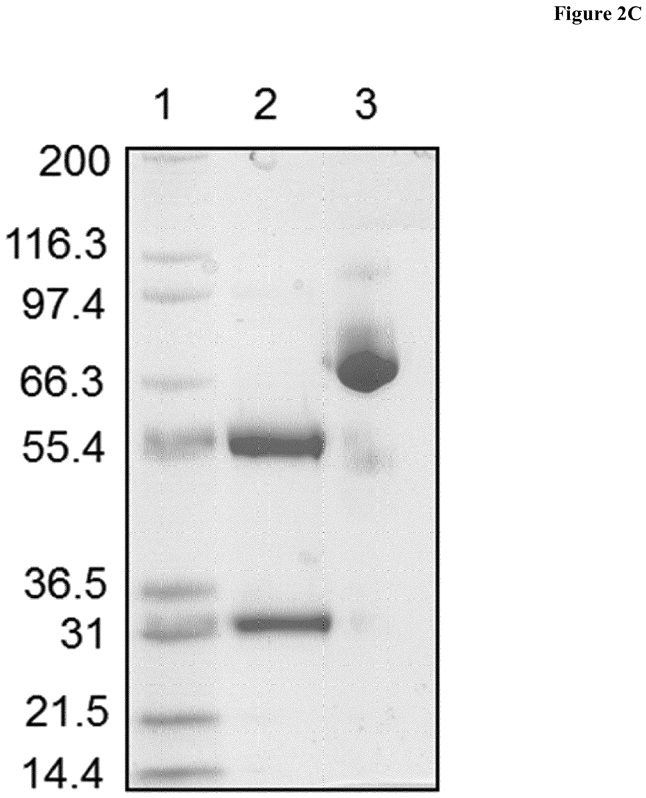

The protein concentration of purified protein samples is determined by measuring the optical density (OD) at 280 nm, using the molar extinction coefficient calculated on the basis of the amino acid sequence. Purity and molecular weight of antibodies are analyzed by SDS-PAGE in the presence and absence of a reducing agent (5 mM 1,4-dithiotreitol) and staining with Coomassie (SimpleBlue™ SafeStain, Invitrogen) (Fig. 2A to 2C). The NuPAGE® Pre-Cast gel system (Invitrogen, USA) is used according to the manufacturer's instruction (4-12% Tris-Acetate gels or 4-12% Bis-Tris). The aggregate content of antibody samples is analyzed using a Superdex200 10/300GL analytical size-exclusion column (Tosoh) equilibrated in 2 mM MOPS, 150 mM NaCl, 0.02 % (w/v) NaN3, pH 7.3 running buffer at 25°C.

**Table 2: Determination of aggregate content**

| Protein | Analytical SEC | | |
|---|---|---|---|
| | HMW [%] | Monomer [%] | LMW[%] |
| human CD3g(G4S)5CD3e-AcTev-Fc(knob)-Avi/ Fc(hole) | 0.95 | **99.05** | 0 |
| cynomolgus CD3g(G4S)5CD3e-AcTev-Fc(knob)-Avi/ Fc(hole) | 0 | **100** | 0 |

### 1B) Preparation of recombinant human CD3e-stalk-Fc(knob)-Avi/CD3d-stalk-Fc(hole), human CD3e-stalk-Fc(knob)-Avi/CD3gC85-stalk-Fc(hole), cynomolgus CD3e stalk-Fc(knob)-Avi/CD3d-stalk-Fc(hole), human CD3e -1-26 - Fc(knob)-Avi/Fc(hole), human CD3e 5-26 - Fc(knob)-Avi/Fc(hole) and cynomolgus CD3e 5-26 - Fc(knob)Avi/Fc(hole) for characterization of the IgGs resulting from immunization

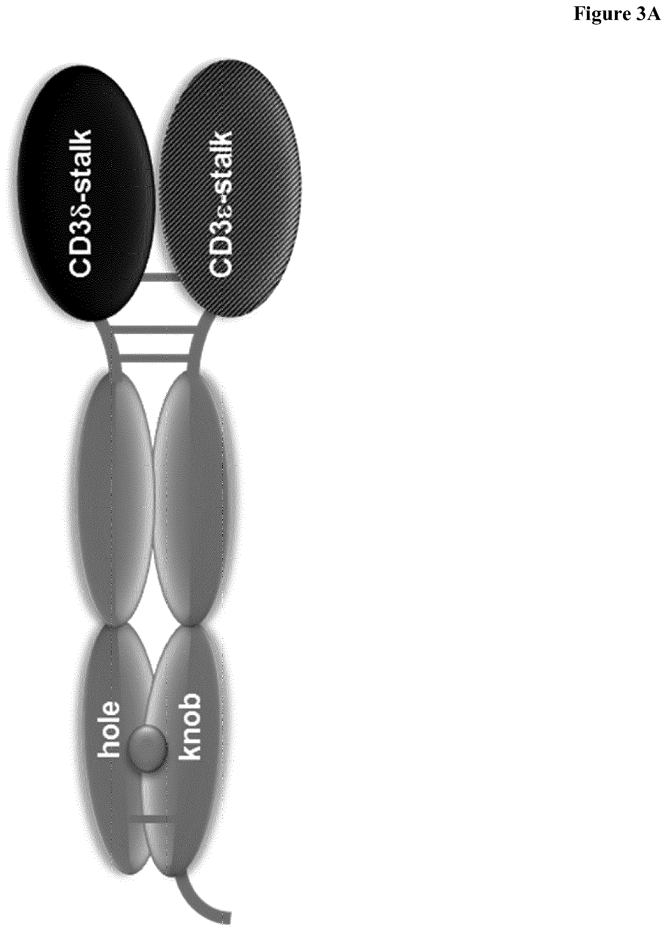

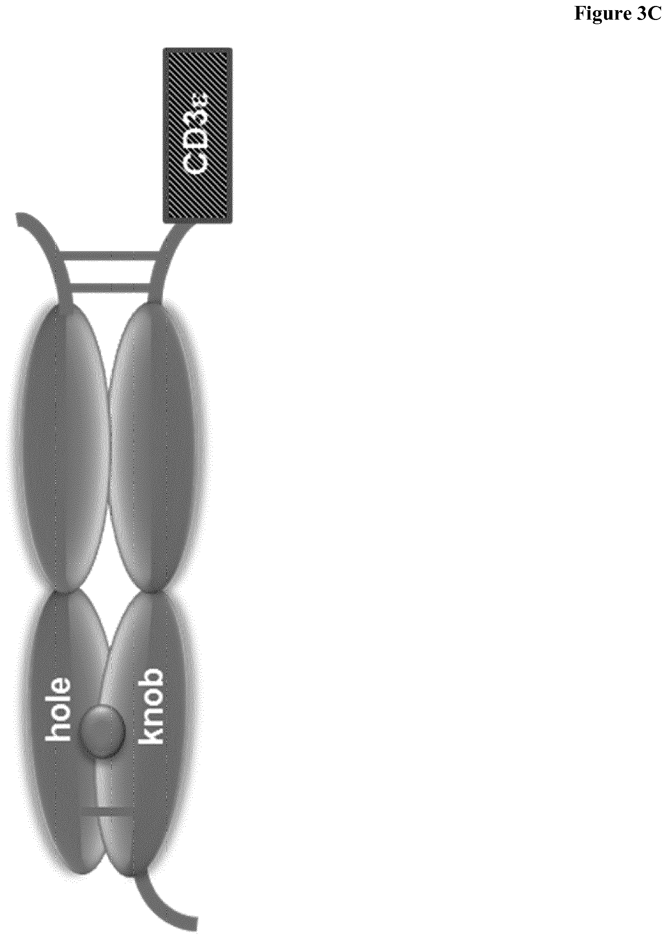

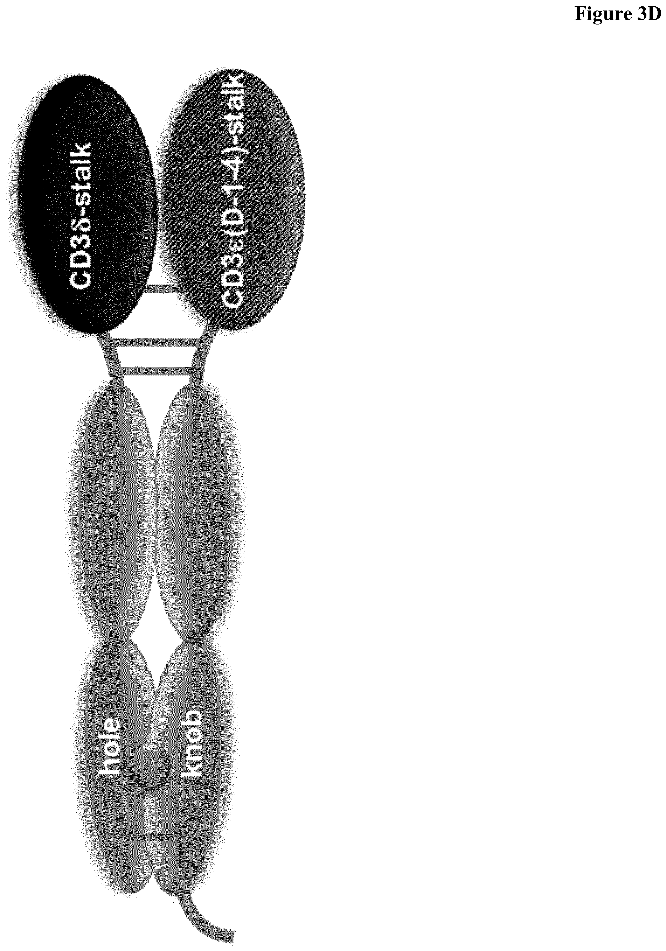

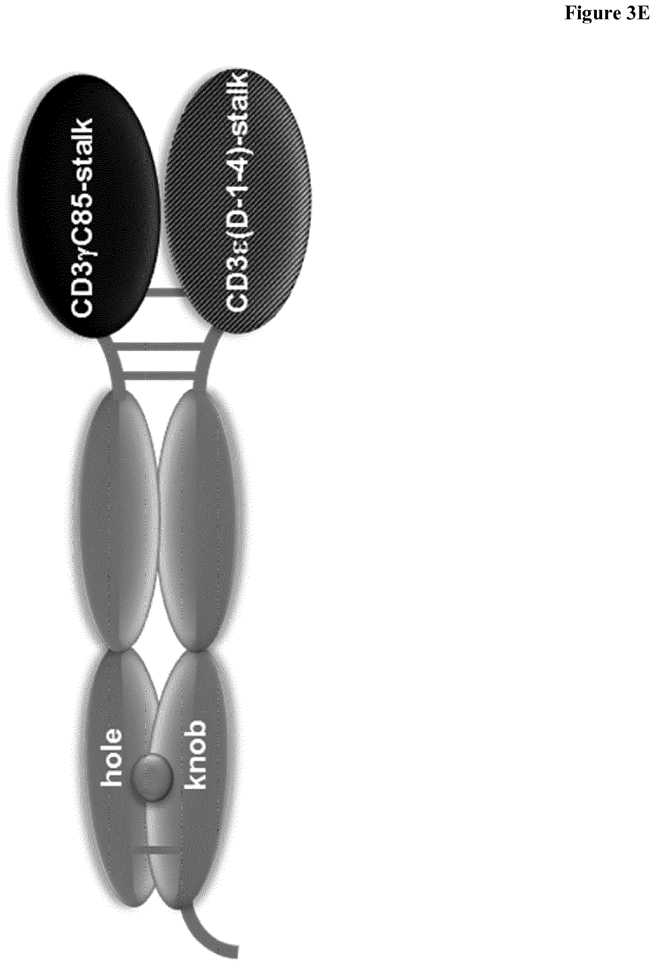

To characterize the antibodies against CD3e generated by immunization several recombinant proteins were produced (Fig. 3A to 3C). Human CD3e-stalk-Fc(knob)-Avi/CD3d-stalk-Fc(hole) (SEQ ID NO: 19/ SEQ ID NO: 20), human CD3e-stalk-Fc(knob)-Avi/CD3gC85-stalk-Fc(hole) (SEQ ID NO: 19/ SEQ ID NO: 21) and cynomolgus CD3e stalk-Fc(knob)-Avi/CD3d-stalk-Fc(hole) (SEQ ID NO: 22/ SEQ ID NO: 23) are recombinant proteins with the complete ectodomain of CD3e including the stalk region fused to Fc(knob) with a C-terminal Avi-tag co-expressed with the ectodomain of either CD3d or CD3g fused to Fc(hole). Human CD3e -1-26 - Fc(knob)-Avi/Fc(hole) (SEQ ID NO: 24/ SEQ ID NO: 17), human CD3e 5-26 - Fc(knob)-Avi/Fc(hole) (SEQ ID NO: 26/ SEQ ID NO: 17) and cynomolgus CD3e 5-26 - Fc(knob)-Avi/Fc(hole) (SEQ ID NO: 25/ SEQ ID NO: 17) are peptide fusion to Fc(knob) co-expressed with Fc(hole). Human CD3e -1-26 - Fc(knob)-Avi/Fc(hole) comprises the first 26 amino acids of mature CD3e whereas human and cynomolgus CD3e 5-26 - Fc(knob)Avi/Fc(hole) are peptide fusions of amino acid residues 5-26 of mature CD3e.

The molecules are produced by co-transfecting HEK293-EBNA cells with the corresponding mammalian expression vectors using polyethylenimine (PEI). The cells are transfected with the corresponding expression vectors in a 1:1 ratio ("vector antigen-Fc(hole)" : "vector antigen-Fc(knob)").

HEK293-EBNA cells are cultivated in suspension serum free in CD CHO culture medium. For the production in 500 ml shake flask 400 million HEK293 EBNA cells are seeded 24 hours before transfection. For transfection cells are centrifuged for 5 min by 210 x g, supernatant is replaced by pre-warmed 20 ml CD CHO medium. Expression vectors are mixed in 20 ml CD CHO medium to a final amount of 200 µg DNA. After addition of 540 µl PEI solution is vortexed for 15 s and subsequently incubated for 10 min at room temperature. Afterwards cells are mixed with the DNA/PEI solution, transferred to a 500 ml shake flask and incubated for 3 hours by 37 °C in an incubator with a 5 % CO2 atmosphere. After incubation time 160 ml F17 medium is added and cell are cultivated for 24 hours. One day after transfection 1 mM valproic acid and 7 % Feed 1 is added. After 7 days cultivation supernatant is collected for purification by centrifugation for 15 min at 210 x g, the solution is sterile filtered (0.22 µm filter) and sodium azide in a final concentration of 0.01 % w/v is added, and kept at 4°C.

The secreted proteins are purified from cell culture supernatants by affinity chromatography using Protein A affinity chromatography, followed by a size exclusion chromatographic step as described above.

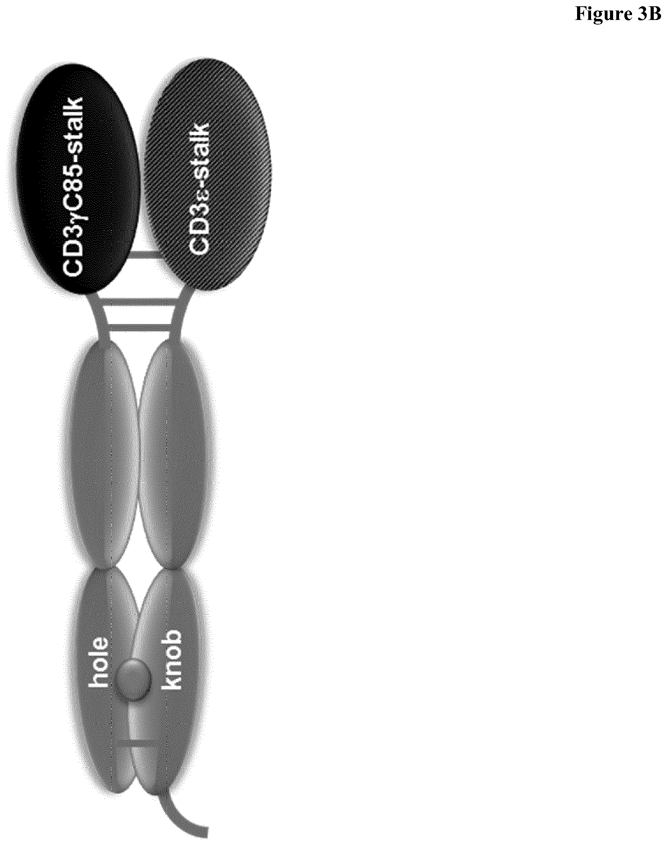

The protein concentration of purified protein samples is determined by measuring the optical density (OD) at 280 nm, using the molar extinction coefficient calculated on the basis of the amino acid sequence. Purity and molecular weight of antibodies are analyzed by SDS-PAGE in the presence and absence of a reducing agent (5 mM 1,4-dithiotreitol) and staining with Coomassie (SimpleBlue™ SafeStain, Invitrogen). The NuPAGE® Pre-Cast gel system (Invitrogen, USA) is used according to the manufacturer's instruction (4-12% Tris-Acetate gels or 4-12% Bis-Tris) (Fig. 3A and 3B). The aggregate content of antibody samples is analyzed using a TSKgel G3000 SW XL analytical size-exclusion column (Tosoh) equilibrated in 25 mM K2HPO4, 125 mM NaCl, 200mM L-Arginine Monohydrocloride, 0.02 % (w/v) NaN3, pH 6.7 running buffer at 25°C.

**Table 3: Determination of aggregate content**

| Protein | Analytical SEC | | |
|---|---|---|---|
| | HMW [%] | Monomer [%] | LMW[%] |
| human CD3e-stalk-Fc(knob)-Avi/CD3d-stalk-Fc(hole) | 4.3 | 95.7 | 0 |
| human CD3e-stalk-Fc(knob)-Avi/CD3gC85-stalk-Fc(hole) | 0 | **100** | 0 |
| cynomolgus CD3e stalk-Fc(knob)-Avi/CD3d-stalk-Fc(hole) | 0 | **100** | 0 |
| human CD3e -1-26 - Fc(knob)Avi/Fc(hole) | 0.7 | **86.3** | 13.0 |
| cynomolgus CD3e 5-26 - Fc(knob)Avi/Fc(hole) | 0 | **100** | 0 |
| human CD3e 5-26 - Fc(knob)Avi/Fc(hole) | 0 | **82.9** | 17.1 |

In addition, the following human and cynomolgus CD3e peptides were generated for refinement of the epitopes of the new antibodies:
a) Biotin-Linker-human CD3e amino acids 1-22
b) human CD3e amino acids 1-22-Linker-Biotin
c) Biotin-Linker- cynomolgus CD3e amino acids 1-22
d) cynomolgus CD3e amino acids 1-22-Linker-Biotin
e) Biotin-Linker- human CD3e amino acids 77-96 (SEQ ID NO: 01)
f) human CD3e amino acids 77-96 (SEQ ID NO: 01)-Linker-Biotin
g) Biotin-Linker- cynomolgus CD3e amino acids 69-88 (SEQ ID NO: 29)
h) cynomolgus CD3e amino acids 69-88 (SEQ ID NO: 29)-Linker-Biotin

These four peptides were synthesized with either an N- or C-terminal biotin in used as a mixture of both variants in the screening assay (if not stated otherwise) to enable the accessibility to all possible epitopes, e.g. if the biotinylated terminus would block one epitope from the antibody, the other biotin variant of the same peptide would allow binding of this antibody.

### Example 2

### Immunization of rabbits

### First immunization campaign

NZW rabbits from Charles River Laboratories International, Inc. were used for immunization.

Recombinant human and cynomolgus CD3g(G4S)5CD3e-AcTev-Fc(knob)-Avi proteins were solved in K₃PO₄ puffer pH 7.0 at a concentration of 1mg/ml and mixed (1:1) with complete Freund's adjuvant (CFA) till generation of stabile emulsion. Three rabbits received an intradermal (i.d.) injection of 2.4 ml of emulsion followed by a second intramuscular (i.m.) and third subcutaneous (s.c.) injection each with 1.2 ml in one week interval. The fourth i.m. injection of 1.2 ml was performed three weeks later followed by two further s.c. and i.m. injections of 1.2 ml in four weeks interval. Cynomolgus recombinant protein was used for 1^{st}, 2^{nd}, 4^{th} and 6^{th} and human recombinant protein for 3^{rd} and 5^{th} immunization.

10 ml peripheral whole blood samples of each animal was collected 4 days after third, fourth, fifth and sixth injection and used for single cell sorting in FACS. Additional 0.5 ml serum of each animal was collected at the same time and used for the determination of human/cyno CD3g(G4S)5CD3e-AcTev-Fc(knob)-Avi specific antibody response.

The second group of three rabbits was immunized with 4 x 10⁷ enriched primary cynomolgus/ human T cells (see below) according to the immunization schedule described above. Cynomolgus in vitro expanded T cells were used for 1^{st}, 2n^{d}, 4^{th} and 6^{th} and human primary T cells enriched from PBLs of healthy donors were used for 3^{rd} and 5^{th} immunization. 10 ml peripheral whole blood samples of each animal was collected 4-6 days after third, fourth, fifth and sixth injection and used for single cell sorting in FACS. Additional 0,5 ml serum of each animal was collected at the same time and used for the determination of human/cyno CD3g(G4S)5CD3e-AcTev-Fc(knob)-Avi specific antibody response.

### Antibody response

The antibody response to the immunization was determined by serial dilution of sera using an ELISA, in which 2.5 µg per well of the recombinant human/cyno CD3g(G4S)5CD3e-AcTev-Fc(knob)-Avi was incubated in 1x PBS at 4°C overnight on Maxisorb 96 wells microtiter plates (Nunc). For detection, goat anti-rabbit IgG linked to a horseradish peroxidase (The Jackson laboratory) was used at 1:16000 dilution. BM Blue POD substrate, precipitating Tetramethylbenzidine (TMB), ready-to-use solution (Roche) was used for visualization. Reaction was stopped via IN HCl and measured in Tecan Infinite by 450/690 nm.

### Generation of primary human T cells as antigen for immunization

The primary human T cells were isolated from 200 ml total blood of 6 healthy human donors by the RosetteSep Human T Cell Enrichment Cocktail (StemCell Technologies) following the instructions of the manufacturer. The quality and the cell numbers of the resulting T cells were confirmed by the cell counting device XT-1800 iVET (Sysmex) (Table 4). FACS analyses using the TriTest from BD detecting CD3-, CD4- and CD8-positive T cells were used for analysis of the quality and purity of the resulting T cells (Table 5). In addition, the viability of these T cells was assessed by PI (Propidium iodide) FACS staining and was over 99.6% viable cells for all samples. For storage until the immunization the T cells were frozen for each donor separately in liquid nitrogen as 4.6 x 10⁷ and 1.15 x 10⁷ cells using the cell numbers measured by the Sysmex device.

**Table 4: Cell numbers (Sysmex) after RosetteSep treatment**

| | White blood cells (WBC) [x10⁶] | Lymphocytes [x10⁶] | Lymphocytes [% of WBCs] |
|---|---|---|---|
| Donor 1 | 158 | 148.6 | 94 |
| Donor 2 | 208 | 196.9 | 94.7 |
| Donor 3 | 58.2 | 53.4 | 91.8 |
| Donor 4 | 126 | 119 | 94.4 |
| Donor 5 | 148 | 138 | 93.1 |
| Donor 6 | 148 | 142 | 96.4 |
| Sum | 846.2 | 798.9 | |

**Table 5: Quality of isolated T cells (TriTest FACS analysis)**

| | CD3+ [% PBMCs] | CD4+ [% PBMCs] | CD8+ [% PBMCs] |
|---|---|---|---|
| Donor 1 | 93.2 | 65.6 | 24.1 |
| Donor 2 | 92.6 | 58.7 | 28.8 |
| Donor 3 | 93.5 | 67.1 | 24.6 |
| Donor 4 | 89.9 | 64.5 | 23.3 |
| Donor 5 | 92.7 | 59.6 | 30.1 |
| Donor 6 | 90.1 | 55.6 | 31.0 |

### Preparation and in vitro expansion of cynomolgus T-lymphocytes

Lymphocytes were isolated from cynomolgus blood (Covance) by density gradient centrifugation using Ficoll-Paque (GE Healthcare). Briefly, 10 ml heparinized blood were diluted with the same volume of RPMI-1640 medium (Invitrogen) and 5 ml aliquots of the diluted blood were layered on top of 5 ml Ficoll-Paque in 15 ml Falcon tubes. After centrifugation at 800 x g for 45 min at room temperature (w/o break) the lymphocyte containing fractions were harvested, pooled and subjected to a second gradient centrifugation to increase the purity of the lymphocyte population. For this, approx. 6 ml of the pooled fraction were diluted with 18 ml RPMI-1640 medium and 6 ml aliquots of the diluted cell suspension were layered on top of 3 ml Ficoll-Paque in 15 ml Falcon tubes. After centrifugation at 800 x g for 30 min at room temperature (w/o break) the lymphocytes were harvested and pooled. Following washing with PBS lymphocytes were resuspended at 6.0E+05 cells/ml in RPMI-1640 medium supplemented with 10% fetal calf serum, 10 mM HEPES, 2 mM L-glutamine, 1 x NEAA, 1 mM sodium pyruvate and 1 x antibiotic-antimycotic (medium and supplements were purchased from Invitrogen). Cells were cultivated in the presence of 20 µg/ml concanavalin A (Sigma-Aldrich) for 3 days at 37°C, 5% CO2 in a humidified atmosphere. Thereafter the medium was exchanged and cells were cultivated in RPMI-1640 medium supplemented with 10% fetal calf serum, 10 mM HEPES, 2 mM L-glutamine, 1 x NEAA, 1 mM sodium pyruvate and 1 x antibiotic-antimycotic and 20 U/ml human IL-2 (Roche) for 9d. During this cultivation period the IL-2 containing medium was exchanged every 2-3 days. Cell viability and cell numbers were monitored throughout the cultivation period and the CD3 expression of the in vitro expanded cynomolgus T-lymphocytes was verified by flow-cytometry using an anti-CD3 mAb (clone SP34; BD Pharmingen). In vitro expanded T-lymphocytes (viability > 80 %) were harvested, washed with PBS and resuspended in freezing medium (10% DMSO, 90 %FCS) at 1.0E+07 cells/ml. Aliquots of the cells were stored in liquid nitrogen.

### Second immunization campaign

### Preparation and in vitro expansion of cynomolgus T-lymphocytes

Lymphocytes were isolated from cynomolgus blood (Covance) by density gradient centrifugation using Ficoll-Paque (GE Healthcare). Briefly, 10 ml heparinized blood were diluted with the same volume of RPMI-1640 medium (Invitrogen) and 9-10 ml aliquots of the diluted blood were layered on top of 5 ml Ficoll-Paque in 15 ml Falcon tubes. After centrifugation at 800 x g for 45 min at room temperature (w/o break) the lymphocyte containing fractions were harvested, pooled and subjected to a second gradient centrifugation to increase the purity of the lymphocyte population. For this, approx. the pooled fraction were diluted with RPMI-1640 medium to the diluted initial volume and 9-10 ml aliquots of the diluted cell suspension were layered on top of 5 ml Ficoll-Paque in 15 ml Falcon tubes. After centrifugation at 800 x g for 30 min at room temperature (w/o break) the lymphocytes were harvested and pooled. Following washing with PBS lymphocytes were resuspended at 6.0E+05 cells/ml in RPMI-1640 medium supplemented with 10% fetal calf serum, 10 mM HEPES, 2 mM L-glutamine, 1 x NEAA, 1 mM sodium pyruvate and 1 x antibiotic-antimycotic (medium and supplements were purchased from Invitrogen). Cells were cultivated in the presence of 20 µg/ml concanavalin A (Sigma-Aldrich) and 20 U/ml human IL-2 (Roche) for 3 days at 37°C, 5% CO2 in a humidified atmosphere. Thereafter the medium was exchanged and cells were cultivated in RPMI-1640 medium supplemented with 10% fetal calf serum, 10 mM HEPES, 2 mM L-glutamine, 1 x NEAA, 1 mM sodium pyruvate and 1 x antibiotic-antimycotic and 20 U/ml human IL-2 (Roche) for 9d. During this cultivation period the IL-2 containing medium was exchanged every 2-3 days. Cell viability and cell numbers were monitored throughout the cultivation period and the CD3 expression of the in vitro expanded cynomolgus T-lymphocytes was verified by flow-cytometry using an anti-CD3 mAb (clone SP34; BD Pharmingen). In vitro expanded T-lymphocytes (viability > 90 %) were harvested, washed with PBS and resuspended in freezing medium (10% DMSO, 90 %FCS) at 1.0E+07 cells/ml. Aliquots of the cells were stored in liquid nitrogen.

### Preparation and in vitro expansion of human T-lymphocytes

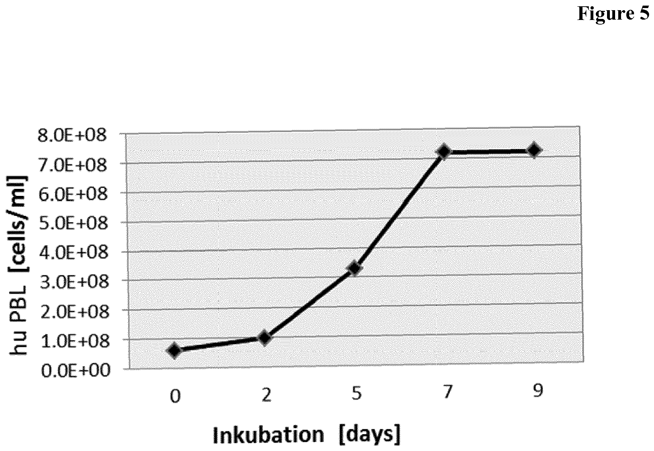

Lymphocytes were isolated from peripheral blood of healthy donor by density gradient centrifugation using Leukosep (Greiner Bio One, 227 288). Briefly, heparinized blood was diluted with the three fold volume of PBS and 25 ml aliquots of the diluted blood were layered in 50 ml Leukosep tubes. After centrifugation at 800 x g for 15 min at room temperature (w/o break) the lymphocyte containing fractions were harvested, washed in PBS and resuspended at 1.0E+06 cells/ml in RPMI-1640 medium supplemented with 10% fetal calf serum, 10 mM HEPES, 2 mM L-glutamine, 1 x NEAA, 1 mM sodium pyruvate and 1 x antibiotic-antimycotic (medium and supplements were purchased from Invitrogen). Cells were cultivated in the presence of 10 µg/ml concanavalin A (Sigma-Aldrich) in T175 flask for 2 days at 37°C, 5% CO2 in a humidified atmosphere. Thereafter the medium was exchanged and cells were cultivated in RPMI-1640 medium supplemented with 10% fetal calf serum, 10 mM HEPES, 2 mM L-glutamine, 1 x NEAA, 1 mM sodium pyruvate and 1 x antibiotic-antimycotic and 20 U/ml human IL-2 (Roche) for further 7d. During this cultivation period cells were split to 1.0E+06 cells/ml every 2-3 days and the IL-2 containing medium was exchanged. Cell viability and cell numbers were monitored throughout the cultivation period (Figure 5) and the CD3 expression of the in vitro expanded human T-lymphocytes was verified by flow-cytometry using an anti-CD3 mAb (clone V9). In vitro expanded T-lymphocytes (viability > 90 %) were harvested, washed with PBS and resuspended in freezing medium (10% DMSO, 90 %FCS) at 1.0E+07 cells/ml. Aliquots of the cells were stored in liquid nitrogen.

### Immunization

Three rabbits were immunized with cynomolgus and human PBLs which had been enriched for T cells (as described above). For each immunization, frozen cells were thawed and counted, separated from the freezing media by centrifugation, and resuspended in PBS, in an adequate volume for the injections. Each rabbit received one intradermal application of 6 x 10⁷ cynomolgus PBLs, resuspended in PBS, at day 0; followed by one intramuscular and one subcutaneous application of 4 x 10⁷ cynomolgus PBLs each, at days 7 and 14, and by a first bleed on day 21.

For 3 additional immunizations of 3-5 x 10⁷ PBLs each, species of origin as well as route of application was alternated: the animals received two intramuscular applications of human PBLs at week 7 and at week 20, and one subcutaneous application of cynomolgus PBLs at week 11.

Blood (10% of estimated total blood volume) was taken on day 21 and 5-7 days after each of the additional immunizations. Serum was prepared, which was used for titer determination by FACS, and peripheral mononuclear cells were isolated, which were used as a source of antigen-specific B cells in the B cell cloning process (Example 3).

### Example 3

### B cell cloning

### Isolation of rabbit peripheral blood mononuclear cells (PBMC)

Three rabbits (described in the Example "Immunization of rabbits") were used as a source of blood. EDTA containing whole blood was diluted twofold with 1x PBS (PAA, Pasching, Austria) before density centrifugation using lympholyte mammal (Cedarlane Laboratories, Burlington, Ontario, Canada) according to the specifications of the manufacturer. The PBMCs were washed twice with 1x PBS.

### EL-4 B5 medium

RPMI 1640 (Pan Biotech, Aidenbach, Germany) supplemented with 10% FCS (Hyclone, Logan, UT, USA), 2mM Glutamine, 1% penicillin/streptomycin solution (PAA, Pasching, Austria), 2mM sodium pyruvate, 10mM HEPES (PAN Biotech, Aidenbach, Germany) and 0,05 mM b-mercaptoethanol (Gibco, Paisley, Scotland)

### Depletion of macrophages/monocytes

Sterile 6-well plates (cell culture grade) were used to deplete macrophages and monocytes through unspecific adhesion. Each well was filled at maximum with 4 ml medium and up to 6x10⁶ PBMCs from the immunized rabbit and allowed to bind for 1 h at 37 °C in the incubator. The cells in the supernatant (peripheral blood lymphocytes (PBLs)) were used for the antigen panning step.

### Coating of plates

For panning on protein sterile streptavidin coated 6-well plates (Microcoat, Bernried, Germany) were coated with 2 µg/ml biotinylated human CD3e protein variants (see Table 6) in PBS for 3 h at room temperature. Each protein variant was coated separately. To enable panning on human surface CD3-cells, CD3-positive Jurkat T cells were seeded in sterile cell culture 6-well plates without fetal calf serum (FCS) and were immediately centrifuged. After 1-4 h of cultivation a confluent cell monolayer was generated. Prior to the panning these 6-well plates were carefully washed with sterile PBS three times.

### Enrichment of B cells on the human CD3e protein variants

For enrichment of antigen specific peripheral B cells 6-well tissue culture plates coated with human CD3e protein variants or covered with human CD3-positive Jurkat T cells were seeded with up to 6x10⁶ PBLs per 4 ml medium and allowed to bind for 1 h at 37°C under 5% CO2. Afterwards the non-adherent cells were removed by carefully washing the wells 1-2 times with 1x PBS. The remaining sticky cells were detached by trypsin for 10 min at 37°C under 5% CO2. Trypsination was stopped with EL-4 B5 medium. The cells were kept on ice until the immune fluorescence staining.

**Table 6: B-cell enrichment strategy for the antibodies of interest**

| **Clone** | **Bleed** | **Cell treatment** |
|---|---|---|
| 417 | 3 | MAbr |
| 426 | 3 | huCD3+ |
| 432 | 3 | huCD3+ |
| 446 | 3 | cynoCD3+ |
| 450 | 3 | cynoCD3+ |
| 463 | 3 | cynoCD3+ |
| 590 | 4 | huCD3+ |
| 596 | 4 | cynoCD3+ |
| 621 | 4 | huCD3+ |
| 627 | 4 | cynoCD3+ |
| 628 | 4 | cynoCD3+ |
| 632 | 4 | Jurkat E6.1+ |
| 645 | 1 | MAbr, huCD3 Protein+ |
| 647 | 1 | MAbr |
| 659 | 1 | MAbr, huCD3 Peptid (77-96)+ |
| 693 | 3 | MAbr, huCD3 Peptid (77-96)+ |
| 695 | 3 | MAbr, huCD3 Protein+ |
| 704 | 2 | MAbr |

### Immune fluorescence staining and Flow Cytometry

The anti-IgG FITC (AbD Serotec, Düsseldorf, Germany) was used for single cell sorting. For surface staining, cells from the depletion and enrichment step were incubated with the anti-IgG FITC antibody in PBS and incubated for 45 min in the dark at 4°C. After staining the PBMCs were washed two fold with ice cold PBS. Finally the PBMCs were resuspended in ice cold PBS and immediately subjected to the FACS analyses. Propidium iodide in a concentration of 5 µg/ml (BD Pharmingen, San Diego, CA, USA) was added prior to the FACS analyses to discriminate between dead and live cells.

A Becton Dickinson FACSAria equipped with a computer and the FACSDiva software (BD Biosciences, USA) were used for single cell sort.

### B-cell cultivation

The cultivation of the rabbit B cells was prepared by a method similar to that described by Zubler et al. (1985). Briefly, single sorted rabbit B cells were incubated in 96-well plates with 200 µl/well EL-4 B5 medium containing Pansorbin Cells (1:100000) (Calbiochem (Merck), Darmstadt, Deutschland), 5% rabbit thymocyte supernatant (charge TSN-M13 (10242), MicroCoat, Bernried, Germany) and gamma-irradiated murine EL-4-B5 thymoma cells (2,5 × 10⁴/well) for 7 days at 37°C in an atmosphere of 5% CO₂ in the incubator. The supernatants of the B-cell cultivation were removed for screening and the remaining cells were harvested immediately and were frozen at - 80°C in 100 µl RLT buffer (Qiagen, Hilden, Germany).

### PCR amplification of V-domains and sequencing

Total RNA was prepared using the NucleoSpin 8/96 RNA kit (Macherey&Nagel; 740709.4, 740698) according to manufacturer's protocol. All steps were done on a epMotion 5075 liquid handling system (Eppendorf). RNA was eluted with 60 µl RNase free water. 6µl of RNA was used to generate cDNA by reverse transcriptase reaction using the Superscript III First-Strand Synthesis SuperMix (Invitrogen 18080-400) and an oligo dT-primer according to the manufacturer's instructions. 4µl of cDNA were used to amplify the immunoglobulin heavy and light chain variable regions (VH and VL) with the AccuPrime Supermix (Invitrogen 12344-040) in a final volume of 50µl using the primers rbHCfinal.up and rbHCfinal.do for the heavy chain and rbLCfinal.up and rbLCfinal.do for the light chain (SEQ ID NOs: 30 to 33). The PCR conditions were as follows: Hot start at 94°C for 5 min; 35 cycles of 20s at 94°C, 20s at 70°C, 45s at 68°C, and a final extension at 68°C for 7 min.

8µl of 50µl PCR solution were loaded on a 48 E-Gel 2% (Invitrogen G8008-02). Positive PCR reactions were cleaned using the NucleoSpin Extract II kit (Macherey&Nagel; 740609250) according to manufacturer's protocol and eluted in 50µl elution buffer. 12µl of purified PCR products were sequenced directly in both directions using the rbHCfinal.up and rbHCfinal.do for heavy chains and rbLCfinal.up and rbLCfinal.do for light chains (SEQ ID NOs: 30 to 33).

### Recombinant expression of rabbit monoclonal antibodies and rabbit/mouse chimeric antibodies.

For recombinant expression of rabbit monoclonal antibodies, PCR-products coding for VH or VL were cloned as cDNA into expression vectors by the overhang cloning method (RS Haun et al., Biotechniques (1992) 13, 515-518; MZ Li et al., Nature Methods (2007) 4, 251-256).

Linearized expression plasmids coding for the rabbit kappa or gamma constant region and VL of VH inserts were amplified by PCR using overlapping primers.

Purified PCR products were incubated with T4 DNA-polymerase which generated single-strand overhangs. The reaction was stopped by dCTP addition.

In the next step, plasmid and insert were combined and incubated with recA which induced site specific recombination. The recombined plasmids were transformed into E.coli. The next day the grown colonies were picked and tested for correct recombined plasmid by plasmid preparation, restriction analysis and DNA-sequencing.

For antibody expression, the isolated HC and LC plasmids were transiently co-transfected into HEK293 cells and the supernatants were harvested after 1 week.

### Example 4

### Functional activity assay (Ca Flux) with human and cynomolgus T cells

### Establishment of a CD3 mediated calcium flux assay

In order to screen the functional activity of the anti-CD3 mAbs a calcium flux assay was established using CD3-positive (Jurkat E6-1) and CD3-negative (Jurkat RT3-T3.5) human T-cell lines. The assay was performed in a 96-well format (secondary screening) or in a 384-well format (primary high throughput screening).

### 96-well format

CD3-positive Jurkat E6-1 cells or CD3-negative Jurkat RT3-T3.5 were plated in black-walled, clear bottom 96-well plates (BD Falcon) at 200,000 cells in 50 µl serum-free medium (RPMI 1640 / 2 mM Glutamine / 1mM sodium pyruvate / 10 mM Hepes / 0.1mM NEAA) per well. Cells were loaded with the calcium sensitive dye (FLIPR® Calcium 5 Assay Kit, Molecular Devices). A stock solution of the dye was prepared according to the manufacturer's instructions. Directly before use Propenecid was added and 50 µl/well of the diluted dye were added to the cells (final concentration of Probenecid will be 2.5 mM/well). For efficient loading cells were incubated with the dye for 2h at room temperature in the dark. Subsequently, cells were stimulated by the addition of 20 µl rabbit anti-CD3 mAb (rabbit B-cell supernatants) or serial dilutions of chimeric V9 mAb, a chimeric anti-CD3 antibody consisting of rabbit immunoglobulin constant regions and the variable regions of the humanized anti-CD3 mAb V9. Unspecific polyclonal rabbit IgG served as negative control. The kinetic of the anti-CD3 induced calcium flux was monitored by measuring the fluorescence (485 nm ex. / 530 nm em.) at 30 s time intervals for 7.5-10 min. The calcium flux induced by the chimeric V9 mAb is shown in Fig. 6A. This data demonstrate that the assay allows the detection of agonistic anti-CD3 mAbs with a minimum concentration of approx. 37 ng/ml (final mAb concentration in the assay: 6.2 ng/ml; signal-to-noise ratio > 2). The chimeric V9 mAb induced calcium mobilization only in CD3-positive Jurkat E6-1 cells and not in CD3-negative Jurkat RT3-T3.5 cells demonstrating the CD3 dependency of the effect. Likewise, there was no calcium flux observed when cells were treated with unspecific rabbit IgG.

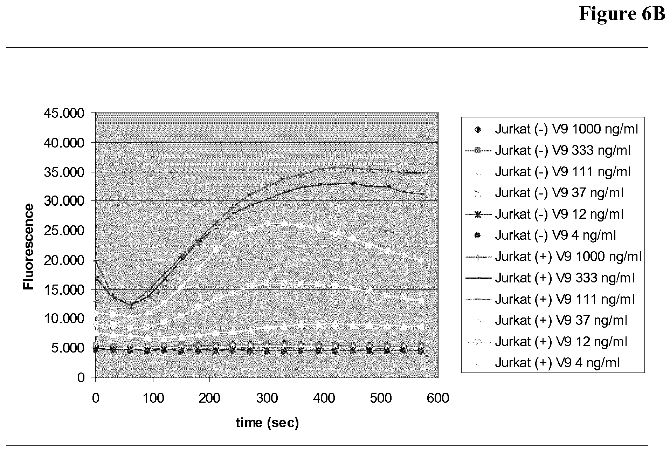

To increase the sensitivity of the assay an additional step was introduced to crosslink CD3 bound mAbs at the surface of the cells. Therefore the cells were stimulated with anti-CD3 mAbs (rabbit B-cell supernatants or chimeric V9 mAb) as described above and the initial CD3 mediated calcium flux was monitored for 7.5-10 min. Thereafter, CD3 bound mAbs were cross-linked by the addition of 20 µl Fc-specific goat anti-rabbit IgG (c=7.5 µg/ml; JacksonImmunoResearch) and the fluorescence (485 nm ex. /530 nm em.) was recorded for additional 7.5 - 10 min. As shown in Fig. 6B the cross-linking of cell surface bound anti-CD3 mAbs (chimeric V9) by the secondary anti-rabbit antibody induces an additional calcium flux with an improved signal-to-noise ratio compared to the initial signal. This modification of the assay improved the sensitivity of the assay and allows the detection of anti-CD3 mAbs at concentrations as low as approx. 12 ng/ml (final mAb concentration in the assay: 2 ng/ml).

### 384-well format

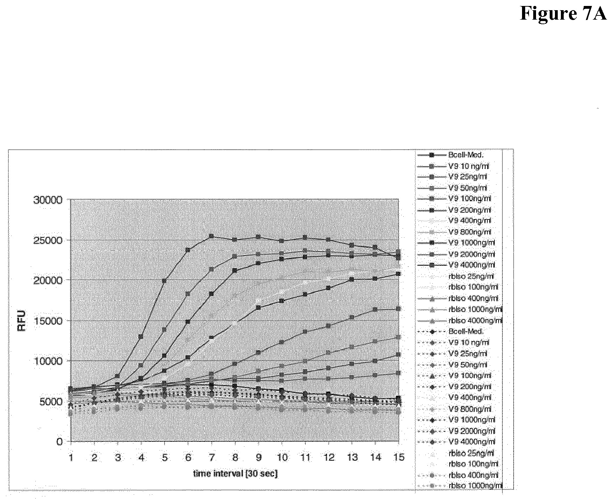

CD3-positive Jurkat E6-1 cells or CD3-negative Jurkat RT3-T3.5 were plated in black-walled, clear bottom 384-well plates (Corning) at 100,000 cells in 25 µl serum-free medium (RPMI 1640 / 2 mM Glutamine / 1mM sodium pyruvate / 10 mM Hepes / 0.1mM NEAA) per well. Cells were loaded with the calcium sensitive dye (FLIPR® Calcium 5 Assay Kit, Molecular Devices). A stock solution of the dye was prepared according to the manufacturer's instructions. Directly before use Propenecid was added and 25 µl/well of the diluted dye were added to the cells (final concentration of Probenecid will be 2.5 mM/well). For efficient loading cells were incubated with the dye for 2 h at room temperature in the dark. Subsequently, cells were stimulated by the addition of 10 µl rabbit anti-CD3 mAb (rabbit B-cell supernatants) or serial dilutions of chimeric V9 mAb, a chimeric anti-CD3 antibody consisting of rabbit immunoglobulin constant regions and the variable regions of the humanized anti-CD3 mAb V9. Unspecific polyclonal rabbit IgG served as negative control. The kinetic of the anti-CD3 induced calcium flux was monitored by measuring the fluorescence (485 nm ex. / 530 nm em.) at 30 s time intervals for 7.5 - 10 min. The calcium flux induced by the chimeric V9 mAb is shown in Figure7A. These data demonstrate that the assay allows the detection of agonistic anti-CD3 mAbs with a minimum concentration of approx. 25 ng/ml (final mAb concentration in the assay: 4.2 ng/ml; signal-to-noise ratio > 2). The chimeric V9 mAb induced calcium mobilization only in CD3-positive Jurkat E6-1 cells and not in CD3-negative Jurkat RT3-T3.5 cells demonstrating the CD3 dependency of the effect. Likewise, there was no calcium flux observed when cells were treated with unspecific rabbit IgG (isotype control).

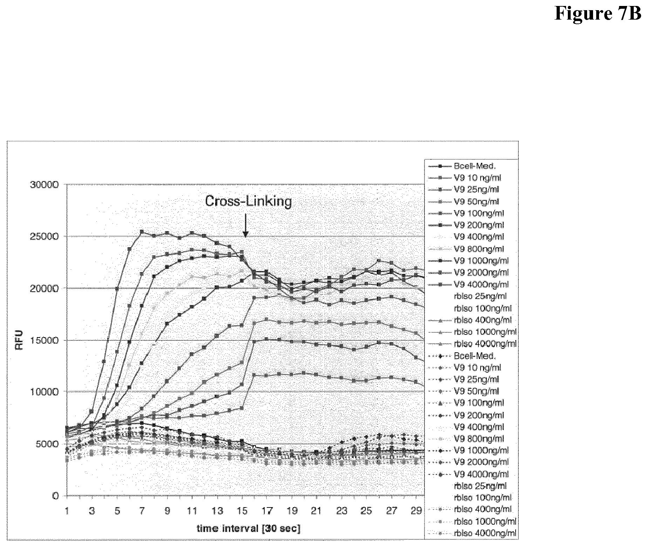

To increase the sensitivity of the assay an additional step was introduced to crosslink CD3 bound mAbs at the surface of the cells. Therefore the cells were stimulated with anti-CD3 mAbs (rabbit B-cell supernatants or chimeric V9 mAb) as described above and the initial CD3 mediated calcium flux was monitored for 7.5 - 10 min. Thereafter, the CD3 bound mAbs were cross-linked by the addition of 10 µl Fc-specific goat anti-rabbit IgG (c=7.5 µg/ml; JacksonImmunoResearch) and the fluorescence (485 nm ex. / 530 nm em.) was recorded for additional 7.5 - 10 min. As shown in Figure 7B the cross-linking of cell surface bound anti-CD3 mAbs (chimeric V9) by the secondary anti-rabbit antibody induces an additional calcium flux with an improved signal-to-noise ratio at low concentrations of the anti-CD3 mAb. This modification of the assay improved the sensitivity of the assay and allows the detection of anti-CD3 mAbs at concentrations as low as approx. 10 ng/ml (final mAb concentration in the assay: 1.7 ng/ml).

**Table 7: Calcium flux assay**

| **Clone** | **Calcium influx** |
|---|---|
| 417 | yes |
| 426 | no |
| 432 | no |
| 446 | no |
| 450 | yes |
| 463 | no |
| 590 | no |
| 596 | no |
| 621 | no |
| 627 | yes |
| 628 | no |
| 632 | no |
| 645 | yes |
| 647 | no |
| 659 | no |
| 693 | yes |
| 695 | yes |
| 704 | yes |

### Example 5

### Binding to human and cynomolgus CD3 proteins and peptides

Binding of anti-CD3 antibodies to human and cynomolgus CD3 proteins and peptides was determined by ELISA. Biotinylated target proteins and peptides were immobilized on a 384-well streptavidin-coated microplate (MaxiSorb; MicroCoat, DE, Cat.No. 11974998/MC1099) in 25 µl/well, in DPBS (PAN Biotech GmbH, DE, Cat.No. P0436500) by incubation over night at 4°C. Target concentrations were 250 ng/ml for all proteins and peptides mentioned in Table 8, with the exception of cynomolgus CD3 peptide -1-22 and human CD3 peptide 77-96, which were immobilized in a concentration of 1000 ng/ml. Peptides used were produced with biotin attached via linkers either to the N- or the C-terminus of the peptide. For target binding ELISA N- and C-terminal biotinylated peptides were mixed in a 1:1 ratio for immobilization on microplates (Table 8) and used separately (Table 8). After three washing steps with washing buffer (0.1% Tween 20 (USB, Cat.No. 20605) in IX PBS (Roche, Cat.No. 1666789)) recombinant anti-CD3 antibodies (25 µl/well, dilution series in 0.5% BSA (Bovine Serum Albumin Fraction V, fatty acid free, Roche, #10735078001), 0.05% Tween 20 in IX PBS) were added and incubated an orbital shaker at room temperature for 1h followed by three washing steps with washing buffer (90 µl/well). Antibodies were detected with peroxidase-linked, species-specific anti-rabbit IgG, (F(ab')2 fragment, from donkey, GE, Cat.No. NA 9340) diluted 1:5000 in IX PBS (w/0.5% BSA, w/0.05% Tween 20) for 1 h at room temperature. Detection antibodies were removed by four washing steps with washing buffer and signal was developed by addition of TMB substrate (TMB solution, Roche). Absorbance was read out after fixed time intervals at EX370nm/EM492nm.

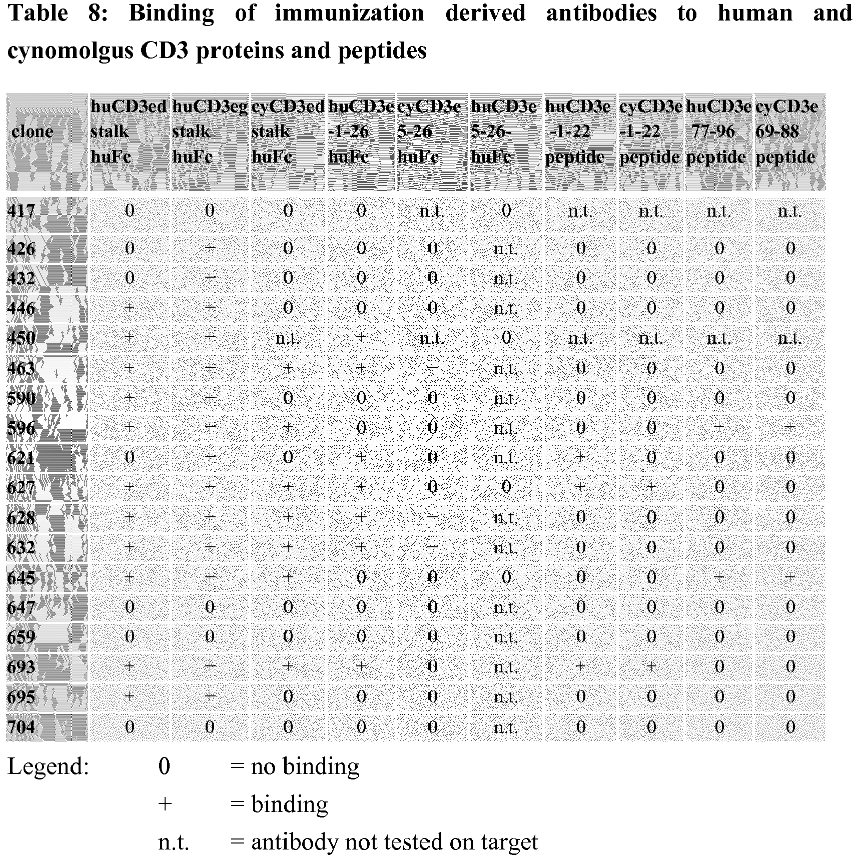

Results from binding ELISA show that clone 596 and clone 645 do bind human and cynomolgus CD3e in an epitope region consisting of amino acids 77-96 (human CD3e) and 69-88 (cynomolgus CD3e), respectively.

### FACS based cellular binding studies: Binding to human and cynomolgus expanded T cells

For the evaluation of cellular binding of generated anti-CD3 antibodies the FACS based binding assay with human and cynomolgus expanded T cells was performed. Briefly, frozen T cells (Example 2) were thawed, separated from the freezing media by centrifugation, and suspended in Jurkat cell medium 2 x 106 cell/ml. 50 µl cell aliquots were incubated with serial dilutions (10µg/ml-0,01 µg/ml in BD FACS buffer) of anti-CD3 antibodies for 1h at 4°C. Following washing with BD FACS buffer cells were stained depend on their origin with Alexa488 labeled anti-rabbit IgG H+L (Invitrogen 34732A) or anti-mouse IgG H+L (Invitrogen 65E1-1) or antihuman IgG H+L (Invitrogen A11013) 10µg/ml in BD FACS buffer) for 1h at 4°C. After washing and centrifugation MFI signals of stained cells were analyzed by BD Biosciences FACSCanto flow cytometer.

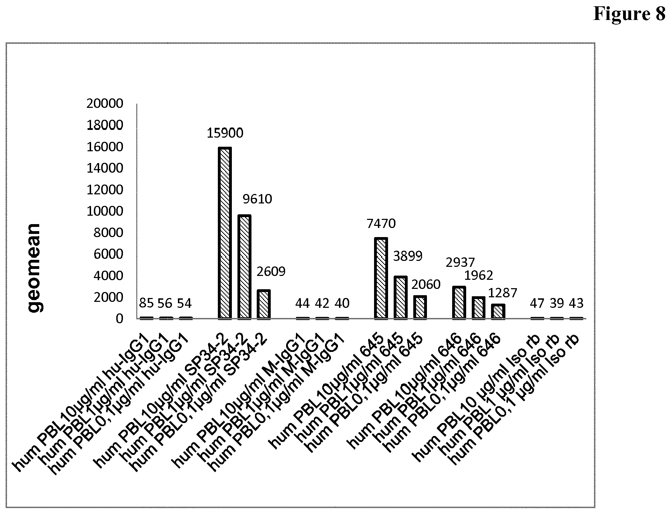

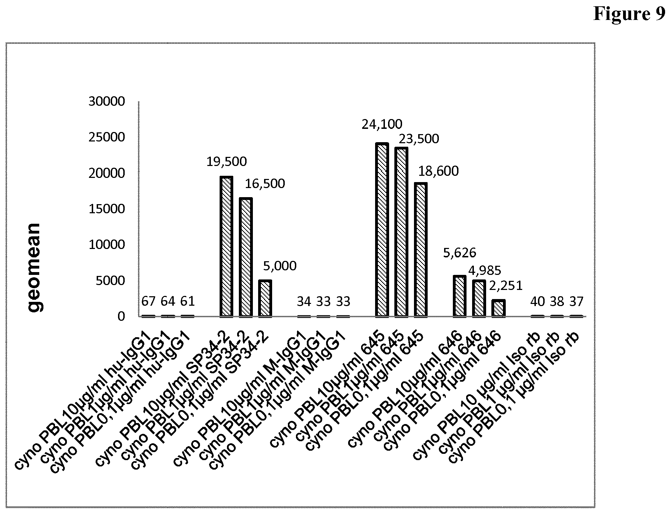

Results from the FACS based cellular binding studies show that clone 450, clone 627 and clone 645 bind to cynomolgus T cells comparable to anti-CD3 reference antibodies (SP34-2= CH2527 and H2C) whereas OKT3 and UCHT1 anti-CD3 reference antibodies do not bind to cynomolgus T cells (Figure 8 and Figure 9).

### Example 6

### Characterization of Binding Epitope

Binding of anti-CD3 antibody clone 645 to N- and C-terminally biotinylated human and cynomolgus CD3 peptides was determined by ELISA as described above (Example 5)

**Table 9: Binding of clone 645 to N- and C-terminally biotinylated human and cynomolgus CD3 peptides**

| **Antibody** | **huCD3 77-96 peptide C-terminal biotinylated** | **huCD3 77-96 peptide N-terminal biotinylated** | **cyCD3 69-88 peptide C-terminal biotinylated** | **cyCD3 69-88 peptide N-terminal biotinylated** |
|---|---|---|---|---|
| **Mab.645** | - | + | + | + |

| | | | | |
|---|---|---|---|---|
| Legend: - = no binding + = binding | | | | |

Results from binding ELISA show that clone 645 does not bind to human CD3 77-96 peptide that is immobilized on a streptavidin-coated microplate via C - terminally fused biotin. However, clone 645 binds the same peptide when immobilized N-terminally as well as both cynomolgus CD3 peptides 69-88 irrespective of the site of biotin fusion.

### Example 8

### Binding Affinity KD values of anti-CD3e antibody

Binding of the clone 645 antibody to human and cynomolgus CD3e was investigated by surface plasmon resonance using a BIACORE T100 instrument (GE Healthcare). Around 2000 resonance units (RU) of the capturing system (10 µg/ml goat anti rabbit IgG Fc Fragment specific; Order Code: 111-005-046; Jackson Immuno Research) were coupled on a CM4 chip (GE Healthcare, BR-1005-34) at pH 5.0 by using an amine coupling kit supplied by the GE Healthcare. Running buffer for Immobilization was HBS-N pH 7.4 (10 mM HEPES, 150 mM NaCl, pH 7.4, GE Healthcare, BR-1006-70). For the followed kinetic assay running and dilution buffer was HBS-P pH 7.4 (10 mM HEPES, 150 mM NaCl, 0.05% Surfactant P20, pH 7.4, GE Healthcare, BR-1006-71). The flow cell was set to 25 °C - and the sample block set to 12 °C - and primed with running buffer twice. The clone 645 antibody was captured by injecting a 1 µg/ml solution for 60 sec at a flow of 10 µl/min. Association was measured by injection of human CD3e(stalk)Fc-Knob-CD3d(stalk)FcHole or cynomolgus CD3e(stalk)Fc-Knob-CD3d(stalk)FcHole in various concentrations in solution for 180 sec at a flow of 30 µl/min starting with 1350 nM, followed by one 1:1.5 dilution and further in 1:3 dilutions. The dissociation phase was monitored for up to 300 sec and triggered by switching from the sample solution to running buffer. The surface was regenerated by washing with two consecutive injections of a Glycine pH 1.7 solution for 60 sec at a flow rate of 10 µl/min. Bulk refractive index differences were corrected by subtracting the response obtained from a goat anti rabbit IgG Fc surface. Blank injections are also subtracted (= double referencing). For calculation of KD and other kinetic parameters the Langmuir 1:1 model was used.

**Table 10: Kinetic affinities of clone 645 to CD3e**

| | kₐ (1/Ms) | k_{d} (1/s) | K_{D} (µM) |
|---|---|---|---|
| **human**CD3e(stalk)Fc-Knob-CD3d(stalk)FcHole | 1.7E+04 | 0.104 | **6.1** |
| **cynomolgus**CD3e(stalk)Fc-Knob-CD3d(stalk)FcHole | 2.1E+04 | 0.013 | **0.62** |

Surface plasmon resonance measurement of the clone 645 interacting with human and cynomolgus target, huCD3e(stalk)Fc-Knob-CD3d(stalk)FcHole and cyCD3e(stalk)Fc-Knob-CD3d(stalk)FcHole. The table shows values derived from single measurement (kₐ: association rate; k_{d}: dissociation rate; K_{D}: affinity).

## Claims

1. Use of a method comprising the step of immunizing a non-human experimental animal with a native cynomolgus antigen as sole antigen for producing a human cynomolgus cross-reactive antibody.

2. Use according to claim 1 wherein the native cynomolgus antigen lacks one or more (contiguous) amino acid stretches that are present in the corresponding human antigen, whereby one of the lacking (contiguous) amino acid stretches in the corresponding human antigen is the main immunogenic epitope of the human antigen.

3. Use according to claims 1 to 2 wherein the native cynomolgus antigen is CD3 epsilon and the human cynomolgus cross-reactive antibody specifically binds to (native) human CD3 epsilon of SEQ ID NO: 02 and specifically binds to a polypeptide of SEQ ID NO: 01.

4. Use according to claims 1 to 3 wherein the non-human experimental animal is immunized one or more times with primary cynomolgus PBLs, whereby the PBLs are optionally enriched for T cells.

5. Use according to claims 1 to 4 wherein the immunizing comprises as first step an intradermal application, as second step an intramuscular application and as third step a subcutaneous application.

6. Use according to claims 1 to 5 wherein the method is without using a denaturing agent.

7. Use according to claims 1 to 6 wherein the human cynomolgus cross-reactive antibody specifically binds to human and cynomolgus CD3 epsilon, to the polypeptide of SEQ ID NO: 01 and activates human T cells.

8. Use of a method comprising the step of immunizing a non-human experimental animal, three times with primary cynomolgus PBLs, whereby the PBLs are optionally enriched for T cells, without using primary human PBLs as immunogen and without using a denaturing agent for producing a human cynomolgus cross-reactive antibody specifically binding to human CD3 epsilon of SEQ ID NO: 02 and specifically binding to a polypeptide of SEQ ID NO: 01, wherein the human cynomolgus cross-reactive antibody specifically binds to human and cynomolgus T cells, activates human T cells and does not bind to the same epitope as antibody the OKT3, the antibody UCHT1 and/or the antibody SP34

9. Method for producing a human cynomolgus cross-reactive antibody comprising the step of immunizing a non-human experimental animal with a native cynomolgus antigen as sole antigen.

10. Method according to claim 9 wherein the native cynomolgus antigen lacks one or more (contiguous) amino acid stretches that are present in the corresponding human antigen, whereby one of the lacking (contiguous) amino acid stretches in the corresponding human antigen is the main immunogenic epitope of the human antigen.

11. Method according to claims 9 or 10 wherein the non-human experimental animal is immunized one or more times with primary cynomolgus PBLs, whereby the PBLs are optionally enriched for T cells.

12. Method according to claims 9 to 11 wherein the immunizing comprises as first step an intradermal application, as second step an intramuscular application and as third step a subcutaneous application.

13. Method for producing a human cynomolgus cross-reactive antibody specifically binding to human CD3 epsilon of SEQ ID NO: 02 and specifically binding to a polypeptide of SEQ ID NO: 01 comprising the step of immunizing a non-human experimental animal, three times with primary cynomolgus PBLs, whereby the PBLs are optionally enriched for T cells without using primary human PBLs as immunogen and without using a denaturing agent, wherein the human cynomolgus cross-reactive antibody specifically binds to human and cynomolgus T cells, activates human T cells and does not bind to the same epitope as the antibody OKT3, the antibody UCHT1 and/or the antibody SP34.

14. Human cynomolgus cross-reactive antibody specifically binding to human CD3 epsilon of SEQ ID NO: 02 and specifically binding to a polypeptide of SEQ ID NO: 01 wherein the human cynomolgus cross-reactive antibody specifically binds to human and cynomolgus T cells and activates human T cells.

15. Human cynomolgus cross-reactive antibody specifically binding to human CD3 epsilon of SEQ ID NO: 02 and specifically binding to a polypeptide of SEQ ID NO: 01 obtainable by immunizing a non-human experimental animal, three times with primary cynomolgus PBLs, whereby the PBLs are optionally enriched for T cells without using primary human PBLs as immunogen and without using a denaturing agent, wherein the human cynomolgus cross-reactive antibody specifically binds to human and cynomolgus T cells, activates human T cells and does not bind to the same epitope as the antibody OKT3, the antibody UCHT1 and/or antibody the SP34.


SEQUENCE LISTING

<110>  F. Hoffmann-La Roche AG

<120>   Antibodies binding to human and cynomolgus CD3 epsilon

<130>  P32142-WO

<150>  EP14170140
<151>  2014-05-28

<150>  EP14180572
<151>  2014-08-11

<160>  34

<170>  PatentIn version 3.5

<210>  1
<211>  20
<212>  PRT
<213>  Homo sapiens

<400>  1

Tyr Pro Arg Gly Ser Lys Pro Glu Asp Ala Asn Phe Tyr Leu Tyr Leu
1               5                   10                  15


Arg Ala Arg Val
            20



<210>  2
<211>  105
<212>  PRT
<213>  Homo sapiens

<400>  2

Gln Asp Gly Asn Glu Glu Met Gly Gly Ile Thr Gln Thr Pro Tyr Lys
1               5                   10                  15


Val Ser Ile Ser Gly Thr Thr Val Ile Leu Thr Cys Pro Gln Tyr Pro
            20                  25                  30


Gly Ser Glu Ile Leu Trp Gln His Asn Asp Lys Asn Ile Gly Gly Asp
        35                  40                  45


Glu Asp Asp Lys Asn Ile Gly Ser Asp Glu Asp His Leu Ser Leu Lys
    50                  55                  60


Glu Phe Ser Glu Leu Glu Gln Ser Gly Tyr Tyr Val Cys Tyr Pro Arg
65                  70                  75                  80


Gly Ser Lys Pro Glu Asp Ala Asn Phe Tyr Leu Tyr Leu Arg Ala Arg
                85                  90                  95

Val Cys Glu Asn Cys Met Glu Met Asp
            100                 105

<210>  3
<211>  207
<212>  PRT
<213>  Homo sapiens

<400>  3

Met Gln Ser Gly Thr His Trp Arg Val Leu Gly Leu Cys Leu Leu Ser
1               5                   10                  15

Val Gly Val Trp Gly Gln Asp Gly Asn Glu Glu Met Gly Gly Ile Thr
            20                  25                  30

Gln Thr Pro Tyr Lys Val Ser Ile Ser Gly Thr Thr Val Ile Leu Thr
        35                  40                  45

Cys Pro Gln Tyr Pro Gly Ser Glu Ile Leu Trp Gln His Asn Asp Lys
    50                  55                  60

Asn Ile Gly Gly Asp Glu Asp Asp Lys Asn Ile Gly Ser Asp Glu Asp
65                  70                  75                  80

His Leu Ser Leu Lys Glu Phe Ser Glu Leu Glu Gln Ser Gly Tyr Tyr
                85                  90                  95

Val Cys Tyr Pro Arg Gly Ser Lys Pro Glu Asp Ala Asn Phe Tyr Leu
            100                 105                 110

Tyr Leu Arg Ala Arg Val Cys Glu Asn Cys Met Glu Met Asp Val Met
        115                 120                 125

Ser Val Ala Thr Ile Val Ile Val Asp Ile Cys Ile Thr Gly Gly Leu
    130                 135                 140

Leu Leu Leu Val Tyr Tyr Trp Ser Lys Asn Arg Lys Ala Lys Ala Lys
145                 150                 155                 160

Pro Val Thr Arg Gly Ala Gly Ala Gly Gly Arg Gln Arg Gly Gln Asn
                165                 170                 175

Lys Glu Arg Pro Pro Pro Val Pro Asn Pro Asp Tyr Glu Pro Ile Arg
            180                 185                 190

Lys Gly Gln Arg Asp Leu Tyr Ser Gly Leu Asn Gln Arg Arg Ile
        195                 200                 205

<210>  4
<211>  9
<212>  PRT
<213>  Artificial Sequence

<220>
<223>  645 HVR-H1-1

<400>  4

Ser Phe Ser Gly Thr Tyr Trp Ile Cys
1               5


<210>  5
<211>  5
<212>  PRT
<213>  Artificial Sequence

<220>
<223>  645 HVR-H1-2

<400>  5

Gly Thr Tyr Trp Ile
1               5


<210>  6
<211>  15
<212>  PRT
<213>  Artificial Sequence

<220>
<223>  645HVR-H2-1

<400>  6

Cys Ile Ser Thr Asp Ser Gly Ser Thr Tyr Tyr Ala Ser Trp Ala
1               5                   10                  15


<210>  7
<211>  11
<212>  PRT
<213>  Artificial Sequence

<220>
<223>  645HVR-H2-2

<400>  7

Thr Asp Ser Gly Ser Thr Tyr Tyr Ala Ser Trp
1               5                   10


<210>  8
<211>  3
<212>  PRT
<213>  Artificial Sequence

<220>

<223>  645HVR-H2-3

<400>  8

Thr Asp Ser
1



<210>  9
<211>  10
<212>  PRT
<213>  Artificial Sequence

<220>
<223>  645HVR-H3

<400>  9

Ser Ala Tyr Ala Ser Glu Gly Tyr Ala Leu
1               5                   10



<210>  10
<211>  13
<212>  PRT
<213>  Artificial Sequence

<220>
<223>  645HVR-L1-1

<400>  10

Gln Ala Ser Glu Ser Val Tyr Asn Asn Asn Tyr Leu Ser
1               5                   10



<210>  11
<211>  10
<212>  PRT
<213>  Artificial Sequence

<220>
<223>  645HVR-L1-2

<400>  11

Gln Ala Ser Glu Ser Val Tyr Asn Asn Asn
1               5                   10



<210>  12
<211>  6
<212>  PRT
<213>  Artificial Sequence

<220>
<223>  645HVR-L2

<400>  12

Tyr Ala Ser Thr Leu Ala
1               5

<210>  13
<211>  13
<212>  PRT
<213>  Artificial Sequence

<220>
<223>  645HVR-L3

<400>  13

Thr Gly Trp Lys Ser Ile Thr Thr Asp Gly Met Gly Ala
1               5                   10


<210>  14
<211>  159
<212>  PRT
<213>  Artificial Sequence

<220>
<223>  645 VH

<400>  14

Met Glu Thr Gly Leu Arg Trp Leu Leu Leu Val Ala Val Leu Lys Gly
1               5                   10                  15

Val Gln Cys Gln Ser Leu Glu Glu Ser Gly Gly Asp Leu Val Lys Pro
            20                  25                  30

Gly Ala Ser Leu Thr Leu Thr Cys Leu Ala Ser Gly Phe Ser Phe Ser
        35                  40                  45

Gly Thr Tyr Trp Ile Cys Trp Val Arg Gln Ala Pro Gly Lys Gly Leu
    50                  55                  60

Glu Trp Ile Gly Cys Ile Ser Thr Asp Ser Gly Ser Thr Tyr Tyr Ala
65                  70                  75                  80

Ser Trp Ala Lys Gly Arg Phe Thr Ile Ser Lys Thr Ser Ser Thr Thr
                85                  90                  95

Val Thr Leu Gln Met Thr Ser Leu Thr Ala Ala Asp Thr Ala Thr Asn
            100                 105                 110

Phe Cys Ala Arg Ser Ala Tyr Ala Ser Glu Gly Tyr Ala Leu Trp Gly
        115                 120                 125

Pro Gly Thr Leu Val Thr Val Ser Ser Gly Gln Pro Lys Ala Pro Ser
    130                 135                 140

Val Phe Pro Leu Ala Pro Cys Cys Gly Asp Thr Pro Ser Ser Thr
145                 150                 155

<210>  15
<211>  166
<212>  PRT
<213>  Artificial Sequence

<220>
<223>  645 VL


<220>
<221>  misc_feature
<222>  (3)..(3)
<223>  Xaa can be any naturally occurring amino acid

<400>  15

Met Asp Xaa Arg Ala Pro Thr Gln Leu Leu Gly Leu Leu Leu Leu Trp
1               5                   10                  15

Leu Pro Gly Ala Thr Phe Ala Ile Val Met Thr Gln Thr Pro Ser Ser
            20                  25                  30

Lys Ser Val Pro Val Gly Asp Thr Val Thr Ile Asn Cys Gln Ala Ser
        35                  40                  45

Glu Ser Val Tyr Asn Asn Asn Tyr Leu Ser Trp Tyr Gln Gln Lys Pro
    50                  55                  60

Gly Gln Pro Pro Lys Leu Leu Ile Tyr Tyr Ala Ser Thr Leu Ala Ser
65                  70                  75                  80

Gly Val Ser Ser Arg Phe Lys Gly Ser Gly Ser Gly Thr Gln Phe Thr
                85                  90                  95

Leu Thr Ile Ser Asp Val Val Cys Asp Asp Ala Ala Thr Tyr Tyr Cys
            100                 105                 110

Thr Gly Trp Lys Ser Ile Thr Thr Asp Gly Met Gly Ala Phe Gly Gly
        115                 120                 125

Gly Thr Glu Val Val Val Lys Gly Asp Pro Val Ala Pro Thr Val Leu
    130                 135                 140

Ile Phe Pro Pro Ala Ala Asp Gln Val Ala Thr Gly Thr Val Thr Ile
145                 150                 155                 160

Val Cys Val Ala Asn Lys
                165


<210>  16

<211>  461
<212>  PRT
<213>  Artificial Sequence

<220>
<223>  human CD3g(G4S)5CD3e-AcTev-Fc(knob)-Avi

<400>  16

Gln Ser Ile Lys Gly Asn His Leu Val Lys Val Tyr Asp Tyr Gln Glu
1               5                   10                  15

Asp Gly Ser Val Leu Leu Thr Cys Asp Ala Glu Ala Lys Asn Ile Thr
            20                  25                  30

Trp Phe Lys Asp Gly Lys Met Ile Gly Phe Leu Thr Glu Asp Lys Lys
        35                  40                  45

Lys Trp Asn Leu Gly Ser Asn Ala Lys Asp Pro Arg Gly Met Tyr Gln
    50                  55                  60

Cys Lys Gly Ser Gln Asn Lys Ser Lys Pro Leu Gln Val Tyr Tyr Arg
65                  70                  75                  80

Met Gly Gly Gly Gly Ser Gly Gly Gly Gly Ser Gly Gly Gly Gly Ser
                85                  90                  95

Gly Gly Gly Gly Ser Gly Gly Gly Gly Ser Asp Gly Asn Glu Glu Met
            100                 105                 110

Gly Gly Ile Thr Gln Thr Pro Tyr Lys Val Ser Ile Ser Gly Thr Thr
        115                 120                 125

Val Ile Leu Thr Cys Pro Gln Tyr Pro Gly Ser Glu Ile Leu Trp Gln
    130                 135                 140

His Asn Asp Lys Asn Ile Gly Gly Asp Glu Asp Asp Lys Asn Ile Gly
145                 150                 155                 160

Ser Asp Glu Asp His Leu Ser Leu Lys Glu Phe Ser Glu Leu Glu Gln
                165                 170                 175

Ser Gly Tyr Tyr Val Cys Tyr Pro Arg Gly Ser Lys Pro Glu Asp Ala
            180                 185                 190

Asn Phe Tyr Leu Tyr Leu Arg Ala Arg Val Gly Ser Glu Gln Leu Tyr
        195                 200                 205

Phe Gln Gly Gly Ser Pro Lys Ser Ala Asp Lys Thr His Thr Cys Pro
    210                 215                 220

Pro Cys Pro Ala Pro Glu Leu Leu Gly Gly Pro Ser Val Phe Leu Phe
225                 230                 235                 240

Pro Pro Lys Pro Lys Asp Thr Leu Met Ile Ser Arg Thr Pro Glu Val
                245                 250                 255

Thr Cys Val Val Val Asp Val Ser His Glu Asp Pro Glu Val Lys Phe
            260                 265                 270

Asn Trp Tyr Val Asp Gly Val Glu Val His Asn Ala Lys Thr Lys Pro
        275                 280                 285

Arg Glu Glu Gln Tyr Asn Ser Thr Tyr Arg Val Val Ser Val Leu Thr
    290                 295                 300

Val Leu His Gln Asp Trp Leu Asn Gly Lys Glu Tyr Lys Cys Lys Val
305                 310                 315                 320

Ser Asn Lys Ala Leu Pro Ala Pro Ile Glu Lys Thr Ile Ser Lys Ala
                325                 330                 335

Lys Gly Gln Pro Arg Glu Pro Gln Val Tyr Thr Leu Pro Pro Cys Arg
            340                 345                 350

Asp Glu Leu Thr Lys Asn Gln Val Ser Leu Trp Cys Leu Val Lys Gly
        355                 360                 365

Phe Tyr Pro Ser Asp Ile Ala Val Glu Trp Glu Ser Asn Gly Gln Pro
    370                 375                 380

Glu Asn Asn Tyr Lys Thr Thr Pro Pro Val Leu Asp Ser Asp Gly Ser
385                 390                 395                 400

Phe Phe Leu Tyr Ser Lys Leu Thr Val Asp Lys Ser Arg Trp Gln Gln
                405                 410                 415

Gly Asn Val Phe Ser Cys Ser Val Met His Glu Ala Leu His Asn His
            420                 425                 430

Tyr Thr Gln Lys Ser Leu Ser Leu Ser Pro Gly Lys Ser Gly Gly Leu
        435                 440                 445

Asn Asp Ile Phe Glu Ala Gln Lys Ile Glu Trp His Glu
    450                 455                 460

<210>  17

<211>  227
<212>  PRT
<213>  Artificial Sequence

<220>
<223>  Fc(hole)

<400>  17

Asp Lys Thr His Thr Cys Pro Pro Cys Pro Ala Pro Glu Leu Leu Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Pro Ile
            100                 105                 110

Glu Lys Thr Ile Ser Lys Ala Lys Gly Gln Pro Arg Glu Pro Gln Val
        115                 120                 125

Cys Thr Leu Pro Pro Ser Arg Asp Glu Leu Thr Lys Asn Gln Val Ser
    130                 135                 140

Leu Ser Cys Ala Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Val Ser Lys Leu Thr Val
            180                 185                 190

Asp Lys Ser Arg Trp Gln Gln Gly Asn Val Phe Ser Cys Ser Val Met
        195                 200                 205

His Glu Ala Leu His Asn His Tyr Thr Gln Lys Ser Leu Ser Leu Ser
    210                 215                 220

Pro Gly Lys
225

<210> 18
<211> 452
<212> PRT
<213> Artificial Sequence

<220>
<223> cynomolgus CD3g(G4S)5CD3e-AcTev-Fc(knob)-Avi

<400> 18

Gln Ser Phe Glu Glu Asn Arg Lys Leu Asn Val Tyr Asn Gln Glu Asp
1               5                   10                  15

Gly Ser Val Leu Leu Thr Cys His Val Lys Asn Thr Asn Ile Thr Trp
            20                  25                  30

Phe Lys Glu Gly Lys Met Ile Asp Ile Leu Thr Ala His Lys Asn Lys
        35                  40                  45

Trp Asn Leu Gly Ser Asn Thr Lys Asp Pro Arg Gly Val Tyr Gln Cys
    50                  55                  60

Lys Gly Ser Lys Asp Lys Ser Lys Thr Leu Gln Val Tyr Tyr Arg Met
65                  70                  75                  80

Gly Gly Gly Gly Ser Gly Gly Gly Gly Ser Gly Gly Gly Gly Ser Gly
                85                  90                  95

Gly Gly Gly Ser Gly Gly Gly Gly Ser Gln Asp Gly Asn Glu Glu Met
            100                 105                 110

Gly Ser Ile Thr Gln Thr Pro Tyr Gln Val Ser Ile Ser Gly Thr Thr
        115                 120                 125

Val Ile Leu Thr Cys Ser Gln His Leu Gly Ser Glu Ala Gln Trp Gln
    130                 135                 140

His Asn Gly Lys Asn Lys Glu Asp Ser Gly Asp Arg Leu Phe Leu Pro
145                 150                 155                 160

Glu Phe Ser Glu Met Glu Gln Ser Gly Tyr Tyr Val Cys Tyr Pro Arg
                165                 170                 175

Gly Ser Asn Pro Glu Asp Ala Ser His His Leu Tyr Leu Lys Ala Arg
            180                 185                 190

Val Gly Ser Glu Gln Leu Tyr Phe Gln Gly Gly Ser Pro Lys Ser Ala
        195                 200                 205

Asp Lys Thr His Thr Cys Pro Pro Cys Pro Ala Pro Glu Leu Leu Gly
    210                 215                 220

Gly Pro Ser Val Phe Leu Phe Pro Pro Lys Pro Lys Asp Thr Leu Met
225                 230                 235                 240

Ile Ser Arg Thr Pro Glu Val Thr Cys Val Val Val Asp Val Ser His
                245                 250                 255

Glu Asp Pro Glu Val Lys Phe Asn Trp Tyr Val Asp Gly Val Glu Val
            260                 265                 270

His Asn Ala Lys Thr Lys Pro Arg Glu Glu Gln Tyr Asn Ser Thr Tyr
        275                 280                 285

Arg Val Val Ser Val Leu Thr Val Leu His Gln Asp Trp Leu Asn Gly
    290                 295                 300

Lys Glu Tyr Lys Cys Lys Val Ser Asn Lys Ala Leu Pro Ala Pro Ile
305                 310                 315                 320

Glu Lys Thr Ile Ser Lys Ala Lys Gly Gln Pro Arg Glu Pro Gln Val
                325                 330                 335

Tyr Thr Leu Pro Pro Cys Arg Asp Glu Leu Thr Lys Asn Gln Val Ser
            340                 345                 350

Leu Trp Cys Leu Val Lys Gly Phe Tyr Pro Ser Asp Ile Ala Val Glu
        355                 360                 365

Trp Glu Ser Asn Gly Gln Pro Glu Asn Asn Tyr Lys Thr Thr Pro Pro
    370                 375                 380

Val Leu Asp Ser Asp Gly Ser Phe Phe Leu Tyr Ser Lys Leu Thr Val
385                 390                 395                 400

Asp Lys Ser Arg Trp Gln Gln Gly Asn Val Phe Ser Cys Ser Val Met
                405                 410                 415

His Glu Ala Leu His Asn His Tyr Thr Gln Lys Ser Leu Ser Leu Ser
            420                 425                 430

Pro Gly Lys Ser Gly Gly Leu Asn Asp Ile Phe Glu Ala Gln Lys Ile
        435                 440                 445

Glu Trp His Glu
    450

<210>  19
<211>  360
<212>  PRT
<213>  Artificial Sequence

<220>
<223>  human CD3e-stalk-Fc(knob)-Avi

<400>  19

<210>  20
<211>  325
<212>  PRT
<213>  Artificial Sequence

<220>
<223>  human CD3d-stalk-Fc(hole)-Avi

<400>  20

Phe Lys Ile Pro Ile Glu Glu Leu Glu Asp Arg Val Phe Val Asn Cys
1               5                   10                  15

Asn Thr Ser Ile Thr Trp Val Glu Gly Thr Val Gly Thr Leu Leu Ser
            20                  25                  30

Asp Ile Thr Arg Leu Asp Leu Gly Lys Arg Ile Leu Asp Pro Arg Gly
        35                  40                  45

Ile Tyr Arg Cys Asn Gly Thr Asp Ile Tyr Lys Asp Lys Glu Ser Thr
    50                  55                  60

Val Gln Val His Tyr Arg Met Cys Arg Ser Glu Gln Leu Tyr Phe Gln
65                  70                  75                  80

Gly Asp Lys Thr His Thr Cys Pro Pro Cys Pro Ala Pro Glu Leu Leu
                85                  90                  95

Gly Gly Pro Ser Val Phe Leu Phe Pro Pro Lys Pro Lys Asp Thr Leu
            100                 105                 110

Met Ile Ser Arg Thr Pro Glu Val Thr Cys Val Val Val Asp Val Ser
        115                 120                 125

His Glu Asp Pro Glu Val Lys Phe Asn Trp Tyr Val Asp Gly Val Glu
    130                 135                 140

Val His Asn Ala Lys Thr Lys Pro Arg Glu Glu Gln Tyr Asn Ser Thr
145                 150                 155                 160

Tyr Arg Val Val Ser Val Leu Thr Val Leu His Gln Asp Trp Leu Asn
                165                 170                 175

Gly Lys Glu Tyr Lys Cys Lys Val Ser Asn Lys Ala Leu Pro Ala Pro
            180                 185                 190

Ile Glu Lys Thr Ile Ser Lys Ala Lys Gly Gln Pro Arg Glu Pro Gln
        195                 200                 205

Val Cys Thr Leu Pro Pro Ser Arg Asp Glu Leu Thr Lys Asn Gln Val
    210                 215                 220

Ser Leu Ser Cys Ala Val Lys Gly Phe Tyr Pro Ser Asp Ile Ala Val
225                 230                 235                 240

Glu Trp Glu Ser Asn Gly Gln Pro Glu Asn Asn Tyr Lys Thr Thr Pro
                245                 250                 255

Pro Val Leu Asp Ser Asp Gly Ser Phe Phe Leu Val Ser Lys Leu Thr
            260                 265                 270

Val Asp Lys Ser Arg Trp Gln Gln Gly Asn Val Phe Ser Cys Ser Val
        275                 280                 285

Met His Glu Ala Leu His Asn His Tyr Thr Gln Lys Ser Leu Ser Leu
    290                 295                 300

Ser Pro Gly Lys Ser Gly Gly Leu Asn Asp Ile Phe Glu Ala Gln Lys
305                 310                 315                 320

Ile Glu Trp His Glu
                325

<210>  21
<211>  337
<212>  PRT
<213>  Artificial Sequence

<220>
<223>  human CD3gC85-stalk-Fc(hole)-Avi

<400>  21

Gln Ser Ile Lys Gly Asn His Leu Val Lys Val Tyr Asp Tyr Gln Glu
1               5                   10                  15

Asp Gly Ser Val Leu Leu Thr Cys Asp Ala Glu Ala Lys Asn Ile Thr
            20                  25                  30

Trp Phe Lys Asp Gly Lys Met Ile Gly Phe Leu Thr Glu Asp Lys Lys
        35                  40                  45

Lys Trp Asn Leu Gly Ser Asn Ala Lys Asp Pro Arg Gly Met Tyr Gln
    50                  55                  60

Cys Lys Gly Ser Gln Asn Lys Ser Lys Pro Leu Gln Val Tyr Tyr Arg
65                  70                  75                  80

Met Cys Gln Asn Gly Ser Glu Gln Leu Tyr Phe Gln Gly Asp Lys Thr
                85                  90                  95

His Thr Cys Pro Pro Cys Pro Ala Pro Glu Leu Leu Gly Gly Pro Ser
            100                 105                 110

Val Phe Leu Phe Pro Pro Lys Pro Lys Asp Thr Leu Met Ile Ser Arg
        115                 120                 125

Thr Pro Glu Val Thr Cys Val Val Val Asp Val Ser His Glu Asp Pro
    130                 135                 140

Glu Val Lys Phe Asn Trp Tyr Val Asp Gly Val Glu Val His Asn Ala
145                 150                 155                 160

Lys Thr Lys Pro Arg Glu Glu Gln Tyr Asn Ser Thr Tyr Arg Val Val
                165                 170                 175

Ser Val Leu Thr Val Leu His Gln Asp Trp Leu Asn Gly Lys Glu Tyr
            180                 185                 190

Lys Cys Lys Val Ser Asn Lys Ala Leu Pro Ala Pro Ile Glu Lys Thr
        195                 200                 205

Ile Ser Lys Ala Lys Gly Gln Pro Arg Glu Pro Gln Val Cys Thr Leu
    210                 215                 220

Pro Pro Ser Arg Asp Glu Leu Thr Lys Asn Gln Val Ser Leu Ser Cys
225                 230                 235                 240

Ala Val Lys Gly Phe Tyr Pro Ser Asp Ile Ala Val Glu Trp Glu Ser
                245                 250                 255

Asn Gly Gln Pro Glu Asn Asn Tyr Lys Thr Thr Pro Pro Val Leu Asp
            260                 265                 270

Ser Asp Gly Ser Phe Phe Leu Val Ser Lys Leu Thr Val Asp Lys Ser
        275                 280                 285

Arg Trp Gln Gln Gly Asn Val Phe Ser Cys Ser Val Met His Glu Ala
    290                 295                 300

Leu His Asn His Tyr Thr Gln Lys Ser Leu Ser Leu Ser Pro Gly Lys
305                 310                 315                 320

Ser Gly Gly Leu Asn Asp Ile Phe Glu Ala Gln Lys Ile Glu Trp His
                325                 330                 335

Glu

<210>  22
<211>  351
<212>  PRT
<213>  Artificial Sequence

<220>
<223>  cynomolgus CD3e stalk-Fc(knob)-Avi

<400>  22

Gln Asp Gly Asn Glu Glu Met Gly Ser Ile Thr Gln Thr Pro Tyr Gln
1               5                   10                  15

Val Ser Ile Ser Gly Thr Thr Val Ile Leu Thr Cys Ser Gln His Leu

20                  25                  30

Gly Ser Glu Ala Gln Trp Gln His Asn Gly Lys Asn Lys Glu Asp Ser
        35                  40                  45

Gly Asp Arg Leu Phe Leu Pro Glu Phe Ser Glu Met Glu Gln Ser Gly
    50                  55                  60

Tyr Tyr Val Cys Tyr Pro Arg Gly Ser Asn Pro Glu Asp Ala Ser His
65                  70                  75                  80

His Leu Tyr Leu Lys Ala Arg Val Ser Glu Asn Cys Val Asp Glu Gln
                85                  90                  95

Leu Tyr Phe Gln Gly Gly Ser Pro Lys Ser Ala Asp Lys Thr His Thr
            100                 105                 110

Cys Pro Pro Cys Pro Ala Pro Glu Leu Leu Gly Gly Pro Ser Val Phe
        115                 120                 125

Leu Phe Pro Pro Lys Pro Lys Asp Thr Leu Met Ile Ser Arg Thr Pro
    130                 135                 140

Glu Val Thr Cys Val Val Val Asp Val Ser His Glu Asp Pro Glu Val
145                 150                 155                 160

Lys Phe Asn Trp Tyr Val Asp Gly Val Glu Val His Asn Ala Lys Thr
                165                 170                 175

Lys Pro Arg Glu Glu Gln Tyr Asn Ser Thr Tyr Arg Val Val Ser Val
            180                 185                 190

Leu Thr Val Leu His Gln Asp Trp Leu Asn Gly Lys Glu Tyr Lys Cys
        195                 200                 205

Lys Val Ser Asn Lys Ala Leu Pro Ala Pro Ile Glu Lys Thr Ile Ser
    210                 215                 220

Lys Ala Lys Gly Gln Pro Arg Glu Pro Gln Val Tyr Thr Leu Pro Pro
225                 230                 235                 240

Cys Arg Asp Glu Leu Thr Lys Asn Gln Val Ser Leu Trp Cys Leu Val
                245                 250                 255

Lys Gly Phe Tyr Pro Ser Asp Ile Ala Val Glu Trp Glu Ser Asn Gly
            260                 265                 270

Gln Pro Glu Asn Asn Tyr Lys Thr Thr Pro Pro Val Leu Asp Ser Asp
        275                 280                 285

Gly Ser Phe Phe Leu Tyr Ser Lys Leu Thr Val Asp Lys Ser Arg Trp
    290                 295                 300

Gln Gln Gly Asn Val Phe Ser Cys Ser Val Met His Glu Ala Leu His
305                 310                 315                 320

Asn His Tyr Thr Gln Lys Ser Leu Ser Leu Ser Pro Gly Lys Ser Gly
                325                 330                 335

Gly Leu Asn Asp Ile Phe Glu Ala Gln Lys Ile Glu Trp His Glu
            340                 345                 350

<210>  23
<211>  334
<212>  PRT
<213>  Artificial Sequence

<220>
<223>  cynomolgus CD3d-stalk-Fc(hole)-Avi

<400>  23

Phe Lys Ile Pro Val Glu Glu Leu Glu Asp Arg Val Phe Val Lys Cys
1               5                   10                  15

Asn Thr Ser Val Thr Trp Val Glu Gly Thr Val Gly Thr Leu Leu Thr
            20                  25                  30

Asn Asn Thr Arg Leu Asp Leu Gly Lys Arg Ile Leu Asp Pro Arg Gly
        35                  40                  45

Ile Tyr Arg Cys Asn Gly Thr Asp Ile Tyr Lys Asp Lys Glu Ser Ala
    50                  55                  60

Val Gln Val His Tyr Arg Met Ser Gln Asn Cys Val Asp Glu Gln Leu
65                  70                  75                  80

Tyr Phe Gln Gly Gly Ser Pro Lys Ser Ala Asp Lys Thr His Thr Cys
                85                  90                  95

Pro Pro Cys Pro Ala Pro Glu Leu Leu Gly Gly Pro Ser Val Phe Leu
            100                 105                 110

Phe Pro Pro Lys Pro Lys Asp Thr Leu Met Ile Ser Arg Thr Pro Glu
        115                 120                 125

Val Thr Cys Val Val Val Asp Val Ser His Glu Asp Pro Glu Val Lys

130                 135                 140

Phe Asn Trp Tyr Val Asp Gly Val Glu Val His Asn Ala Lys Thr Lys
145                 150                 155                 160

Pro Arg Glu Glu Gln Tyr Asn Ser Thr Tyr Arg Val Val Ser Val Leu
                165                 170                 175

Thr Val Leu His Gln Asp Trp Leu Asn Gly Lys Glu Tyr Lys Cys Lys
            180                 185                 190

Val Ser Asn Lys Ala Leu Pro Ala Pro Ile Glu Lys Thr Ile Ser Lys
        195                 200                 205

Ala Lys Gly Gln Pro Arg Glu Pro Gln Val Cys Thr Leu Pro Pro Ser
    210                 215                 220

Arg Asp Glu Leu Thr Lys Asn Gln Val Ser Leu Ser Cys Ala Val Lys
225                 230                 235                 240

Gly Phe Tyr Pro Ser Asp Ile Ala Val Glu Trp Glu Ser Asn Gly Gln
                245                 250                 255

Pro Glu Asn Asn Tyr Lys Thr Thr Pro Pro Val Leu Asp Ser Asp Gly
            260                 265                 270

Ser Phe Phe Leu Val Ser Lys Leu Thr Val Asp Lys Ser Arg Trp Gln
        275                 280                 285

Gln Gly Asn Val Phe Ser Cys Ser Val Met His Glu Ala Leu His Asn
    290                 295                 300

His Tyr Thr Gln Lys Ser Leu Ser Leu Ser Pro Gly Lys Ser Gly Gly
305                 310                 315                 320

Leu Asn Asp Ile Phe Glu Ala Gln Lys Ile Glu Trp His Glu
                325                 330

<210>  24
<211>  286
<212>  PRT
<213>  Artificial Sequence

<220>
<223>  human CD3e 1-27 - Fc(knob)Avi

<400>  24

Gln Asp Gly Asn Glu Glu Met Gly Gly Ile Thr Gln Thr Pro Tyr Lys
1               5                   10                  15

Val Ser Ile Ser Gly Thr Thr Val Ile Leu Thr Gly Ser Glu Gln Leu
            20                  25                  30

Tyr Phe Gln Gly Gly Ser Pro Lys Ser Ala Asp Lys Thr His Thr Cys
        35                  40                  45

Pro Pro Cys Pro Ala Pro Glu Leu Leu Gly Gly Pro Ser Val Phe Leu
    50                  55                  60

Phe Pro Pro Lys Pro Lys Asp Thr Leu Met Ile Ser Arg Thr Pro Glu
65                  70                  75                  80

Val Thr Cys Val Val Val Asp Val Ser His Glu Asp Pro Glu Val Lys
                85                  90                  95

Phe Asn Trp Tyr Val Asp Gly Val Glu Val His Asn Ala Lys Thr Lys
            100                 105                 110

Pro Arg Glu Glu Gln Tyr Asn Ser Thr Tyr Arg Val Val Ser Val Leu
        115                 120                 125

Thr Val Leu His Gln Asp Trp Leu Asn Gly Lys Glu Tyr Lys Cys Lys
    130                 135                 140

Val Ser Asn Lys Ala Leu Pro Ala Pro Ile Glu Lys Thr Ile Ser Lys
145                 150                 155                 160

Ala Lys Gly Gln Pro Arg Glu Pro Gln Val Tyr Thr Leu Pro Pro Cys
                165                 170                 175

Arg Asp Glu Leu Thr Lys Asn Gln Val Ser Leu Trp Cys Leu Val Lys
            180                 185                 190

Gly Phe Tyr Pro Ser Asp Ile Ala Val Glu Trp Glu Ser Asn Gly Gln
        195                 200                 205

Pro Glu Asn Asn Tyr Lys Thr Thr Pro Pro Val Leu Asp Ser Asp Gly
    210                 215                 220

Ser Phe Phe Leu Tyr Ser Lys Leu Thr Val Asp Lys Ser Arg Trp Gln
225                 230                 235                 240

Gln Gly Asn Val Phe Ser Cys Ser Val Met His Glu Ala Leu His Asn
                245                 250                 255

His Tyr Thr Gln Lys Ser Leu Ser Leu Ser Pro Gly Lys Ser Gly Gly

260                 265                 270

Leu Asn Asp Ile Phe Glu Ala Gln Lys Ile Glu Trp His Glu
        275                 280                 285

<210>  25
<211>  281
<212>  PRT
<213>  Artificial Sequence

<220>
<223>  cynomolgus CD3e 6-27 - Fc(knob)Avi

<400>  25

Glu Met Gly Ser Ile Thr Gln Thr Pro Tyr Gln Val Ser Ile Ser Gly
1               5                   10                  15

Thr Thr Val Ile Leu Thr Gly Ser Glu Gln Leu Tyr Phe Gln Gly Gly
            20                  25                  30

Ser Pro Lys Ser Ala Asp Lys Thr His Thr Cys Pro Pro Cys Pro Ala
        35                  40                  45

Pro Glu Leu Leu Gly Gly Pro Ser Val Phe Leu Phe Pro Pro Lys Pro
    50                  55                  60

Lys Asp Thr Leu Met Ile Ser Arg Thr Pro Glu Val Thr Cys Val Val
65                  70                  75                  80

Val Asp Val Ser His Glu Asp Pro Glu Val Lys Phe Asn Trp Tyr Val
                85                  90                  95

Asp Gly Val Glu Val His Asn Ala Lys Thr Lys Pro Arg Glu Glu Gln
            100                 105                 110

Tyr Asn Ser Thr Tyr Arg Val Val Ser Val Leu Thr Val Leu His Gln
        115                 120                 125

Asp Trp Leu Asn Gly Lys Glu Tyr Lys Cys Lys Val Ser Asn Lys Ala
    130                 135                 140

Leu Pro Ala Pro Ile Glu Lys Thr Ile Ser Lys Ala Lys Gly Gln Pro
145                 150                 155                 160

Arg Glu Pro Gln Val Tyr Thr Leu Pro Pro Cys Arg Asp Glu Leu Thr
                165                 170                 175

Lys Asn Gln Val Ser Leu Trp Cys Leu Val Lys Gly Phe Tyr Pro Ser
            180                 185                 190

Asp Ile Ala Val Glu Trp Glu Ser Asn Gly Gln Pro Glu Asn Asn Tyr
        195                 200                 205

Lys Thr Thr Pro Pro Val Leu Asp Ser Asp Gly Ser Phe Phe Leu Tyr
    210                 215                 220

Ser Lys Leu Thr Val Asp Lys Ser Arg Trp Gln Gln Gly Asn Val Phe
225                 230                 235                 240

Ser Cys Ser Val Met His Glu Ala Leu His Asn His Tyr Thr Gln Lys
                245                 250                 255

Ser Leu Ser Leu Ser Pro Gly Lys Ser Gly Gly Leu Asn Asp Ile Phe
            260                 265                 270

Glu Ala Gln Lys Ile Glu Trp His Glu
        275                 280

<210> 26
<211> 281
<212> PRT
<213> Artificial Sequence

<220>
<223> human CD3e 6-27 - Fc(knob)Avi

<400> 26

Glu Met Gly Gly Ile Thr Gln Thr Pro Tyr Lys Val Ser Ile Ser Gly
1               5                   10                  15

Thr Thr Val Ile Leu Thr Gly Ser Glu Gln Leu Tyr Phe Gln Gly Gly
            20                  25                  30

Ser Pro Lys Ser Ala Asp Lys Thr His Thr Cys Pro Pro Cys Pro Ala
        35                  40                  45

Pro Glu Leu Leu Gly Gly Pro Ser Val Phe Leu Phe Pro Pro Lys Pro
    50                  55                  60

Lys Asp Thr Leu Met Ile Ser Arg Thr Pro Glu Val Thr Cys Val Val
65                  70                  75                  80

Val Asp Val Ser His Glu Asp Pro Glu Val Lys Phe Asn Trp Tyr Val
                85                  90                  95

Asp Gly Val Glu Val His Asn Ala Lys Thr Lys Pro Arg Glu Glu Gln
            100                 105                 110

Tyr Asn Ser Thr Tyr Arg Val Val Ser Val Leu Thr Val Leu His Gln
        115                 120                 125

Asp Trp Leu Asn Gly Lys Glu Tyr Lys Cys Lys Val Ser Asn Lys Ala
    130                 135                 140

Leu Pro Ala Pro Ile Glu Lys Thr Ile Ser Lys Ala Lys Gly Gln Pro
145                 150                 155                 160

Arg Glu Pro Gln Val Tyr Thr Leu Pro Pro Cys Arg Asp Glu Leu Thr
                165                 170                 175

Lys Asn Gln Val Ser Leu Trp Cys Leu Val Lys Gly Phe Tyr Pro Ser
            180                 185                 190

Asp Ile Ala Val Glu Trp Glu Ser Asn Gly Gln Pro Glu Asn Asn Tyr
        195                 200                 205

Lys Thr Thr Pro Pro Val Leu Asp Ser Asp Gly Ser Phe Phe Leu Tyr
    210                 215                 220

Ser Lys Leu Thr Val Asp Lys Ser Arg Trp Gln Gln Gly Asn Val Phe
225                 230                 235                 240

Ser Cys Ser Val Met His Glu Ala Leu His Asn His Tyr Thr Gln Lys
                245                 250                 255

Ser Leu Ser Leu Ser Pro Gly Lys Ser Gly Gly Leu Asn Asp Ile Phe
            260                 265                 270

Glu Ala Gln Lys Ile Glu Trp His Glu
        275                 280

<210>  27
<211>  197
<212>  PRT
<213>  Macaca fascicularis

<400>  27

Met Gln Ser Gly Thr Arg Trp Arg Val Leu Gly Leu Cys Leu Leu Ser
1               5                   10                  15

Ile Gly Val Trp Gly Gln Asp Gly Asn Glu Glu Met Gly Ser Ile Thr
            20                  25                  30

Gln Thr Pro Tyr Gln Val Ser Ile Ser Gly Thr Thr Val Ile Leu Thr
        35                  40                  45

Cys Ser Gln His Leu Gly Ser Glu Ala Gln Trp Gln His Asn Gly Lys
    50                  55                  60

Asn Lys Glu Asp Ser Gly Asp Arg Leu Phe Leu Pro Glu Phe Ser Glu
65                  70                  75                  80

Met Glu Gln Ser Gly Tyr Tyr Val Cys Tyr Pro Arg Gly Ser Asn Pro
                85                  90                  95

Glu Asp Ala Ser His His Leu Tyr Leu Lys Ala Arg Val Cys Glu Asn
            100                 105                 110

Cys Met Glu Met Asp Val Met Ala Val Ala Thr Ile Val Ile Val Asp
        115                 120                 125

Ile Cys Ile Thr Leu Gly Leu Leu Leu Leu Val Tyr Tyr Trp Ser Lys
    130                 135                 140

Asn Arg Lys Ala Lys Ala Lys Pro Val Thr Arg Gly Ala Gly Ala Gly
145                 150                 155                 160

Gly Arg Gln Arg Gly Gln Asn Lys Glu Arg Pro Pro Pro Val Pro Asn
                165                 170                 175

Pro Asp Tyr Glu Pro Ile Arg Lys Gly Gln Gln Asp Leu Tyr Ser Gly
            180                 185                 190

Leu Asn Gln Arg Arg
        195

<210>  28
<211>  96
<212>  PRT
<213>  Macaca fascicularis

<400>  28

Gln Asp Gly Asn Glu Glu Met Gly Ser Ile Thr Gln Thr Pro Tyr Gln
1               5                   10                  15

Val Ser Ile Ser Gly Thr Thr Val Ile Leu Thr Cys Ser Gln His Leu
            20                  25                  30

Gly Ser Glu Ala Gln Trp Gln His Asn Gly Lys Asn Lys Glu Asp Ser
        35                  40                  45

Gly Asp Arg Leu Phe Leu Pro Glu Phe Ser Glu Met Glu Gln Ser Gly
    50                  55                  60

Tyr Tyr Val Cys Tyr Pro Arg Gly Ser Asn Pro Glu Asp Ala Ser His
65                  70                  75                  80

His Leu Tyr Leu Lys Ala Arg Val Cys Glu Asn Cys Met Glu Met Asp
                85                  90                  95

<210>  29
<211>  20
<212>  PRT
<213>  Macaca fascicularis

<400>  29

Tyr Pro Arg Gly Ser Asn Pro Glu Asp Ala Ser His His Leu Tyr Leu
1               5                   10                  15

Lys Ala Arg Val
            20

<210>  30
<211>  37
<212>  DNA
<213>  Artificial Sequence

<220>
<223>  Primer rbHCfinal.up

<400>  30
aagcttgcca ccatggagac tgggctgcgc tggcttc                                37

<210>  31
<211>  21
<212>  DNA
<213>  Artificial Sequence

<220>
<223>  Primer rbHCfinal.do

<400>  31
ccattggtga gggtgcccga g                                                 21

<210>  32
<211>  34
<212>  DNA
<213>  Artificial Sequence

<220>
<223>  Primer rbLCfinal.up

<400>  32
aagcttgcca ccatggacay gagggccccc actc                                   34

<210>  33
<211>  26
<212>  DNA

<213>  Artificial Sequence

<220>
<223>  Primer rbLCfinal.do

<400>  33
cagagtrctg ctgaggttgt aggtac                                          26


<210>  34
<211>  355
<212>  PRT
<213>  Artificial Sequence

<220>
<223>  human CD3e(D1-5) stalk-Fc(knob)-Avi

<400>  34

Glu Met Gly Gly Ile Thr Gln Thr Pro Tyr Lys Val Ser Ile Ser Gly
1               5                   10                  15

Thr Thr Val Ile Leu Thr Cys Pro Gln Tyr Pro Gly Ser Glu Ile Leu
            20                  25                  30

Trp Gln His Asn Asp Lys Asn Ile Gly Gly Asp Glu Asp Asp Lys Asn
        35                  40                  45

Ile Gly Ser Asp Glu Asp His Leu Ser Leu Lys Glu Phe Ser Glu Leu
    50                  55                  60

Glu Gln Ser Gly Tyr Tyr Val Cys Tyr Pro Arg Gly Ser Lys Pro Glu
65                  70                  75                  80

Asp Ala Asn Phe Tyr Leu Tyr Leu Arg Ala Arg Val Ser Glu Asn Cys
                85                  90                  95

Val Asp Glu Gln Leu Tyr Phe Gln Gly Gly Ser Pro Lys Ser Ala Asp
            100                 105                 110

Lys Thr His Thr Cys Pro Pro Cys Pro Ala Pro Glu Leu Leu Gly Gly
        115                 120                 125

Pro Ser Val Phe Leu Phe Pro Pro Lys Pro Lys Asp Thr Leu Met Ile
    130                 135                 140

Ser Arg Thr Pro Glu Val Thr Cys Val Val Val Asp Val Ser His Glu
145                 150                 155                 160

Asp Pro Glu Val Lys Phe Asn Trp Tyr Val Asp Gly Val Glu Val His
                165                 170                 175

Asn Ala Lys Thr Lys Pro Arg Glu Glu Gln Tyr Asn Ser Thr Tyr Arg
            180                 185                 190

Val Val Ser Val Leu Thr Val Leu His Gln Asp Trp Leu Asn Gly Lys
        195                 200                 205

Glu Tyr Lys Cys Lys Val Ser Asn Lys Ala Leu Pro Ala Pro Ile Glu
    210                 215                 220

Lys Thr Ile Ser Lys Ala Lys Gly Gln Pro Arg Glu Pro Gln Val Tyr
225                 230                 235                 240

Thr Leu Pro Pro Cys Arg Asp Glu Leu Thr Lys Asn Gln Val Ser Leu
                245                 250                 255

Trp Cys Leu Val Lys Gly Phe Tyr Pro Ser Asp Ile Ala Val Glu Trp
            260                 265                 270

Glu Ser Asn Gly Gln Pro Glu Asn Asn Tyr Lys Thr Thr Pro Pro Val
        275                 280                 285

Leu Asp Ser Asp Gly Ser Phe Phe Leu Tyr Ser Lys Leu Thr Val Asp
    290                 295                 300

Lys Ser Arg Trp Gln Gln Gly Asn Val Phe Ser Cys Ser Val Met His
305                 310                 315                 320

Glu Ala Leu His Asn His Tyr Thr Gln Lys Ser Leu Ser Leu Ser Pro
                325                 330                 335

Gly Lys Ser Gly Gly Leu Asn Asp Ile Phe Glu Ala Gln Lys Ile Glu
            340                 345                 350

Trp His Glu
        355